# EUROPÄISCHE PATENTSCHRIFT

(11) **EP 3 393 244 B1**
(45) Veröffentlichungstag und Bekanntmachung des Hinweises auf die Patenterteilung: **18.12.2019**
(21) Anmeldenummer: 16816273.3
(22) Anmeldetag: 19.12.2016
(51) Int. Cl.: A01N 43/54, C07D 239/42, C07D 401/12, C07D 405/12, C07D 409/12

(54) **2-AMINO-5-KETOXIM-PYRIMIDINDERIVATE UND DEREN VERWENDUNG ZUR BEKÄMPFUNG UNERWÜNSCHTEN PFLANZENWACHSTUMS**
2-AMINO-5-KETOXIM PYRIMIDINE DERIVATIVES AND USE THEREOF FOR COMBATING UNDESIRED PLANT GROWTH
DERIVES DE 2-AMINO-5-CETOXIME-PYRIMIDINE ET LEUR UTILISATION POUR LUTTER CONTRE LA CROISSANCE INDESIRABLE DE PLANTES

(30) Priorität: 21.12.2015 EP 15201481
(43) Veröffentlichungstag der Anmeldung: 31.10.2018
(73) Patentinhaber: Bayer CropScience Aktiengesellschaft, 40789 Monheim am Rhein (DE)
(72) Erfinder: MINN, Klemens, 65795 Hattersheim (DE); BUSCATO ARSEQUELL, Estella, 60486 Frankfurt am Main (DE); JAKOBI, Harald, 60598 Frankfurt (DE); DIETRICH, Hansjörg, 65835 Liederbach am Taunus (DE); GATZWEILER, Elmar, 61231 Bad Nauheim (DE); ROSINGER, Christopher Hugh, 65719 Hofheim (DE)
(74) Vertreter: BIP Patents
(86) Internationale Anmeldenummer: PCT/EP2016/081655
(87) Internationale Veröffentlichungsnummer: WO 2017/108656

(56) Entgegenhaltungen:
- WO-A1-2010/076010
- WO-A1-2013/144187

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen sowie im Ziergartenbereich und zur generellen Bekämpfung von Unkräutern und Ungräsern in Umweltbreichen, in denen Pflanzenwuchs störend ist.

Insbesondere betrifft die Erfindung substituierte 2-Amino-5- Ketoxim-Pyrimidinderivate, Verfahren zu deren Herstellung sowie deren Verwendung zur Bekämpfung von Schadpflanzen.

Die erfindungsgemäßen Verbindungen der Formel (I) weisen in der 5-Positon des Pyrimidins ein Ketoxim und in der 2-Positon des Pyrimidins einen in der alpha Position zum Aromaten über ein Amin gebundenen teilhydrierten bicyclischen Substituienten auf, wobei das Pyrimidin in der 4-Position und 6-Position ebenfalls substituiert sein kann und das Ketoxim zusammen mit dem Substituenten in der benachbarten Position einen Ring bilden kann.

Aus dem Stand der Technik ist die herbizide Wirkung von Diaminopyrimidinen und auch von Monoaminopyrimidinen bereits bekannt.

Monaminopyrimidinderivate mit herbizider Wirkung, nämlich 5-Amino-Pyrimidinderivate, sind beispielsweise in Dokument WO 2013/144187 A1 offenbart, während 2.4-Diaminopyrimidine und deren Anwendung im Bereich des Pflanzenschutzes beispielsweise durch die Dokumente EP 0523533 A1, WO 2010/076009 und WO 2010/076010 offenbart werden.

2.4-Diaminopyrimidine mit bicyclischem Rest, welche am verbrückten und am benachbarten Kohlenstoffatom eine (1R, 2S)-Konfiguration aufweisen und sich zugleich durch eine herbizide Wirksamkeit auszeichnen, sind aus US 2010/0167934 A1 bekannt.

Die Anwendung der bekannten Pyrimidinderivate als selektive Herbizide zur Schadpflanzenbekämpfung oder als Pflanzenwachstumsregulatoren in verschiedenen Nutzpflanzenkulturen erfordert jedoch häufig eine hohe Kosten verursachende Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich.

Es ist deshalb erstrebenswert, alternative chemische Wirkstoffe auf Basis von Pyrimidinderivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Die Aufgabe der vorliegenden Erfindung besteht in der Bereitstellung alternativer Pyrimidin-Derivate, welche als Herbizide oder Pflanzenwachstumsregulatoren, mit einer zufriedenstellenden herbiziden Wirkung und einem breiten Wirkspektrum gegenüber Schadpflanzen und/oder mit einer hohen Selektivität in Nutzpflanzenkulturen, eingesetzt werden können. Außerdem weisen die alternativen Pyrimidin-Derivate im Vergleich zu den aus dem Stand der Technik bekannten Pyrimidinderivaten ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen zeigen.

Gelöst wird die Aufgabe durch speziell substituierte 2-Amino-5-Ketoxim-Pyrimidinderivate der Formel (I) gemäß Anspruch 1, welche vorteilhaft als Herbizide und auch als Pflanzenwachstumsregulatoren eingesetzt werden können.

Gegenstand der vorliegenden Erfindung sind daher Verbindungen der Formel (I) 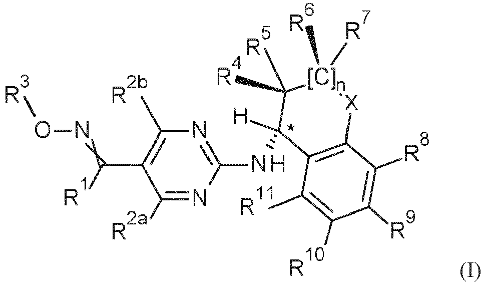 und deren agrochemisch verträglichen Salze, in welchen
- R¹: ausgewählt ist aus der Gruppe, bestehend aus
- -: (C₁-C₆)-Alkyl, (C₁-C₆)-Halogenalkyl, (C₁-C₆)-Alkylcarbonyl-(C₁-C₄)-alkyl;
- -: (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- -: (C₂-C₆)-Alkenyl, (C₂-C₆)-Halogenalkenyl;
- -: (C₂-C₆)-Alkinyl, (C₂-C₆)-Halogenalkinyl; Tri-(C₁-C₆)-alkylsilyl-(C₂-C₆)-alkinyl, Di-(C₁-C₆)-alkydsilyl-(C₂-C₆)-alkinyl, Mono-(C₁-C₆)-alkylsilyl-(C₂-C₆)-alkinyl; Phenylsilyl-(C₂-C₆)-alkmyl;
- -: (C₆-C₁₄)-Aryl, welche jeweils am Arylteil mit Halogen, (C₁-C₆)-Alkyl und/oder (C₁-C₆)-Haloalkyl substituiert sein können;
- -: (C₆-C₁₄)-Aryl-(C₁-C₆)-alkyl;
- -: Aminocarbonyl-(C₁-C₆)-alkyl;
- -: (C₁-C₆)-Alkoxy-(C₁-C₆)-alkyl;
- -: (C₃-C₈)-Cycloalkyl, welches unsubstituiert oder einfach oder mehrfach am Cycloalkylrest durch (C₁-C₆)-Alkyl und/oder Halogen substituiert sein kann; (C₃-C₈)-Cycloalkyl-(C₁-C₆)-alkyl, (C₃-C₈)-Cycloalkyl-(C₁-C₆)-haloalkyl;
- -: (C₃-C₈)-Cycloalkenyl, (C₃-C₈)-Cycloalkenyl-(C₁-C₆)-alkyl, (C₃-C₈)-Cycloalkenyl-(C₁-C₆)-halogenalkyl;
- -: Hydioxy-(C₁-C₆)-alkyl, Cyano-(C₁-C₆)-alkyl;
- -: (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl;
- R^{2a} und R^{2b},: jeweils unabhängig voneinander, ausgewählt sind aus der Gruppe, bestehend aus
- -: Wasserstoff, Halogen, Hydroxy, Cyano, C(O)OH, C(O)NH₂;
- -: (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- -: (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- -: (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- -: (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl; Tri-(C₁-C₆)-alkylsilyl-(C₂-C₆)-alkinyl, Di-(C₁-C₆)-alkydsilyl-(C₂-C₆)-alkinyl, Mono-(C₁-C₆)-alkylsilyl-(C₂-C₆)-alkinyl; Phenylsilyl-(C₂-C₆)-alkinyl;
- -: (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- -: (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- -: Aminocarbonyl-(C₁-C₆)-alkyl, Di-(C₁-C₆)-Alkylaminocarbonyl-(C₁-C₆)-alkyl;
- -: N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- -: (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- -: (C₃-C₈)-Cycloalkyl, welches unsubstituiert oder einfach oder mehrfach am Cycloalkylrest durch (C₁-C₆)-Alkyl und/oder Halogen substituiert ist; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- -: (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloalkenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- -: Hydroxy-(C₁-C₆)-alkyl, Hydroxy-(C₁-C₆)-alkoxy Cyano-(C₁-C₆)-alkoxy, Cyano-(C₁-C₆)-alkyl;
- -: (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-hatoatkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-Alkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio;
- R¹ mitR^{2a}: über eine Bindung miteinander verbunden sind, so dass zusammen mit dem Kohlenstoff 4 des Pyrimidinrings (bzw. Kohlenstoff 6 des Pyrimidinrings, je nach Zählweise) und dem Kohlenstoff 5 des Pyrimidins und der Carbonylgruppe in der 5-Positon des Pyrimidins sich ein 5 bis 7 gliedriger teilhydrierter Carbo- oder Heterocyclus ergibt, der unsubstituiert ist oder substituiert ist mit einem oder mehreren Substituenten ausgewählt aus der Gruppe bestehend aus (C₁-C₆)-Alkyl, (C₁-C₆)-Haloalkyl, (C₃-C₈)-Cycloalkyl, spiro-(C₂-C₅)-Cycloalkyl und Phenyl (C₆H₅) substituiert ist, wobei die Phenylgruppe mit einem oder mehreren Substituenten ausgewählt aus Fluor, Chlor, Brom oder Iod substituiert sein kann;
- R³: ausgewählt ist aus der Gruppe, bestehend aus
- -: Wasserstoff;
- -: (C₁-C₆)-Alkyl, (C₁-C₆)-Halogenalkyl, (C₁-C₆)-Alkylcarbonyl-(C₁-C₄)-alkyl;
- -: (C₂-C₆)-Alkenyl, (C₂-C₆)-Halogenalkenyl;
- -: (C₂-C₆)-Alkinyl, (C₂-C₆)-Halogenalkinyl; Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- -: (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- -: (C₆-C₁₄)-Aryl, welche jeweils am Arylteil mit Halogen, (C₁-C₆)-Alkyl und/oder (C₁-C₆)-Haloalkyl substituiert sein können;
- -: (C₂-C₁₄)-Het-Aryl, welche jeweils am Het-Arylteil mit Halogen, (C₁-C₆)-Alkyl und/oder (C₁-C₆)-Haloalkyl substituiert sein können;
- -: (C₆-C₁₄)-Aryl-(C₁-C₆)-alkyl;
- -: Aminocarbonyl-(C₁-C₆)-alkyl;
- -: (C₁-C₆)-Alkoxy-(C₁-C₆)-alkyl;
- -: (C₃-C₈)-Cycloalkyl, welches einfach oder mehrfach am Cycloalkylrest durch (C₁-C₆)-Alkyl und/oder Halogen substituiert sein kann; (C₃-C₈)-Cycloalkyl-(C₁-C₆)-alkyl, (C₃-C₈)-Cycloalkyl-(C₁-C₆)-haloalkyl;
- -: (C₃-C₈)-Cycloalkenyl-(C₁-C₆)-alkyl, (C₃-C₈)-Cycloalkenyl-(C₁-C₆)-halogenalkyl, Hydroxy-(C₁-C₆)-alkyl, Cyano-(C₁-C₆)-alkyl;
- -: (C₁-C₆)-Alkylsulfbnyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloallcyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl;
- R⁴ und R⁵: jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy; oder die Reste R⁴ und R⁵ zusammen mit dem Kohlenstoffatom, an welchem sie gebunden sind, einen drei- bis siebengliedrigen Ring bilden;
- R⁶ und R⁷: jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
- n: die Laufzahl 0, 1 oder 2 ist;
- R⁸, R⁹, R¹⁰ und R¹¹: jeweils unabhängig voneinander, ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro, wobei die Reste R⁹ und R¹⁰ ringbildend durch eine -O-CH₂-O- Gruppe verbunden sein können;
- X: für eine Bindung, CH₂, O, S, Carbonyl, NH, CR¹²R¹³,NR¹⁴,CH₂O oder CH₂S steht, wobei bei den beiden letztgenannten Gruppen das Kohlenstoffatom an den aromatischen Teil und das Heteroatom O oder S an den teilhydrierten Teil des Amins gebunden ist;
- R¹² und R¹³: jeweils unabhängig voneinander, ausgewählt sind aus der Gruppe, bestehend aus Wasserstoff, (C₁-C₆)-Alkyl und (C₁-C₆)-Haloalkyl; und
- R¹⁴: ausgewählt ist aus der Gruppe, bestehend aus Wasserstoff, (C₁-C₆)-Alkyl und (C₁-C₆)-Haloalkyl.

Die erfindungsgemäßen 2-Amino-5-Ketoxim-pyrimidine der Formel (I) unterscheiden sich von den aus den Dokumenten EP 0523533 A1, WO 2010/076009 und WO 2010/076010 bekannten Herbiziden mit 2.4-Diaminopyrimidin-Struktur in der Zahl der Aminogruppen, d.h. die erfindungsgemäßen 2-Amino-5-Ketoxim-pyrimidine sind durch nur eine Aminogruppe substituiert.

Bei den erfindungsgemäßen Monoaminopyrimidinen der Formel (I) stehen die Reste R^{2a} und R^{2b} also nicht für eine Aminogruppe, d.h. die R^{2a} und R^{2b} sind nicht über ein Stickstoffatom mit dem Pyrimidinring verbunden.

Die Aminogruppe in der 2-Positon des Pyrimidins weist bei den erfindungsgemäßen Monoaminopyrimidinen der Formel (I) einen teilhydrierten bicyclischen Substituenten auf, wobei der teilhydrierte bicyclische Substituent in der alpha Position zum Aromaten an das Amin gebunden ist.

Von den aus Dokument WO 2013/144187 A1 bekannten Pyrimidinen mit nur einem Aminosubstituenten (Monoaminopyrimidinen) unterscheiden sich die 2-Amino-5-keto-pyrimidine der Formel (I) der vorliegenden Erfindung dagegen dadurch, dass die Aminogruppe in 2-Position, d.h. zwischen den beiden zum Pyrimidinring gehörenden Stickstoffatomen, und nicht in 5-Position angeordnet ist.

Die Verbindungen der Formel (I) zeichnen sich, neben einem guten Wirksamkeitsprofil und einer guten Kulturpflanzenverträglichkeit, durch ihre kostengünstige Herstellung aus, da die erfindungsgemäßen Substanzen aus preiswerten und gut zugänglichen Vorstufen durch kostengünstige Prozesse hergestellt werden können. Daher kann auf die Verwendung teurer und schwer zugänglicher Zwischenprodukte verzichtet werden.

Im Folgenden werden, jeweils für die einzelnen Substituenten, bevorzugte, besonders bevorzugte und ganz besonders bevorzugte Bedeutungen beschrieben. Die übrigen Substituenten der allgemeinen Formel (I), welche nachfolgend nicht genannt werden, weisen die oben genannte Bedeutung auf. Das gilt auch für die Laufzahl n, d.h. die Laufzahl n ist in den nachfolgenden Ausführungsformen 0, 1 oder 2.

Eine erste Ausführungsform der vorliegenden Erfindung umfasst Verbindungen der allgemeinen Formel (I), in welchen
- R¹: bevorzugt ausgewählt ist aus der Gruppe, bestehend aus (C₁-C₆)-Alkyl, (C₁-C₆)-Haloalkyl, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist;
- R¹: besonders bevorzugt ausgewählt ist aus der Gruppe, bestehend aus (C₁-C₃)-Alkyl, (C₁-C₃)-Haloalkyl, (C₁-C₃)-Alkoxy-(C₁-C₃)-alkyl, (C₃-C₆)-Cycloalkyl und (C₃-C₆)-Cycloalkyl-(C₁-C₃)-alkyl;
- R¹: ganz besonders bevorzugt CH₃, CH₂CH₃, CH₂CH₂CH₃, CH(CH₃)₂, CF₃, Cyclopropyl, Cyclobutyl, Cyclopentyl, Cyclohexyl, CH₂-Cyclopropyl oder CH₂-Cyclohexyl ist;
- R¹: am meisten bevorzugt CH₃ ist.

Eine zweite Ausführungsform der vorliegenden Erfindung umfasst Verbindungen der allgemeinen Formel (I), in welchen
- R^{2a} und R^{2b}: jeweils unabhängig voneinander, bevorzugt ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist;
- R^{2a} und R^{2b}: jeweils unabhängig voneinander, besonders bevorzugt ausgewählt sind aus der Gruppe, bestehend aus Wasserstoff, (C₁-C₃)-Alkoxy, (C₁-C₃)-Alkoxy-(C₁-C₃)-alkyl, (C₃-C₈)-Cycloalkyl, (C₃-C₈)-Cycloalkyl-(C₁-C₃)-alkyl und (C₃-C₈)-Cycloalkyl-(C₁-C₃)-haloalkyl, wobei der Cycloalkylrest jeweils unsubstituiert ist oder durch (C₁-C₃)-Alkyl und/oder Halogen substituiert ist;
- R^{2a} und R^{2b}: jeweils unabhängig voneinander, ganz besonders bevorzugt ausgewählt sind aus der Gruppe, bestehend aus Wasserstoff, CH₃, CH₂CH₃, CH(CH₃)₂, C(CH₃)₃, CF₃, CF₂H, CF₂Cl, Cyclopropyl und CH₂OCH₃;
- R^{2a} und R^{2b}: am meisten bevorzugt jeweils unabhängig voneinander ausgewählt ist aus Wasserstoff und CH₃.

Eine dritte Ausführungsform der vorliegenden Erfindung umfasst Verbindungen der allgemeinen Formel (I), in welchen
- R¹ mit R^{2a}: bevorzugt über eine Bindung miteinander verbunden sind, so dass sich zusammen mit den Kohlenstoffen 4 und 5 des Pyrimidins und der Carbonylgruppe in der 5-Positon des Pyrimidins ein 5- bis 7-gliedriger teilhydrierter Carbo- oder Heterocyclus ergibt, worin die Heteroatome ausgewählt sind aus der Gruppe bestehend aus Sauerstoff oder Schwefel, wobei anstatt des Schwefels eine Sulfoxidgruppe oder eine Sulfonylgruppe vorliegen kann, und der Carbo- oder Heterocyclus unsubstituiert oder mit einem oder mehreren Substituenten ausgewählt aus der Gruppe bestehend aus (C₁-C₃)-Alkyl, (C₁-C₃)-Haloalkyl, (C₃-C₈)-Cycloalkyl, spiro(C₂-C₅)-Cycloalkyl und Phenyl (C₆H₅) substituiert ist, welches mit einem oder mehreren Substituenten ausgewählt aus Fluor, Chlor, Brom oder Iod substituiert sein kann ist;
- R¹ mit R^{2a}: ganz besonders bevorzugt über eine Bindung miteinander verbunden sind, so dass zusammen mit den Kohlenstoffen 4 und 5 des Pyrimidins und der Carbonylgruppe in der 5-Positon des Pyrimidins sich ein sechsgliedriger teilhydrierter Carbo- oder Heterocyclus ergibt, worin die Heteroatome ausgewählt sind aus der Gruppe bestehend aus Sauerstoff oder Schwefel, wobei anstatt des Schwefels eine Sulfoxidgruppe oder eine Sulfonylgruppe vorliegen kann, und der Carbo- oder Heterocyclus unsubstituiert ist oder substituiert ist mit einem oder mehreren Substituenten ausgewählt aus der Gruppe bestehend aus Methyl, Ethyl, Trifluormethyl, (C₃-C₆)-Cyclopropyl, und Phenyl (C₆H₅), welches einfach oder zweifach mit Fluor substituiert sein kann, und in welchen
- R¹ mit R^{2a}: am meisten bevorzugt über eine Bindung miteinander verbunden sind, so dass sich zusammen mit dem Kohlenstoffen 4 und 5 des Pyrimidins und der Carbonylgruppe in der 5-Positon des Pyrimidins ein 6-gliedriger teilhydrierter Carbocyclus ergibt.

Eine vierte Ausführungsform der vorliegenden Erfindung umfasst Verbindungen der allgemeinen Formel (I), in welchen
- R³: bevorzugt ausgewählt ist aus der Gruppe, bestehend aus Wasserstoff, (C₁-C₆)-Alkyl, (C₁-C₆)-Halogenalkyl, (C₁-C₆)-Alkoxy-(C₁-C₆)-alkyl, (C₂-C₆)-Alkenyl, (C₂-C₆)-Halogenalkenyl, (C₂-C₆)-Alkinyl, (C₂-C₆)-Halogenalkiny, (C₆-C₁₄)-Aryl-(C₁-C₆)-alkyl;
- R³: besonders bevorzugt ausgewählt ist aus der Gruppe, bestehend aus Wasserstoff, (C₁-C₃)-Alkyl, (C₂-C₄)-Alkinyl;
- R³: ganz besonders bevorzugt ausgewählt ist aus der Gruppe, bestehend aus Wasserstoff, CH₃ und CH₂CH₃; und in welchen
- R³: am meisten bevorzugt Wasserstoff ist.

Eine fünfte Ausführungsform der vorliegenden Erfindung umfasst Verbindungen der allgemeinen Formel (I), in welchen
- R⁴ und R⁵: jeweils unabhängig voneinander, bevorzugt ausgewählt sind aus der Gruppe, bestehend aus Wasserstoff, Hydroxy, (C₁-C₆)-Alkyl, (C₁-C₆)-Alkylphenyl und (C₁-C₆)-Alkoxy;
- R⁴ und R⁵: jeweils unabhängig voneinander, besonders bevorzugt ausgewählt sind aus der Gruppe, bestehend aus Wasserstoff, (C₁-C₆)-Alkyl und (C₁-C₆)-Alkoxy;
- R⁴ und R⁵: jeweils unabhängig voneinander, ganz besonders bevorzugt ausgewählt sind aus der Gruppe, bestehend aus Wasserstoff, (C₁-C₃)-Alkyl, (C₁-C₆)-Alkoxy; und in welchen
- R⁴ und R⁵: jeweils unabhängig voneinander, am meisten bevorzugt Wasserstoff oder Methyl sind.

In dieser fünften Ausführungsform ist speziell bevorzugt, wenn mindestens einer der Reste R⁴ bzw. R⁵ Wasserstoff ist. D.h. wenn jeweils mindestens einer der Reste R⁴, bzw. R⁵ Wasserstoff ist und der andere Rest R⁴, bzw. R⁵ ungleich Wasserstoff, insbesondere (C₁-C₆)-Alkyl, bevorzugt Methyl (CH₃), ist.

Eine sechste Ausführungsform der vorliegenden Erfindung umfasst Verbindungen der allgemeinen Formel (I), in welchen
- R⁶ und R⁷: unabhängig voneinander, bevorzugt ausgewählt sind aus der Gruppe, bestehend aus Wasserstoff, (C₁-C₆)-Alkyl und (C₆-C₁₄)-Aryl;
- R⁶ und R⁷: unabhängig voneinander, besonders bevorzugt ausgewählt sind aus der Gruppe, bestehend aus Wasserstoff, Methyl und Phenyl; und in welchen
- R⁶ und R⁷: ganz besonders bevorzugt jeweils Wasserstoff oder Methyl sind; und in welchen
- R⁶ und R⁷: am meisten bevorzugt Wasserstoff bedeuten.

Eine siebte Ausführungsform der vorliegenden Erfindung umfasst Verbindungen der allgemeinen Formel (I), in welchen
- R⁸: bevorzugt ausgewählt ist aus der Gruppe, bestehend aus Wasserstoff, Halogen, (C₁-C₆)-Alkyl, (C₁-C₆)Alkoxy, und (C₆-C₁₄)-Aryl;
- R⁸: besonders bevorzugt ausgewählt ist aus der Gruppe, bestehend aus Wasserstoff, Halogen, (C₁-C₃)-Alkyl und (C₆-C₈)-Aryl; und
- R⁸: ganz besonders bevorzugt ausgewählt ist aus der Gruppe, bestehend aus Wasserstoff, Fluor, Chlor, CH₃, und Phenyl, und in welchen
- R⁸: am meisten bevorzugt Wasserstoff ist.

Eine achte Ausführungsform der vorliegenden Erfindung umfasst Verbindungen der allgemeinen Formel (I), in welchen
- R⁹: bevorzugt ausgewählt ist aus der Gruppe, bestehend aus Wasserstoff, Halogen, (C₁-C₆)-Alkyl und (C₁-C₆)-Alkoxy;
- R⁹: besonders bevorzugt ausgewählt ist aus der Gruppe, bestehend aus Wasserstoff, Chlor, Fluor und (C₁-C₃)-Alkoxy; und
- R⁹: ganz besonders bevorzugt ausgewählt ist aus der Gruppe, bestehend aus Wasserstoff, Fluor, Chlor und Methoxy, und in welchen
- R⁹: am meisten bevorzugt Wasserstoff ist.

Eine neunte Ausführungsform der vorliegenden Erfindung umfasst Verbindungen der allgemeinen Formel (I), in welchen
- R¹⁰: bevorzugt ausgewählt ist aus der Gruppe, bestehend aus Wasserstoff, Halogen, Cyano, Aminocarbonyl, Hydroxycarbonyl, (C₁-C₆)-Alkyl, (C₁-C₆)-Haloalkyl, (C₁-C₆)-Alkoxy, (C₂-C₆)-Alkenyl, (C₂-C₆)-Alkinyl, (C₁-C₆)-Alkyl-(C₂-C₆)-alkinyl, Hydroxy-(C₁-C₆)-Alkyl-(C₂-C₆)-alkinyl, (C₁-C₆)-Alkoxy-(C₂-C₆)-Alkinyl und Aryl-(C₂-C₆)-Alkinyl;
- R¹⁰: besonders bevorzugt ausgewählt ist aus der Gruppe, bestehend aus Wasserstoff, Fluor, Chlor, Brom, Iod, Cyano, Methoxy (OCH3), (C₁-C₃)-Alkyl, (C₁-C₃)-Haloalkyl, (C₁-C₃)-Alkoxy, (C₂-C₄)-Alkenyl, (C₂-C₄)-Alkinyl, (C₁-C₃)-Alkyl- (C₂-C₄)-alkinyl, Hydroxy-(C₁-C₃)-Alkyl-(C₂-C₄)-alkinyl, (C₁-C₃)-Alkoxy-(C₂-C₄)-Alkinyl und Phenyl-(C₂-C₄)-Alkinyl;
- R¹⁰: ganz besonders bevorzugt ausgewählt ist aus der Gruppe, bestehend aus Wasserstoff, Fluor, Chlor, Brom, Iod, Cyano, Methyl, C≡CH, und C≡CCH₃, und in welchen
- R¹⁰: am meisten bevorzugt Wasserstoff oder CH₃ ist.

Eine zehnte Ausführungsform der vorliegenden Erfindung umfasst Verbindungen der allgemeinen Formel (I), in welchen
- R¹¹: bevorzugt ausgewählt ist aus der Gruppe, bestehend aus Wasserstoff und (C₁-C₆)-Alkyl;
- R¹¹: besonders bevorzugt ausgewählt ist aus der Gruppe, bestehend aus Wasserstoff und (C₁-C₄)-Alkyl; und
- R¹¹: ganz besonders bevorzugt Wasserstoff oder Methyl ist, und in welchen
- R¹¹: am meisten bevorzugt Wasserstoff ist.

Eine elfte Ausführungsform der vorliegenden Erfindung umfasst Verbindungen der allgemeinen Formel (I), in welchen
- X: bevorzugt ausgewählt ist aus der Gruppe, bestehend aus einer chemischen Bindung, CH₂, O, S, Carbonyl, NH, CH(C₁-C₆)Alkyl, N(C₁-C₆)Alkyl, OCH₂, und SCH₂ wobei bei den beiden letztgenannten Gruppen das Kohlenstoffatom an den aromatischen Teil und das Heteroatom O oder S an den teilhydrierten Teil des Amins gebunden ist;
- X: besonders bevorzugt ausgewählt ist aus der Gruppe bestehend aus einer chemischen Bindung, CH₂, O, S, CHCH₃, NCH₃, OCH₂, und SCH₂ wobei bei den beiden letztgenannten Gruppen das Kohlenstoffatom an den aromatischen Teil und das Heteroatom O oder S an den teilhydrierten Teil des Amins gebunden ist; und in welchen
- X: am meisten bevorzugt für eine chemische Bindung oder CH₂ steht.

Im Rahmen der vorliegenden Erfindung ist es möglich, die einzelnen bevorzugten, besonders bevorzugten und ganz besonders bevorzugten Bedeutungen für die Substituenten R¹ bis R¹¹ und X beliebig miteinander zu kombinieren, wobei die Laufzahl n 0, 1 oder 2 ist, bevorzugt 0 oder 1 ist.

Das heißt, dass Verbindungen der allgemeinen Formel (I) von der vorliegenden Erfindung umfasst sind, in welchen beispielsweise der Substituent R¹ eine bevorzugte Bedeutung aufweist und die Substituenten R² bis R¹⁴ die allgemeine Bedeutung aufweisen oder aber der Substituent R² eine bevorzugte Bedeutung aufweist, der Substituent R³ eine besonders bevorzugte, bzw. eine ganz besonders bevorzugte, Bedeutung aufweist und die übrigen Substituenten eine allgemeine Bedeutung aufweisen.

Vier dieser Kombinationen der oben für die Substituenten R¹ bis R¹¹ und X gegebenen Definitionen werden nachfolgend beispielhaft erläutert und jeweils als weitere Ausführungsformen offenbart:
Kombination der oben für die Substituenten R¹ bis R¹¹ und X jeweils als besonders bevorzugt bezeichneten Definitionen (zwölfte Ausführungsform), Kombination der oben für die Substituenten R¹ bis R¹¹ und X jeweils als ganz besonders bevorzugt bezeichneten Definitionen (dreizehnten Ausführungsform), und Kombination der oben für den Substituenten R¹ als ganz besonders bevorzugt bezeichneten Definition mit den oben für die Substituenten R¹ bis R¹¹ und X jeweils als besonders bevorzugt bezeichneten Definitionen (vierzehnten und fünfzehnten Ausführungsform).

Die vorgenannten auf den Kombinationen der Substituenten basierenden weiteren Ausführungsformen werden nachfolgend aus Gründen der Klarheit explizit offenbart:
Eine zwölfte Ausführungsform der vorliegenden Erfindung umfasst Verbindungen der allgemeinen Formel (I), in welchen
- R¹: ausgewählt ist aus der Gruppe, bestehend aus (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist;
- R^{2a} und R^{2b}: jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist;
- R¹ mit R^{2a}: über eine Bindung miteinander verbunden sind, so dass sich zusammen mit den Kohlenstoffen 4 und 5 des Pyrimidins und der Carbonylgruppe in der 5-Positon des Pyrimidins ein 5- bis 7-gliedriger teilhydrierter Carbo- oder Heterocyclus ergibt, worin die Heteroatome ausgewählt sind aus der Gruppe bestehend aus Sauerstoff oder Schwefel, wobei anstatt des Schwefels eine Sulfoxidgruppe oder eine Sulfonylgruppe vorliegen kann, und der Carbo- oder Heterocyclus unsubstituiert ist oder mindestens einfach substituiert ist und wobei die Substituenten ausgewählt sind aus der Gruppe bestehend aus (C₁-C₃)-Alkyl, (C₁-C₃)-Haloalkyl, (C₃-C₈)-Cycloalkyl, und Phenyl (C₆H₅), welches durch Fluor, Chlor, Brom oder Iod substituiert sein kann;
- R³: ausgewählt ist aus der Gruppe bestehend Wasserstoff, (C₁-C₆)-Alkyl, (C₁-C₆)-Halogenalkyl, (C₁-C₆)-Alkoxy-(C₁-C₆)-alkyl, (C₂-C₆)-Alkenyl, (C₂-C₆)-Halogenalkenyl, (C₂-C₆)-Alkinyl, und (C₂-C₆)-Halogenalkinyl;
- R⁴ und R⁵: jeweils unabhängig voneinander, ausgewählt sind aus der Gruppe, bestehend aus Wasserstoff, Hydroxy, (C₁-C₆)-Alkyl, (C₁-C₆)-Alkylphenyl und (C₁-C₆)-Alkoxy;
- R⁶ und R⁷: unabhängig voneinander ausgewählt sind aus der Gruppe, bestehend aus Wasserstoff, (C₁-C₆)-Alkyl und (C₆-C₁₄)-Aryl;
- R⁸: ausgewählt ist aus der Gruppe, bestehend aus Wasserstoff, Halogen, (C₁-C₆)-Alkyl, (C₁-C₆)Alkoxy, und (C₆-C₁₄)-Aryl;
- R⁹: ausgewählt ist aus der Gruppe, bestehend aus Wasserstoff, Halogen, (C₁-C₆)-Alkyl und (C₁-C₆)-Alkoxy;
- R¹⁰: ausgewählt ist aus der Gruppe, bestehend aus Wasserstoff, Halogen, Cyano, Aminocarbonyl, Hydroxycarbonyl, (C₁-C₆)-Alkyl, (C₁-C₆)-Haloalkyl, (C₁-C₆)-Alkoxy, (C₂-C₆)-Alkenyl, (C₂-C₆)-Alkinyl, (C₁-C₆)-Alkyl-(C₂-C₆)-alkinyl, Hydroxy-(C₁-C₆)-Alkyl-(C₂-C₆)-alkinyl, (C₁-C₆)-Alkoxy-(C₂-C₆)-Alkinyl und Aryl-(C₂-C₆)-Alkinyl;
- R¹¹: ausgewählt ist aus der Gruppe, bestehend aus Wasserstoff und (C₁-C₆)-Alkyl; und
- X: ausgewählt ist aus der Gruppe, bestehend aus einer chemischen Bindung, CH₂, O, S, Carbonyl, NH, CH(C₁-C₆)Alkyl, N(C₁-C₆)Alkyl, OCH₂, und SCH₂, wobei bei den beiden letztgenannten Gruppen das Kohlenstoffatom an den aromatischen Teil und das Heteroatom O oder S an den teilhydrierten Teil des Amins gebunden ist.

Eine dreizehnte Ausführungsform der vorliegenden Erfindung umfasst Verbindungen der allgemeinen Formel (I), in welchen
- R¹: ausgewählt ist aus der Gruppe, bestehend aus, (C₁-C₃)-Alkyl, (C₁-C₃)-Haloalkyl, (C₁-C₃)-Alkoxy, (C₁-C₃)-Alkoxy-(C₁-C₃)-alkyl, (C₃-C₆)-Cycloalkyl und (C₃-C₆)-Cycloalkyl-(C₁-C₃)-alkyl;
- R^{2a} und R^{2b}: jeweils unabhängig voneinander, ausgewählt sind aus der Gruppe, bestehend aus Wasserstoff, (C₁-C₃)-Alkoxy, (C₁-C₃)-Alkoxy-(C₁-C₃)-alkyl, (C₃-C₈)-Cycloalkyl, (C₃-C₈)-Cycloalkyl-(C₁-C₃)-alkyl und (C₃-C₈)-Cycloalkyl-(C₁-C₃)-haloalkyl, wobei der Cycloalkylrest jeweils unsubstituiert ist oder durch (C₁-C₃)-Alkyl und/oder Halogen substituiert ist;
- R³: ausgewählt ist aus der Gruppe bestehend aus Wasserstoff, (C₁-C₃)-Alkyl, und (C₂-C₄)-Alkinyl;
- R⁴ und R⁵: jeweils unabhängig voneinander ausgewählt sind aus der Gruppe, bestehend aus Wasserstoff, (C₁-C₆)-Alkyl und (C₁-C₆)-Alkoxy;
- R⁶ und R⁷: unabhängig voneinander, ausgewählt sind aus der Gruppe, bestehend aus Wasserstoff, Methyl und Phenyl;
- R⁸: ausgewählt ist aus der Gruppe, bestehend aus Wasserstoff, Halogen, (C₁-C₃)-Alkyl und (C₆-C₈)-Aryl;
- R⁹: ausgewählt ist aus der Gruppe, bestehend aus Wasserstoff, Chlor, Fluor und (C₁-C₃)-Alkoxy;
- R¹⁰: ausgewählt ist aus der Gruppe, bestehend aus Wasserstoff, Fluor, Chlor, Brom, Iod, Cyano, Methoxy (OCH₃), (C₁-C₃)-Alkyl, (C₁-C₃)-Haloalkyl, (C₁-C₃)-Alkoxy, (C₂-C₄)-Alkenyl, (C₂-C₄)-Alkinyl, (C₁-C₃)-Alkyl- (C₂-C₄)-alkinyl, Hydroxy-(C₁-C₃)-Alkyl-(C₂-C₄)-alkinyl, (C₁-C₃)-Alkoxy-(C₂-C₄)-Alkinyl und Phenyl-(C₂-C₄)-Alkinyl;
- R¹¹: ausgewählt ist aus der Gruppe, bestehend aus Wasserstoff und (C₁-C₄)-Alkyl;
- X: für eine chemische Bindung oder O, S, Carbonyl, CH₂, NH, CHCH₃, NCH₃, C(CH₃)₂, OCH₂ und SCH₂ steht, wobei bei den beiden letztgenannten Gruppen das Kohlenstoffatom an den aromatischen Teil und das Heteroatom O oder S an den teilhydrierten Teil des Amins gebunden ist.

Eine vierzehnte Ausführungsform der vorliegenden Erfindung umfasst Verbindungen der allgemeinen Formel (I), in welchen
- R¹: CH₃, CH₂CH₃, CH₂CH₂CH₃, CH(CH₃)₂, CF₃, Cyclopropyl, Cyclobutyl, Cyclopentyl, Cyclohexyl, CH₂-Cyclopropyl oder CH₂-Cyclohexyl ist;
- R^{2a} und R^{2b}: jeweils unabhängig voneinander ausgewählt sind aus der Gruppe, bestehend aus Wasserstoff, CH₃, CH₂CH₃, CH(CH₃)₂, C(CH₃)₃, CF₃, CF₂H, CF₂Cl, Cyclopropyl und CH₂OCH₃;
- R³: ausgewählt ist aus der Gruppe bestehend aus Wasserstoff und (C₁-C₃)-Alkyl,
- R⁴ und R⁵: jeweils unabhängig voneinander ausgewählt sind aus der Gruppe, bestehend aus Wasserstoff, (C₁-C₃)-Alkyl und (C₁-C₆)-Alkoxy;
- R⁶ und R⁷: jeweils Wasserstoff oder Methyl sind;
- R⁸: ausgewählt ist aus der Gruppe, bestehend aus Wasserstoff, Fluor, Chlor, CH₃, und Phenyl;
- R⁹: ausgewählt ist aus der Gruppe, bestehend aus Wasserstoff, Fluor, Chlor und Methoxy;
- R¹⁰: ausgewählt ist aus der Gruppe, bestehend aus Wasserstoff, Fluor, Chlor, Brom, Iod, Cyano, Methyl, C≡CH, und C≡CCH₃;
- R¹¹: Wasserstoff oder Methyl ist; und
- X: für eine chemische Bindung steht oder O, S, CH₂, CHCH₃, OCH₂, SCH₂ und NCH₃, steht, wobei bei den beiden letztgenannten Gruppen das Kohlenstoffatom an den aromatischen Teil und das Heteroatom O oder S an den teilhydrierten Teil des Amins gebunden ist.

Eine fünfzehnte Ausführungsform der vorliegenden Erfindung umfasst Verbindungen der allgemeinen Formel (I), in welchen
- R¹ mit R^{2a}: über eine Bindung miteinander verbunden sind, so dass sich zusammen mit dem Kohlenstoffen 4 und 5 des Pyrimidins und der Carbonylgruppe in der 5-Positon des Pyrimidins ein 6 gliedriger teilhydrierter Carbocyclus ergibt;
- R^{2b}: ausgewählt ist aus der Gruppe bestehend aus Wasserstoff und CH₃;
- R³: ausgewählt ist aus der Gruppe bestehend aus Wasserstoff und (C₁-C₃)-Alkyl,
- R⁴ und R⁵: jeweils unabhängig voneinander ausgewählt sind aus der Gruppe, bestehend aus Wasserstoff, (C₁-C₃)-Alkyl und (C₁-C₆)-Alkoxy;
- R⁶ und R⁷: jeweils Wasserstoff oder Methyl sind;
- R⁸: ausgewählt ist aus der Gruppe bestehend aus Wasserstoff, Fluor, Chlor, CH₃, und Phenyl;
- R⁹: ausgewählt ist aus der Gruppe bestehend aus Wasserstoff, Fluor, Chlor und Methoxy;
- R¹⁰: ausgewählt ist aus der Gruppe bestehend aus Wasserstoff, Fluor, Chlor, Brom, Iod, Cyano, Methyl, C≡CH, und C≡CCH₃;
- R¹¹: Wasserstoff oder Methyl ist; und
- X: für eine chemische Bindung oder CH₂ steht.

Im Rahmen der vorliegenden Erfindung sind von der Verbindung der allgemeinen Formel (I) auch Verbindungen umfasst, die durch a) Protonierung, b) Alkylierung oder c) Oxidation an einem Stickstoffatom quaterniert sind. Insbesondere sind diesebzüglich die entsprechenden N-Oxide zu nennnen.

Die Verbindungen der Formel (I) können Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR'R''R''']⁺, worin R bis R''' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Im Folgenden werden die Verbindungen der Formel (I) und ihre Salze auch kurz als erfindungsgemäß verwendete oder erfindungsgemäße "Verbindungen (I)" bezeichnet.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 6 Kohlenstoffatomen, insbesondere 1 bis 4 Kohlenstoffatomen, bzw. bei ungesättigten Gruppen mit 2 bis 6 Kohlenstoffatomen, insbesondere 2 bis 4 Kohlenstoffatomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexy und 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen, sowie die entsprechenden ungesättigten und/oder substituierten Reste im Kohlenstoffgerüst, die jeweils geradkettig oder verzweigt sein können. Beispiele sind Methylen, Ethylen, n- und i- Propylen und n-, s-, i-, t-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder lod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CF₂Cl, CHF₂, CH₂F, CF₃CF₂, CH₂FCHCI, CCI₃, CHCI₂, CH₂CH₂CI; Haloalkoxy ist z.B. OCF₃, OCHF₂, OCH₂F, CF₃CF₂O, OCH₂CF₃ und OCH₂CH₂CI; entsprechendes gilt für Haloalkenyl und andere durch Halogen substituierte Reste.

Aryl bedeutet ein mono-, bi- oder polycyclisches aromatisches System, beispielsweise Phenyl oder Naphthyl, vorzugsweise Phenyl.

Vor allem aus den Gründen der höheren herbiziden Wirkung, besseren Selektivität und/oder besseren Herstellbarkeit sind erfindungsgemäße Verbindungen der allgemeinen Formel (I) oder deren agrochemischen Salze oder quartären N-Derivate von besonderem Interesse, worin einzelne Reste eine der bereits genannten oder im Folgenden genannten bevorzugten Bedeutungen haben, oder insbesondere solche, worin eine oder mehrere der bereits genannten oder im folgenden genannten bevorzugten Bedeutungen kombiniert auftreten.

Die oben angeführten allgemeinen oder in Vorzugsbereichen angeführten Restedefinitionen gelten sowohl für die Endprodukte der allgemeinen Formel (I) als auch entsprechend für die die jeweils zur Herstellung benötigten Ausgangs- und Zwischenprodukte. Diese Restedefinitionen können untereinander, als auch zwischen den angegebenen bevorzugten Bereichen vertauscht werden.

Sofern Tautomere möglich sind, sind mit der beschriebenen Form alle möglichen tautomere Strukturen umfasst. Wie nachfolgend dargestellt, sind wenn zum Beispiel für R^{2a} und / oder R^{2b} = Hydroxy beschrieben ist, die möglichen Keto-Tautomeren ebenfalls erfasst.

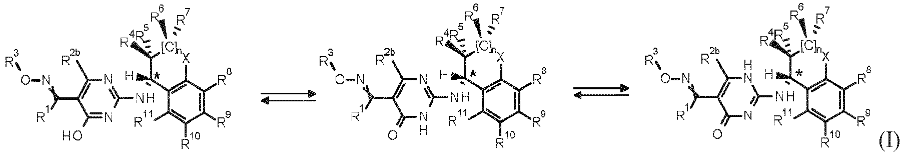

Die vorliegenden Verbindungen der allgemeinen Formel (I) weisen an der Bindestelle zum Aminopyrimidin ein chirales Kohlenstoffatom auf, welches in der unten dargestellten Struktur durch die Kennzeichnung (*) verdeutlicht ist:

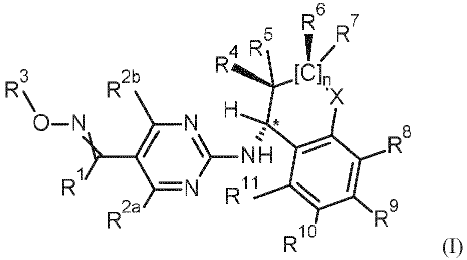

Gemäß den Regeln nach Cahn, Ingold und Prelog (CIP-Regeln) kann dieses Kohlenstoffatom sowohl eine (R)- als auch eine (S)-Konfiguration aufweisen.

Von der vorliegenden Erfindung werden Verbindungen der allgemeinen Formel (I) sowohl mit (S)- als auch mit (R)-Konfiguration erfasst, d.h., dass die vorliegende Erfindung die Verbindungen der allgemeinen Formel (I) erfasst, in welchen das betreffende Kohlenstoffatom
(1) eine (R)-Konfiguration; oder
(2) eine (S)-Konfiguration
   aufweist.
   Darüber hinaus werden im Rahmen der vorliegenden Erfindung auch
(3) beliebige Mischungen von Verbindungen der allgemeinen Formel (I), welche eine (R)-Konfiguration (Verbindungen der allgemeinen Formel (I-(R)) aufweisen, mit Verbindungen der allgemeinen Formel (I), welche eine (S)-Konfiguration (Verbindungen der allgemeinen Formel (I-S)) aufweisen, erfasst, wobei eine racemische Mischung der Verbindungen der allgemeinen Formel (I) mit (R)- und (S)-Konfiguration von der vorliegenden Erfindung ebenfalls umfasst ist.

Allerdings sind im Rahmen der vorliegenden Erfindung insbesondere Verbindungen der allgemeinen Formel (I) mit (R)-Konfiguration mit einer Selektivität von 60 bis 100%, vorzugsweise 80 bis 100%, insbesondere 90 bis 100%, ganz besonders 95 bis 100%, bevorzugt, wobei die jeweilige (R)-Verbindung mit einer Enantioselektivität von jeweils mehr als 50% ee, vorzugsweise 60 bis 100% ee, insbesondere 80 bis 100% ee, ganz besonders 90 bis 100% ee, meist bevorzugt 95 bis 100% ee, bezogen auf den Gesamtgehalt an betreffender (R)-Verbindung vorliegt.

Daher betrifft die vorliegende Erfindung insbesondere Verbindungen der allgemeinen Formel (I*), in welchen die stereochemische Konfiguration an dem mit (*) gekennzeichnet Kohlenstoffatom mit einer stereochemischen Reinheit von 60 bis 100 % (R), vorzugsweise 80 bis 100 % (R), insbesondere 90 bis 100 % (R), ganz besonders 95 bis 100 % (R), vorliegt.

Unter Berücksichtigung der Regel nach Cahn, Ingold und Prelog kann es an dem mit (*) gekennzeichneten Kohlenstoffatom auch zu einer Situation kommen, in welcher aufgrund der Priorität der jeweiligen Substituenten die (S)-Konfiguration am mit (*) gekennzeichneten Kohlenstoffatom bevorzugt ist. Dieses ist beispielweise dann der Fall, wenn die Reste R⁴ und/oder R⁵ einem C₁-C₆-Alkoxyrest entsprechen.

Daher sind im Rahmen der vorliegenden Erfindung insbesondere Verbindungen der allgemeinen Formel (I) bevorzugt, die in ihrer räumlichen Anordnung den jenigen Verbindungen der allgemeinen Formel (I) mit R⁴ und R⁵ =Wasserstoff mit (R)-Konfiguration entsprechen, mit einer Selektivität von 60 bis 100 %, vorzugsweise 80 bis 100 %, insbesondere 90 bis 100 %, ganz besonders 95 bis 100 %, bevorzugt, wobei die jeweilige (R)-analoge-Verbindung mit einer Enantioselektivität von jeweils mehr als 50 % ee, vorzugsweise 60 bis 100 % ee, insbesondere 80 bis 100 % ee, ganz besonders 90 bis 100 % ee, meist bevorzugt 95 bis 100 % ee, bezogen auf den Gesamtgehalt an betreffender (R)-analogen-Verbindung, vorliegt. Daher betrifft die vorliegende Erfindung insbesondere Verbindungen der allgemeinen Formel (I), in welchen die stereochemische Konfiguration an dem mit (*) gekennzeichnet Kohlenstoffatom mit einer stereochemischen Reinheit von 60 bis 100 % (R, bzw. analog-R), vorzugsweise 80 bis 100 % (R, bzw. analog-R), insbesondere 90 bis 100 % (R, bzw. analog-R), ganz besonders 95 bis 100 % (R, bzw. analog-R), vorliegt.

Insbesondere können die erfindungsgemäßen Verbindungen der allgemeinen Formel (I) noch weitere Chiralitätszentren an den mit (**) und (***) gekennzeichneten Kohlenstoffatomen aufweisen:

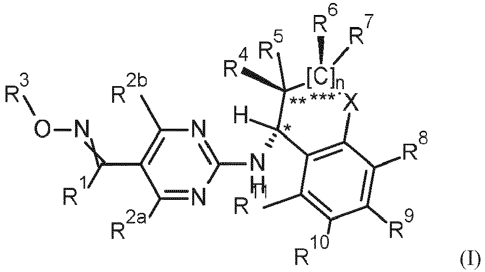

Im Rahmen der vorliegenden Erfindung sind beliebige stereochemische Konfigurationen an den mit (*), (**) und (***) gekennzeichneten Kohlenstoffatomen möglich:

| **Konfiguration Kohlenstoffatom (*)** | **Konfiguration Kohlenstoffatom (**)** | **Konfiguration Kohlenstoffatom (***)** |
|---|---|---|
| R | R | R |
| R | R | S |
| R | S | R |
| S | R | R |
| R | S | S |
| S | R | S |
| S | S | R |
| S | S | S |

Darüber hinaus können, je nach Wahl der jeweiligen Reste, weitere Stereoelemente in den erfindungsgemäßen Verbindungen der allgemeinen Formel (I) vorliegen.

Sind beispielsweise eine oder mehrere Alkenylgruppen vorhanden, so können Diastereomere (Z- und E-Isomere) auftreten.

Sind beispielsweise ein oder mehrere asymmetrische Kohlenstoffatome vorhanden, so können Enantiomere und Diastereomere auftreten.

Entsprechende Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfaßt werden, jedoch nicht mit ihrer spezifischen Stereoform angegeben sind und deren Gemische.

Die Doppelbindung zwischen dem Kohlenstoff- und dem Stickstoffatom in der Oxim-Gruppierung (C=N-O-R³) ist in den Formel der vorliegenden Erfindung X-artig dargestellt. Diese Darstellung bedeutet, dass der Rest -OR³ sowohl in die Richtung des Pyrimdins als auch in Richtung R¹ zeigen kann.

Im Rahmen der vorliegenden Erfindung sind sowohl solche Verbindungen erfasst, bei denen der Rest R³ räumlich in Richtung des Pyrimidin zeigt und auch solche, bei denen der Rest R³ räumlich vom Pyrimidin weg zeigt. Die Benennung der Substanzen erfolgt gemäß den Regeln nach Cahn, Ingold und Prelog (CIP-Regeln), dabei bezeichnet man die Verbindungen, bei denen die vorrangigen Substituenten relativ zur π-Ebene der C=N Einheit auf der gleichen Seite stehen mit Z (Zusammen) und diejenigen, bei denen die vorrangige Substituenten auf unterschiedlichen Seiten stehen mit E (Entgegen). Die Erfindung umfasst sowohl die jeweiligen angereicherten Substanzen als auch beliebige Mischungen davon.

Die Kombinationsmöglichkeiten der verschiedenen Substituenten der allgemeinen Formel (I) sind so zu verstehen, dass die allgemeinen Grundsätze des Aufbaus chemischer Verbindungen zu beachten sind, d.h. die Formel (I) nicht Verbindungen umfasst, von denen der Fachmann weiß, dass sie chemisch nicht möglich sind.

Im Folgenden werden in tabellarischer Form Beispiele der Verbindungen der allgemeinen Formel (I) wiedergegeben.

In den nachfolgenden Tabellen 1 und 2 werden die in Formel (I) allgemein definierten Substituenten spezifiziert. Dabei steht:
- "**St N H**³" für stereochemische Anordnung in der alpha-Position zum Aromaten des bicyclischen Amines am Kohlenstoffatom, an das NH und Wasserstoff gebunden sind, "St R⁴ R⁵" und "St R⁶ R⁷" steht analog für die Kohlenstoffatome an die die jeweiligen Substituenten gebunden sind,
- E/Z steht für die Anordnung des Restes OR³ in Bezug zum C=N Strukturelement,
- die Bindung der Substituenten ist jeweils links,
- sofern für X zwei Bindestellen angegeben sind, bindet die linke Bindung an den Aromaten und die rechte Bindung an den hydrierten Teil des bicyclischen Amins,
- ein Bindestrich "-" für eine direkte Bindung, und
- falls n=0 ist, enthält die Tabelle in dem entsprechenden Feld für R⁶ und R⁷ keinen Eintrag.

**Tabelle 1:**

| **Nr.** | **R¹** | **R^{2a}** | **R^{2b}** | **R³** | **E/Z** | **St N H** | **R⁴** | **R⁵** | **St R⁴ R⁵** | **R⁶** | **R⁷** | **n** | **St R⁶ R⁷** | **R⁸** | **R⁹** | **R¹⁰** | **R¹¹** | **X** |
|---|---|---|---|---|---|---|---|---|---|---|---|---|---|---|---|---|---|---|
| 1.1a | CH₃ | H | H | H | E | rac | H | H | | H | H | 2 | | CH₃ | H | CH₃ | H | - |
| 1.1b | CH₃ | H | H | H | Z | rac | H | H | | H | H | 2 | | CH₃ | H | CH₃ | H | - |
| 1.2a | CH₃ | H | H | H | E | R | H | H | | H | H | 2 | | H | H | H | H | - |
| 1.2b | CH₃ | H | H | H | Z | R | H | H | | H | H | 2 | | H | H | H | H | - |
| 1.3a | CH₃ | H | H | H | E | R | H | H | | H | H | 2 | | H | H | H | H | - |
| 1.3b | CH₃ | H | H | H | Z | R | H | H | | H | H | 2 | | H | H | H | H | - |
| 1.4a | CH₃ | H | H | H | E | R | H | H | | H | H | 1 | | H | H | H | H | O |
| 1.4b | CH₃ | H | H | H | Z | R | H | H | | H | H | 1 | | H | H | H | H | O |
| 1.5a | CH₃ | H | H | H | E | R | H | H | | H | H | 1 | | F | H | H | H | O |
| 1.5b | CH₃ | H | H | H | Z | R | H | H | | H | H | 1 | | F | H | H | H | O |
| 1.6a | CH₃ | H | H | H | E | R | H | H | | H | H | 1 | | H | H | H | H | O |
| 1.6b | CH₃ | H | H | H | Z | R | H | H | | H | H | 1 | | H | H | H | H | O |
| 1.7a | CH₃ | H | H | H | E | rac | H | H | | H | H | 1 | | CH₃ | H | H | H | O |
| 1.7b | CH₃ | H | H | H | Z | rac | H | H | | H | H | 1 | | CH₃ | H | H | H | O |
| 1.8a | CH₃ | H | H | H | E | rac | H | H | | H | H | 1 | | CH₃ | H | H | H | O |
| 1.8b | CH₃ | H | H | H | Z | rac | H | H | | H | H | 1 | | CH₃ | H | H | H | O |
| 1.9a | CH₂CH₃ | H | H | H | E | R | CH₃ | H | S | H | H | 1 | | H | H | H | H | - |
| 1.9b | CH₂CH₃ | H | H | H | Z | R | CH₃ | H | S | H | H | 1 | | H | H | H | H | - |
| 1.10a | CH₃ | H | H | H | E | R | CH₃ | H | S | H | H | 1 | | H | H | H | H | - |
| 1.10b | CH₃ | H | H | H | Z | R | CH₃ | H | S | H | H | 1 | | H | H | H | H | - |
| 1.11a | Cyclopropyl | H | H | H | E | R | CH₃ | H | S | H | H | 1 | | H | H | H | H | - |
| 1.11b | Cyclopropyl | H | H | H | Z | R | CH₃ | H | S | H | H | 1 | | H | H | H | H | - |
| 1.12a | CH₂CH₂CH₃ | H | H | H | E | R | CH₃ | H | S | H | H | 1 | | H | H | H | H | - |
| 1.12b | CH₂CH₂CH₃ | H | H | H | Z | R | CH₃ | H | S | H | H | 1 | | H | H | H | H | - |
| 1.13a | CH₃ | H | H | H | E | R | CH₃ | H | S | H | H | 1 | | H | H | H | H | - |
| 1.13b | CH₃ | H | H | H | Z | R | CH₃ | H | S | H | H | 1 | | H | H | H | H | - |
| 1.14a | CH₃ | H | H | H | E | R | H | H | | H | H | 1 | | H | H | H | H | - |
| 1.14b | CH₃ | H | H | H | Z | R | H | H | | H | H | 1 | | H | H | H | H | - |
| 1.15a | CH₃ | H | H | H | E | R | H | H | | H | H | 1 | | H | H | F | H | - |
| 1.15b | CH₃ | H | H | H | Z | R | H | H | | H | H | 1 | | H | H | F | H | - |
| 1.16a | CH₃ | H | H | H | E | R | H | H | | H | H | 1 | | H | H | Cl | H | - |
| 1.16b | CH₃ | H | H | H | Z | R | H | H | | H | H | 1 | | H | H | C1 | H | - |
| 1.17a | CH₃ | H | H | H | E | rac | H | H | | H | H | 1 | | H | OCH₃ | OCH₃ | H | - |
| 1.17b | CH₃ | H | H | H | z | rac | H | H | | H | H | 1 | | H | OCH₃ | OCH₃ | H | - |
| 1.18a | CH₃ | H | H | H | E | rac | H | H | | H | H | 1 | | H | H | CCH | H | - |
| 1.18b | CH₃ | H | H | H | Z | rac | H | H | | H | H | 1 | | H | H | CCH | H | - |
| 1.19a | CH₃ | H | H | H | E | rac | H | H | | H | H | 2 | | H | H | CH₃ | H | - |
| 1.19b | CH₃ | H | H | H | Z | rac | H | H | | H | H | 2 | | H | H | CH₃ | H | - |
| 1.20a | CH₃ | H | H | H | E | rac | CH₃ | H | rac | H | H | 2 | | H | H | H | H | - |
| 1.20b | CH₃ | H | H | H | Z | rac | CH₃ | H | rac | H | H | 2 | | H | H | H | H | - |
| 1.21a | CH₃ | H | H | H | E | rac | CH₃ | H | rac | H | H | 1 | | H | H | H | H | O |
| 1.21a | CH₃ | H | H | H | Z | rac | CH₃ | H | rac | H | H | 1 | | H | H | H | H | O |
| 1.22a | CH₃ | CH₃ | H | H | E | rac | H | H | rac | H | H | 2 | | H | H | H | H | - |
| 1.22b | CH₃ | CH₃ | H | H | Z | rac | H | H | rac | H | H | 2 | | H | H | H | H | - |
| 1.23a | CH₃ | CH₃ | H | H | E | rac | H | H | | H | H | 2 | | CH₃ | H | CH₃ | H | - |
| 1.23b | CH₃ | CH₃ | H | H | Z | rac | H | H | | H | H | 2 | | CH₃ | H | CH₃ | H | - |
| 1.24a | CH₃ | CH₃ | H | H | E | R | H | H | | H | H | 2 | | H | H | CH₃ | H | - |
| 1.24b | CH₃ | CH₃ | H | H | Z | R | H | H | | H | H | 2 | | H | H | CH₃ | H | - |
| 1.25a | CH₃ | CH₃ | H | H | E | R | CH₃ | H | S | H | H | 2 | | H | H | H | H | - |
| 1.25b | CH₃ | CH₃ | H | H | Z | R | CH₃ | H | S | H | H | 2 | | H | H | H | H | - |
| 1.26a | CH₃ | CH₃ | H | H | E | R | CH₃ | H | S | H | H | 1 | | H | H | CH₃ | H | - |
| 1.26b | CH₃ | CH₃ | H | H | Z | R | CH₃ | H | S | H | H | 1 | | H | H | CH₃ | H | - |
| 1.27a | CH₃ | CH₃ | H | H | E | R | CH₃ | H | S | H | H | 1 | | H | H | CH₃ | H | - |
| 1.27b | CH₃ | CH₃ | H | H | Z | R | CH₃ | H | S | H | H | 1 | | H | H | CH₃ | H | - |
| 1.28a | CH₂CH₂CH₃ | CH₃ | H | H | E | R | CH₃ | H | S | H | H | 1 | | H | H | CH₃ | H | - |
| 1.28b | CH₂CH₂CH₃ | CH₃ | H | H | Z | R | CH₃ | H | S | H | H | 1 | | H | H | CH₃ | H | - |
| 1.29a | Cyclobutyl | CH₃ | H | H | E | R | CH₃ | H | S | H | H | 1 | | H | H | CH₃ | H | - |
| 1.29b | Cyclobutyl | CH₃ | H | H | Z | R | CH₃ | H | S | H | H | 1 | | H | H | CH₃ | H | - |
| 1.30a | Cclopentyl | CH₃ | H | H | E | R | CH₃ | H | S | H | H | 1 | | H | H | CH₃ | H | - |
| 1.30b | Cclopentyl | CH₃ | H | H | Z | R | CH₃ | H | S | H | H | 1 | | H | H | CH₃ | H | - |
| ¹.31a | Cyclohexyl | CH₃ | H | H | E | R | CH₃ | H | S | H | H | 1 | | H | H | CH₃ | H | - |
| 1.31b | Cyclohexyl | CH₃ | H | H | Z | R | CH₃ | H | S | H | H | 1 | | H | H | CH₃ | H | - |
| 1.32a | CH₂-Cyclohexyl | CH₃ | H | H | E | R | CH₃ | H | S | H | H | 1 | | H | H | CH₃ | H | - |
| 1.32b | CH₂-Cyclohexyl | CH₃ | H | H | Z | R | CH₃ | H | S | H | H | 1 | | H | H | CH₃ | H | - |
| 1.33a | CH₂-Cyclopropyl | CH₃ | H | H | E | R | CH₃ | H | S | H | H | 1 | | H | H | CH₃ | H | - |
| 1.33b | CH₂-Cyclopropyl | CH₃ | H | H | Z | R | CH₃ | H | S | H | H | 1 | | H | H | CH₃ | H | - |
| 1.34a | Cyclobutyl | CH₃ | H | H | E | R | CH₃ | H | S | H | H | 1 | | F | H | CH₃ | H | O |
| 1.34b | Cyclobutyl | CH₃ | H | H | Z | R | CH₃ | H | S | H | H | 1 | | F | H | CH₃ | H | O |
| 1.35a | CH₃ | CH₃ | H | H | E | R | H | H | | H | H | 1 | | F | H | H | H | O |
| 1.35b | CH₃ | CH₃ | H | H | Z | R | H | H | | H | H | 1 | | F | H | H | H | O |
| 1.36a | CH₃ | CH₃ | H | H | E | R | CH₃ | H | S | H | H | 1 | | F | H | H | H | O |
| 1.36b | CH₃ | CH₃ | H | H | Z | R | CH₃ | H | S | H | H | 1 | | F | H | H | H | O |
| 1.37a | CH₃ | CH₃ | H | H | E | R | CH₃ | H | S | H | H | 2 | | F | H | H | H | - |
| 1.37b | CH₃ | CH₃ | H | H | Z | R | CH₃ | H | S | H | H | 2 | | F | H | H | H | - |
| 1.38a | CH₃ | CH₃ | H | H | E | R | H | H | | H | H | 2 | | F | H | H | H | - |
| 1.38b | CH₃ | CH₃ | H | H | Z | R | H | H | | H | H | 2 | | F | H | H | H | - |
| 1.39a | CH₃ | CH₃ | H | H | E | rac | H | H | | H | H | 2 | | F | H | H | H | - |
| 1.39b | CH₃ | CH₃ | H | H | Z | rac | H | H | | H | H | 2 | | F | H | H | H | - |
| 1.40a | CF₂CF₃ | CH₂CH₃ | H | H | E | R | CH₃ | H | S | H | H | 1 | | H | H | CH₃ | H | - |
| 1.40b | CH₂CH₃ | CH₂CH₃ | H | H | Z | R | CH₃ | H | S | H | H | 1 | | H | H | CH₃ | H | - |
| 1.41a | Cyclopropyl | Cyclo-propyl | H | H | E | R | CH₃ | H | S | H | H | 1 | | H | H | CH₃ | H | - |
| 1.41b | Cyclopropyl | Cyclo-propyl | H | H | Z | R | CH₃ | H | S | H | H | 1 | | H | H | CH₃ | H | - |
| 1.42a | CH₃ | CF₃ | H | H | E | R | CH₃ | H | S | H | H | 1 | | H | H | CH₃ | H | - |
| 1.42b | CH₃ | CF₃ | H | H | Z | R | CH₃ | H | S | H | H | 1 | | H | H | CH₃ | H | - |
| 1.43a | CH₃ | Cyclo-propyl | H | H | E | R | CH₃ | H | S | H | H | 1 | | H | H | CH₃ | H | - |
| 1.43b | CH₃ | Cyclo-propyl | H | H | Z | R | CH₃ | H | S | H | H | 1 | | H | H | CH₃ | H | - |
| 1.44a | CH₂CH₃ | CF₃ | H | H | E | R | CH₃ | H | S | H | H | 1 | | H | H | CH₃ | H | - |
| 1.44b | CH₂CH₃ | CF₃ | H | H | Z | R | CH₃ | H | S | H | H | 1 | | H | H | CH₃ | H | - |
| 1.45a | CH₃ | CH₃ | CH₃ | H | E | R | CH₃ | H | S | H | H | 1 | | H | H | CH₃ | H | - |
| 1.45b | CH₃ | CH₃ | CH₃ | H | Z | R | CH₃ | H | S | H | H | 1 | | H | H | CH₃ | H | - |
| 1.46a | CH₃ | CH₂OCH₃ | H | H | E | R | CH₃ | H | S | H | H | 1 | | H | H | CH₃ | H | - |
| 1.46b | CH₃ | CH₂OCH₃ | H | H | Z | R | CH₃ | H | S | H | H | 1 | | H | H | CH₃ | H | - |
| 1.47a | CH₃ | CH₂OCH₃ | H | H | E | R | CH₃ | H | S | H | H | 1 | | H | H | H | H | - |
| 1.47b | CH₃ | CH₂OCH₃ | H | H | Z | R | CH₃ | H | S | H | H | 1 | | H | H | H | H | - |
| 1.48a | CH₃ | CH₂OCH₃ | H | H | E | R | CH₃ | H | S | H | H | 2 | | H | H | CH₃ | H | - |
| 1.48b | CH₃ | CH₂OCH₃ | H | H | Z | R | CH₃ | H | S | H | H | 2 | | H | H | CH₃ | H | - |
| 1.49a | CH₃ | CH₂OCH₃ | CH₃ | H | E | R | CH₃ | H | S | H | H | 1 | | H | H | CH₃ | H | - |
| 1.49b | CH₃ | CH₂OCH₃ | CH₃ | H | Z | R | CH₃ | H | S | H | H | 1 | | H | H | CH₃ | H | - |
| 1.50a | CH₃ | Cyclo-propyl | CH₃ | H | E | R | CH₃ | H | S | H | H | 1 | | H | H | CH₃ | H | - |
| 1.50b | CH₃ | Cyclo-propyl | CH₃ | H | Z | R | CH₃ | H | S | H | H | 1 | | H | H | CH₃ | H | - |
| 1.51a | CF₃ | CH₂OCH₃ | H | H | E | R | CH₃ | H | S | H | H | 1 | | H | H | CH₃ | H | - |
| 1.51b | CF₃ | CH₂OCH₃ | H | H | Z | R | CH₃ | H | S | H | H | 1 | | H | H | CH₃ | H | - |
| 1.52a | CF₃ | CH₃ | H | H | E | R | CH₃ | H | S | H | H | 1 | | H | H | CH₃ | H | - |
| 1.52b | CF₃ | CH₃ | H | H | Z | R | CH₃ | H | S | H | H | 1 | | H | H | CH₃ | H | - |
| 1.53a | CF₃ | CH₂CH₃ | H | H | E | R | CH₃ | H | S | H | H | 1 | | H | H | CH₃ | H | - |
| 1.53b | CF₃ | CH₂CH₃ | H | H | Z | R | CH₃ | H | S | H | H | 1 | | H | H | CH₃ | H | - |
| 1.54a | CF₃ | CH₃ | CH₃ | H | E | R | CH₃ | H | S | H | H | 1 | | H | H | CH₃ | H | - |
| 1.54b | CF₃ | CH₃ | CH₃ | H | Z | R | CH₃ | H | S | H | H | 1 | | H | H | CH₃ | H | - |
| 1.55a | CF₃ | H | H | H | E | R | CH₃ | H | S | H | H | 1 | | H | H | CH₃ | H | - |
| 1.55b | CF₃ | H | H | H | Z | R | CH₃ | H | S | H | H | 1 | | H | H | CH₃ | H | - |
| 1.56a | CF₃ | CH₃ | H | H | E | R | H | H | | H | H | 2 | | F | H | H | H | - |
| 1.56b | CF₃ | CH₃ | H | H | Z | R | H | H | | H | H | 2 | | F | H | H | H | - |
| 1.57a | CF₃ | H | H | H | E | R | H | H | | H | H | 2 | | F | H | H | H | - |
| 1.57b | CF₃ | H | H | H | Z | R | H | H | | H | H | 2 | | F | H | H | H | - |
| 1.58a | CF₃ | CH₂OCH₃ | H | H | E | R | H | H | | H | H | 2 | | F | H | H | H | - |
| 1.58b | CF₃ | CH₂OCH₃ | H | H | Z | R | H | H | | H | H | 2 | | F | H | H | H | - |
| 1.59a | CF₃ | CH₂OCH₃ | H | H | E | R | H | H | | H | H | 1 | | F | H | H | H | O |
| 1.59b | CF₃ | CH₂OCH₃ | H | H | Z | R | H | H | | H | H | 1 | | F | H | H | H | O |
| 1.60a | CF₃ | CH₃ | H | H | E | R | H | H | | H | H | 1 | | F | H | H | H | - |
| 1.60b | CF₃ | CH₃ | H | H | Z | R | H | H | | H | H | 1 | | F | H | H | H | - |
| 1.61a | CF₃ | CH₃ | H | H | E | R | CH₃ | H | S | H | H | 1 | | F | H | H | H | - |
| 1.61b | CF₃ | CH₃ | H | H | Z | R | CH₃ | H | S | H | H | 1 | | F | H | H | H | - |
| 1.62a | CF₃ | H | H | H | E | R | CH₃ | H | S | H | H | 1 | | F | H | H | H | O |
| 1.62b | CF₃ | H | H | H | Z | R | CH₃ | H | S | H | H | 1 | | F | H | H | H | O |
| 1.63a | CH₃ | CH₃ | CH₃ | H | E | R | CH₃ | H | S | H | H | 1 | | F | H | H | H | O |
| 1.63b | CH₃ | CH₃ | CH₃ | H | Z | R | CH₃ | H | S | H | H | 1 | | F | H | H | H | O |
| 1.64a | CH₃ | CF₃ | H | H | E | R | H | H | | H | H | 1 | | F | H | H | H | O |
| 1.64b | CH₃ | CF₃ | H | H | Z | R | H | H | | H | H | 1 | | F | H | H | H | O |
| 1.65a | CH₃ | CH₃ | CH₃ | H | E | rac | H | H | | H | H | 1 | | F | H | H | H | S |
| 1.65b | CH₃ | CH₃ | CH₃ | H | Z | rac | H | H | | H | H | 1 | | F | H | H | H | S |
| 1.66a | CH₃ | CH₃ | H | H | E | rac | H | H | | H | H | 1 | | H | H | H | H | -NMe- |
| 1.66b | CH₃ | CH₃ | H | H | Z | rac | H | H | | H | H | 1 | | H | H | H | H | -NMe- |
| 1.67a | CH₃ | CH₃ | H | H | E | rac | H | H | | H | H | 1 | | H | H | H | H | -CHMe- |
| 1.67b | CH₃ | CH₃ | H | H | Z | rac | H | H | | H | H | 1 | | H | H | H | H | -CHMe- |
| 1.68a | CH₃ | CH₃ | H | H | E | rac | H | H | | H | H | 1 | | H | H | H | H | -C(=O)- |
| 1.68b | CH₃ | CH₃ | H | H | Z | rac | H | H | | H | H | 1 | | H | H | H | H | -C(=O)- |
| 1.69a | CH₃ | CH₃ | H | H | E | rac | H | H | | CH₃ | H | 1 | rac | H | H | H | H | O |
| 1.69b | CH₃ | CH₃ | H | H | Z | rac | H | H | | CH₃ | H | 1 | rac | H | H | H | H | O |
| 1.70a | CH₃ | CH₃ | H | H | E | rac | CH₃ | CH₃ | | H | H | 1 | | H | H | H | H | - |
| 1.70b | CH₃ | CH₃ | H | H | Z | rac | CH₃ | CH₃ | | H | H | 1 | | H | H | H | H | - |
| 1.71a | CH₃ | CH₃ | H | H | E | rac | H | H | | CH₃ | CH₃ | 1 | | H | H | H | H | -CH₂- |
| 1.71b | CH₃ | CH₃ | H | H | Z | rac | H | H | | CH₃ | CH₃ | 1 | | H | H | H | H | -CH₂- |
| 1.72a | CH₃ | CH₃ | H | H | E | rac | H | H | | H | H | 1 | | CH₃ | H | CH₃ | H | O |
| 1.72b | CH₃ | CH₃ | H | H | Z | rac | H | H | | H | H | 1 | | CH₃ | H | CH₃ | H | O |
| 1.73a | CH₂CH₃ | CH₃ | H | H | E | rac | H | H | | H | H | 2 | | CH₃ | H | CH₃ | H | - |
| 1.73b | CH₂CH₃ | CH₃ | H | H | Z | rac | H | H | | H | H | 2 | | CH₃ | H | CH₃ | H | - |
| 1.74a | CH₃ | CH₃ | H | H | E | rac | H | H | | | | 0 | | H | H | H | H | O |
| 1.74b | CH₃ | CH₃ | H | H | Z | rac | H | H | | | | 0 | | H | H | H | H | O |
| 1.75a | CH₃ | CH₃ | H | H | E | rac | CH₃ | H | rac | H | H | 1 | | CH₃ | H | CH₃ | H | - |
| 1.75b | CH₃ | CH₃ | H | H | Z | rac | CH₃ | H | rac | H | H | 1 | | CH₃ | H | CH₃ | H | - |
| 1.76a | CH₃ | H | H | H | E | rac | H | H | | H | H | 1 | | CH₃ | H | H | CH₃ | - |
| 1.76b | CH₃ | H | H | H | Z | rac | H | H | | H | H | 1 | | CH₃ | H | H | CH₃ | - |
| 1.77a | CH₃ | H | H | H | E | rac | H | H | | H | H | 1 | | Cl | H | H | H | - |
| 1.77b | CH₃ | H | H | H | Z | rac | H | H | | H | H | 1 | | Cl | H | H | H | - |
| 1.78a | CH₃ | H | H | H | E | rac | H | H | | H | H | 1 | | CH₃ | H | H | H | - |
| 1.78b | CH₃ | H | H | H | Z | rac | H | H | | H | H | 1 | | CH₃ | H | H | H | - |
| 1.79a | CH₃ | H | H | H | E | rac | H | H | | H | H | 1 | | H | H | I | H | - |
| 1.79b | CH₃ | H | H | H | Z | rac | H | H | | H | H | 1 | | H | H | I | H | - |
| 1.80a | CH₃ | H | H | H | E | rac | H | H | | H | H | 1 | | H | H | CN | H | - |
| 1.80b | CH₃ | H | H | H | Z | rac | H | H | | H | H | 1 | | H | H | CN | H | - |
| 1.81a | CH₃ | CH₃ | H | H | E | rac | H | H | | H | H | 2 | | CH₃ | CH₃ | CH₃ | H | - |
| 1.81b | CH₃ | CH₃ | H | H | Z | rac | H | H | | H | H | 2 | | CH₃ | CH₃ | CH₃ | H | - |
| 1.82a | CH₃ | CH₃ | H | H | E | R | H | H | | H | H | 2 | | H | H | H | H | - |
| 1.82b | CH₃ | CH₃ | H | H | Z | R | H | H | | H | H | 2 | | H | H | H | H | - |
| 1.83a | CH₃ | CH₃ | H | H | E | rac | H | H | | H | H | 1 | | H | H | H | H | - |
| 1.83b | CH₃ | CH₃ | H | H | Z | rac | H | H | | H | H | 1 | | H | H | H | H | - |
| 1.84a | CH₃ | H | H | H | E | R | H | H | | H | H | 2 | | CH₃ | H | CH₃ | H | - |
| 1.84b | CH₃ | H | H | H | Z | R | H | H | | H | H | 2 | | CH₃ | H | CH₃ | H | - |
| 1.85a | CH₂-CH₂-CH₂-CH₃ | H | H | H | E | R | CH₃ | H | S | H | H | 1 | | H | H | H | H | - |
| 1.85b | CH₂-CH₂-CH₂-CH₃ | H | H | H | Z | R | CH₃ | H | S | H | H | 1 | | H | H | H | H | - |
| 1.86a | CH₂-Cyclopropyl | H | H | H | E | R | CH₃ | H | S | H | H | 1 | | H | H | CH₃ | H | - |
| 1.86b | CH₂-Cyclopropyl | H | H | H | Z | R | CH₃ | H | S | H | H | 1 | | H | H | CH₃ | H | - |
| 1.87a | CH₃ | CH₃ | H | H | E | rac | H | H | | H | H | 1 | | CH₃ | H | F | H | O |
| 1.87b | CH₃ | CH₃ | H | H | Z | rac | H | H | | H | H | 1 | | CH₃ | H | F | H | O |
| 1.88a | CH₃ | CH₃ | H | H | E | R | H | H | | H | H | 2 | | H | F | H | H | - |
| 1.88b | CH₃ | CH₃ | H | H | Z | R | H | H | | H | H | 2 | | H | F | H | H | - |
| 1.89a | CH₃ | CH₃ | H | H | E | R | H | H | | H | H | 1 | | H | H | H | H | - |
| 1.89b | CH₃ | CH₃ | H | H | Z | R | H | H | | H | H | 1 | | H | H | H | H | |
| 1.90a | CH₃ | H | H | H | E | R | H | H | | H | H | 1 | | F | H | H | H | - |
| 1.90b | CH₃ | H | H | H | Z | R | H | H | | H | H | 1 | | F | H | H | H | - |
| 1.91a | CH₃ | CH₃ | H | H | E | rac | H | H | | H | H | 1 | | H | H | i-prop | H | - |
| 1.91b | CH₃ | CH₃ | H | H | Z | rac | H | H | | H | H | 1 | | H | H | i-prop | H | - |
| 1.92a | CH₃ | CH₃ | H | H | E | rac | H | H | | H | H | 1 | | H | OCH₃ | H | H | - |
| 1.92b | CH₃ | CH₃ | H | H | Z | rac | H | H | | H | H | 1 | | H | OCH₃ | H | H | - |
| 1.93a | CH₃ | CH₃ | H | H | E | rac | H | H | | H | H | 1 | | CH₃ | CH₃ | H | H | O |
| 1.93b | CH₃ | CH₃ | H | H | Z | rac | H | H | | H | H | 1 | | CH₃ | CH₃ | H | H | O |
| 1.94a | CH₃ | CH₃ | H | H | E | rac | H | H | | H | H | 1 | | H | H | i-prop | H | O |
| 1.94b | CH₃ | CH₃ | H | H | Z | rac | H | H | | H | H | 1 | | H | H | i-prop | H | O |
| 1.95a | CH₃ | H | H | H | E | R | H | H | | H | H | 1 | | CH₃ | H | CH₃ | H | - |
| 1.95b | CH₃ | H | H | H | Z | R | H | H | | H | H | 1 | | CH₃ | H | CH₃ | H | - |
| 1.96a | CH₃ | CH₃ | H | H | E | rac | H | H | | CH₃ | CH₃ | 1 | | H | OCH₃ | H | H | O |
| 1.96b | CH₃ | CH₃ | H | H | Z | rac | H | H | | CH₃ | CH₃ | 1 | | H | OCH₃ | H | H | O |
| 1.97a | CH₃ | CH₃ | H | CH₃ | E | R | CH₃ | H | S | H | H | 1 | | H | H | CH₃ | H | - |
| 1.97b | CH₃ | CH₃ | H | CH₃ | Z | R | CH₃ | H | S | H | H | 1 | | H | H | CH₃ | H | - |
| 1.98a | H | CH₃ | H | CH₃ | E | R | CH₃ | H | S | H | H | 1 | | H | H | CH₃ | H | - |
| 1.98b | H | CH₃ | H | CH₃ | Z | R | CH₃ | H | S | H | H | 1 | | H | H | CH₃ | H | - |
| 1.99a | H | CH₃ | H | CH₂C≡CH | E | R | CH₃ | H | S | H | H | 1 | | H | H | CH₃ | H | - |
| 1.99b | H | CH₃ | H | CH₂C≡CH | Z | R | CH₃ | H | S | H | H | 1 | | H | H | CH₃ | H | - |
| 1.100a | H | CH₃ | H | CH₂C≡CCH₃ | E | R | CH₃ | H | S | H | H | 1 | | H | H | CH₃ | H | - |
| 1.100b | H | CH₃ | H | CH₂C≡CCH₃ | Z | R | CH₃ | H | S | H | H | 1 | | H | H | CH₃ | H | - |
| 1.101a | H | CH₃ | H | -Ph-4-F | E | R | CH₃ | H | S | H | H | 1 | | H | H | CH₃ | H | - |
| 1.101b | H | CH₃ | H | -Ph-4-F | Z | R | CH₃ | H | S | H | H | 1 | | H | H | CH₃ | H | - |
| 1.102a | H | CH₃ | H | CH₂CH(CH₃)₂ | E | R | CH₃ | H | S | H | H | 1 | | H | H | CH₃ | H | - |
| 1.102b | H | CH₃ | H | CH₂CH(CH₃)₂ | Z | R | CH₃ | H | S | H | H | 1 | | H | H | CH₃ | H | - |
| 1.103a | H | CH₃ | H | CH₂CH₃ | E | R | CH₃ | H | S | H | H | 1 | | H | H | CH₃ | H | - |
| 1.103b | H | CH₃ | H | CH₂CH₃ | Z | R | CH₃ | H | S | H | H | 1 | | H | H | CH₃ | H | - |
| 1.104a | H | CH₃ | H | CH(CH₃)₂ | E | R | CH₃ | H | S | H | H | 1 | | H | H | CH₃ | H | - |
| 1.104b | H | CH₃ | H | CH(CH₃)₂ | Z | R | CH₃ | H | S | H | H | 1 | | H | H | CH₃ | H | - |
| 1.105a | H | CH₃ | H | CH₂Ph-3-Cl | E | R | CH₃ | H | S | H | H | 1 | | H | H | CH₃ | H | - |
| 1.105b | H | CH₃ | H | CH₂Ph-3-Cl | Z | R | CH₃ | H | S | H | H | 1 | | H | H | CH₃ | H | - |
| 1.106a | H | CH₃ | H | CH₂Py-3yl-2-Cl | E | R | CH3 | H | S | H | H | 1 | | H | H | CH₃ | H | - |
| 1.106b | H | CH₃ | H | CH₂Py-3yl-2-Cl | Z | R | CH₃ | H | S | H | H | 1 | | H | H | CH₃ | H | - |
| 1.107a | H | CH₃ | H | CH₂Ph-4-CH₃ | E | R | CH₃ | H | S | H | H | 1 | | H | H | CH₃ | H | - |
| 1.107b | H | CH₃ | H | CH₂Ph-4-CH₃ | Z | R | CH₃ | H | S | H | H | 1 | | H | H | CH₃ | H | - |
| 1.108a | H | CH₃ | H | CH₂CH₂Ph | E | R | CH₃ | H | S | H | H | 1 | | H | H | CH₃ | H | - |
| 1.108b | H | CH₃ | H | CH₂CH₂Ph | Z | R | CH₃ | H | S | H | H | 1 | | H | H | CH₃ | H | - |
| 1.109a | H | CH₃ | H | CH₂CH₂Ph-3-Cl | E | R | CH₃ | H | S | H | H | 1 | | H | H | CH₃ | H | - |
| 1.109b | H | CH₃ | H | CH₂CH₂Ph-3-Cl | Z | R | CH₃ | H | S | H | H | 1 | | H | H | CH₃ | H | - |
| 1.110a | H | CH₃ | H | CH₂Ph-3,5-Cl | E | R | CH₃ | H | S | H | H | 1 | | H | H | CH₃ | H | - |
| 1.110b | H | CH₃ | H | CH₂Ph-3,5-Cl | Z | R | CH₃ | H | S | H | H | 1 | | H | H | CH₃ | H | - |
| 1.111a | H | CH₃ | H | C(CH₃)₃ | E | R | CH₃ | H | S | H | H | 1 | | H | H | CH₃ | H | - |
| 1.111 b | H | CH₃ | H | C(CH₃)₃ | Z | R | CH₃ | H | S | H | H | 1 | | H | H | CH₃ | H | - |
| 1.112a | H | CH₃ | H | C(CH₃)₂COOCH₃ | E | R | CH₃ | H | S | H | H | 1 | | H | H | CH₃ | H | - |
| 1.112b | H | CH₃ | H | C(CH₃)₂COOCH₃ | Z | R | CH₃ | H | S | H | H | 1 | | H | H | CH₃ | H | - |
| 1.113a | CH₂CH₃ | CH₂CH₃ | H | CH₃ | E | R | CH₃ | H | S | H | H | 1 | | H | H | CH₃ | H | - |
| 1.113b | CH₂CH₃ | CH₂CH₃ | H | CH₃ | Z | R | CH₃ | H | S | H | H | 1 | | H | H | CH₃ | H | - |
| 1.114a | CH₂CH₃ | CH₂CH₃ | H | CH₂CH₃ | E | R | CH₃ | H | S | H | H | 1 | | H | H | CH₃ | H | - |
| 1.114b | CH₂CH₃ | CH₂CH₃ | H | CH₂CH₃ | Z | R | CH₃ | H | S | H | H | 1 | | H | H | CH₃ | H | - |
| 1.115a | CH₂CH₃ | CH₂CH₃ | H | CH₂-4Cl-Ph | E | R | CH₃ | H | S | H | H | 1 | | H | H | CH₃ | H | - |
| 1.115b | CH₂CH₃ | CH₂CH₃ | H | CH₂-4Cl-Ph | Z | R | CH₃ | H | S | H | H | 1 | | H | H | CH₃ | H | - |
| 1.116a | CH₂CH₃ | CH₂CH₃ | H | CH₂-4MeO-Ph | E | R | CH₃ | H | S | H | H | 1 | | H | H | CH₃ | H | - |
| 1.116 b | CH₂CH₃ | CH₂CH₃ | H | CH₂-4MeO-Ph | Z | R | CH₃ | H | S | H | H | 1 | | H | H | CH₃ | H | - |
| 1.117a | CH₂CH₃ | CH₂CH₃ | H | CH₂C≡CH | E | R | CH₃ | H | S | H | H | 1 | | H | H | CH₃ | H | - |
| 1.117b | CH₂CH₃ | CH₂CH₃ | H | CH₂C≡CH | Z | R | CH₃ | H | S | H | H | 1 | | H | H | CH₃ | H | - |
| 1.118a | CH₂CH₃ | CH₂CH₃ | H | CH₂C(≡O)OCH₃ | E | R | CH₃ | H | S | H | H | 1 | | H | H | CH₃ | H | - |
| 1.118b | CH₂CH₃ | CH₂CH₃ | H | CH₂C(=O)OCH₃ | Z | R | CH₃ | H | S | H | H | 1 | | H | H | CH₃ | H | - |
| 1.119a | CH₃ | CH₃ | H | H | E | R | H | H | | H | H | 2 | | H | H | F | H | - |
| 1.119b | CH₃ | CH₃ | H | H | Z | R | H | H | | H | H | 2 | | H | H | F | H | - |
| 1.120a | CH₃ | CH₃ | H | CH₃ | E | R | H | H | | H | H | 2 | | H | H | F | H | - |
| 1.120b | CH₃ | CH₃ | H | CH₃ | Z | R | H | H | | H | H | 2 | | H | H | F | H | - |
| 1.121a | CH₃ | CHFCH₃ | H | H | E | R | CH₃ | H | S | H | H | 1 | | H | H | CH₃ | H | - |
| 1.121b | CH₃ | CHFCH₃ | H | H | Z | R | CH₃ | H | S | H | H | 1 | | H | H | CH₃ | H | - |
| 1.122a | CH₃ | CHFCH₃ | H | CH₃ | E | R | CH₃ | H | S | H | H | 1 | | H | H | CH₃ | H | - |
| 1.122b | CH₃ | CHFCH₃ | H | CH₃ | Z | R | CH₃ | H | S | H | H | 1 | | H | H | CH₃ | H | - |
| 1.123a | CH₃ | CClF₂ | H | H | E | R | CH₃ | H | S | H | H | 1 | | H | H | CH₃ | H | - |
| 1.123b | CH₃ | CClF₂ | H | H | Z | R | CH₃ | H | S | H | H | 1 | | H | H | CH₃ | H | - |
| 1.124a | CH₃ | CF(CH₃)₂ | H | H | E | R | CH₃ | H | S | H | H | 1 | | H | H | CH₃ | H | - |
| 1.124b | CH₃ | CF(CH₃)₂ | H | H | Z | R | CH₃ | H | S | H | H | 1 | | H | H | CH₃ | H | - |
| 1.125a | CH₃ | CF(CH_{S})₂ | H | CH₃ | E | R | CH₃ | H | S | H | H | 1 | | H | H | CH₃ | H | - |
| 1.125b | CH₃ | CF(CH₃)₂ | H | CH₃ | Z | R | CH₃ | H | S | H | H | 1 | | H | H | CH₃ | H | - |
| 1.126a | CH₃ | CF₂H | H | H | E | R | CH₃ | H | S | H | H | 1 | | H | H | CH₃ | H | - |
| 1.126b | CH₃ | CF₂H | H | H | Z | R | CH₃ | H | S | H | H | 1 | | H | H | CH₃ | H | - |
| 1.127a | CH₃ | CF₂H | H | CH₃ | E | R | CH₃ | H | S | H | H | 1 | | H | H | CH₃ | H | - |
| 1.127b | CH₃ | CF₂H | H | CH₃ | Z | R | CH₃ | H | S | H | H | 1 | | H | H | CH₃ | H | - |

**Tabelle 2:**

| **Nr.** | **R¹** | **R^{2a}** | **R^{2b}** | **R³** | **E/Z** | **St N H** | **R⁴** | **R⁵** | **St R⁴ R⁵** | **R⁶** | **R⁷** | **N** | **St R⁶ R⁷** | **R⁸** | **R⁹** | **R¹⁰** | **R¹¹** | **X** |
|---|---|---|---|---|---|---|---|---|---|---|---|---|---|---|---|---|---|---|
| 2.1a | -CH₂-Cl₂-CH₂- | | H | H | E | rac | H | H | | H | H | 2 | | CH₃ | H | CH₃ | H | - |
| 2.1b | -CH₂-CH₂-CH₂- | | H | H | Z | rac | H | H | | H | H | 2 | | CH₃ | H | CH₃ | H | - |
| 2.2a | -CH₂-CHMe-CH₂- | | H | H | E | rac | H | H | | H | H | 2 | | CH₃ | H | CH₃ | H | - |
| 2.2b | -CH₂-CHMe-CH₂- | | H | H | Z | rac | H | H | | H | H | 2 | | CH₃ | H | CH₃ | H | - |
| 2.3a | -CH₂-CHEt-CH₂- | | H | H | E | rac | H | H | | H | H | 2 | | CH₃ | H | CH₃ | H | - |
| 2.3b | -CH₂-CHEt-CH₂- | | H | H | Z | rac | H | H | | H | H | 2 | | CH₃ | H | CH₃ | H | - |
| 2.4a | -CH₂-CMe₂-CH₂- | | H | H | E | rac | H | H | | H | H | 2 | | CH₃ | H | CH₃ | H | - |
| 2.4b | -CH₂-CMe₂-CH₂- | | H | H | Z | rac | H | H | | H | H | 2 | | CH₃ | H | CH₃ | H | - |
| 2.5a | -CH₂-O-CH₂- | | H | H | E | rac | H | H | | H | H | 2 | | CH₃ | H | CH₃ | H | - |
| 2.5b | -CH₂-O-CH₂- | | H | H | Z | rac | H | H | | H | H | 2 | | CH₃ | H | CH₃ | H | - |
| 2.6a | -CH₂-CH(CF₃)-CH₂- | | H | H | E | rac | H | H | | H | H | 2 | | CH₃ | H | CH₃ | H | - |
| 2.6b | -CH₂-CH(CF₃)-CH₂- | | H | H | Z | rac | H | H | | H | H | 2 | | CH₃ | H | CH₃ | H | - |
| 2.7a | -CH₂-CH(2-F-Ph)-CH₂- | | H | H | E | rac | H | H | | H | H | 2 | | CH₃ | H | CH₃ | H | - |
| 2.7b | -CH₂-CH(2-F-Ph)-CH₂- | | H | H | Z | rac | H | H | | H | H | 2 | | CH₃ | H | CH₃ | H | - |
| 2.8a | -CH₂-CH(2,4-di-F-Ph)-CH₂- | | H | H | E | rac | H | H | | H | H | 2 | | CH₃ | H | CH₃ | H | - |
| 2.8b | -CH₂-CH(2,4-di-F-Ph)-CH₂- | | H | H | Z | rac | H | H | | H | H | 2 | | CH₃ | H | CH₃ | H | - |
| 2.9a | -CH₂-CH(4-F-Ph)-CH₂- | | H | H | E | rac | H | H | | H | H | 2 | | CH₃ | H | CH₃ | H | - |
| 2.9b | -CH₂-CH(4-F-Ph)-CH₂- | | H | H | Z | rac | H | H | | H | H | 2 | | CH₃ | H | CH₃ | H | - |
| 2.10a | -CH₂-CH₂-CH₂-CH₂- | | H | H | E | rac | H | H | | H | H | 2 | | CH₃ | H | CH₃ | H | - |
| 2.10b | -CH₂-CH₂-CH₂-CH₂- | | H | H | Z | rac | H | H | | H | H | 2 | | CH₃ | H | CH₃ | H | - |
| 2.11a | -CH₂-CH₂-CH₂- | | CH₃ | H | E | rac | H | H | | H | H | 2 | | CH₃ | H | CH₃ | H | - |
| 2.11b | -CH₂-CH₂-CH₂- | | CH₃ | H | Z | rac | H | H | | H | H | 2 | | CH₃ | H | CH₃ | H | - |
| 2.12a | -CH₂-CH₂-CH₂- | | CF₃ | H | E | rac | H | H | | H | H | 2 | | CH₃ | H | CH₃ | H | - |
| 2.12b | -CH₂-CH₂-CH₂- | | CF₃ | H | Z | rac | H | H | | H | H | 2 | | CH₃ | H | CH₃ | H | - |
| 2.13a | -CH₂-CH₂-CH₂- | | H | H | E | R | H | H | | H | H | 2 | | F | H | H | H | - |
| 2.13b | -CH₂-CH₂-CH₂- | | H | H | Z | R | H | H | | H | H | 2 | | F | H | H | H | - |
| 2.14a | -CH₂-CHMe-CH₂- | | H | H | E | R | H | H | | H | H | 2 | | F | H | H | H | - |
| 2.14b | -CH₂-CHMe-CH₂- | | H | H | Z | R | H | H | | H | H | 2 | | F | H | H | H | - |
| 2.15a | -CH₂-CMe₂-CH₂- | | H | H | E | R | H | H | | H | H | 2 | | F | H | H | H | - |
| 2.15b | -CH₂-CMe₂-CH₂- | | H | H | Z | R | H | H | | H | H | 2 | | F | H | H | H | - |
| 2.16a | -CH₂-CH(CF₃)-CH₂- | | H | H | E | R | H | H | | H | H | 2 | | F | H | H | H | - |
| 2.16b | -CH₂-CH(CF₃)-CH₂- | | H | H | Z | R | H | H | | H | H | 2 | | F | H | H | H | - |
| 2.17a | -CH₂-O-CH₂- | | H | H | E | R | H | H | | H | H | 2 | | F | H | H | H | - |
| 2.17b | -CH₂-O-CH₂- | | H | H | Z | R | H | H | | H | H | 2 | | F | H | H | H | - |
| 2.18a | -CH₂-CH(2-F-Ph)-CH₂- | | H | H | E | R | H | H | | H | H | 2 | | F | H | H | H | - |
| 2.18b | -CH₂-CH(2-F-Ph)-CH₂- | | H | H | Z | R | H | H | | H | H | 2 | | F | H | H | H | - |
| 2.19a | -CH₂-CH₂-CH₂- | | H | H | E | R | H | H | | H | H | 1 | | F | H | H | H | O |
| 2.19b | -CH₂-CH₂-CH₂- | | H | H | Z | R | H | H | | H | H | 1 | | F | H | H | H | O |
| 2.20a | -CH₂-CH₂-CH₂- | | H | H | E | R | H | H | | H | H | 1 | | F | H | H | H | S |
| 2.20b | -CH₂-CH₂-CH₂- | | H | H | Z | R | H | H | | H | H | 1 | | F | H | H | H | S |
| 2.21a | -CH₂-CMe₂-CH₂- | | H | H | E | R | H | H | | H | H | 1 | | F | H | H | H | O |
| 2.21b | -CH₂-CMe₂-CH₂- | | H | H | Z | R | H | H | | H | H | 1 | | F | H | H | H | O |
| 2.22a | -CH₂-CH(4-F-Ph)-CH₂- | | H | H | E | R | H | H | | H | H | 2 | | F | H | H | H | - |
| 2.22b | -CH₂-CH(4-F-Ph)-CH₂- | | H | H | Z | R | H | H | | H | H | 2 | | F | H | H | H | - |
| 2.230a | -CH₂-CH₂-CH₂- | | H | H | E | R | H | H | | H | H | 2 | | H | H | H | H | - |
| 2.23a | -CH₂-CH₂-CH₂- | | H | H | Z | R | H | H | | H | H | 2 | | H | H | H | H | - |
| 2.23b | -CH₂-O-CH₂- | | H | H | E | R | H | H | | H | H | 2 | | H | H | H | H | - |
| 2.24a | -CH₂-O-CH₂- | | H | H | Z | R | H | H | | H | H | 2 | | H | H | H | H | - |
| 2.24b | -CH₂-CMe₂-CH₂- | | H | H | E | R | H | H | | H | H | 2 | | H | H | H | H | - |
| 2.25a | -CH₂-CMe₂-CFl₂- | | H | H | Z | R | H | H | | H | H | 2 | | H | H | H | H | - |
| 2.25b | -CH₂-CHMe-CH₂- | | H | H | E | R | H | H | | H | H | 2 | | H | H | H | H | - |
| 2.26a | -CH₂-CHMe-CH₂- | | H | H | Z | R | H | H | | H | H | 2 | | H | H | H | H | - |
| 2.26b | -CH₂-CH₂-CH₂- | | H | H | E | R | H | H | | H | H | 1 | | H | H | H | H | O |
| 2.27a | -CH₂-CH₂-CH₂- | | H | H | Z | R | H | H | | H | H | 1 | | H | H | H | H | O |
| 2.27b | -CH₂-O-CH₂- | | H | H | E | R | H | H | | H | H | 1 | | H | H | H | H | 0 |
| 2.28a | -CH₂-O-CH₂- | | H | H | Z | R | H | H | | H | H | 1 | | H | H | H | H | O |
| 2.28b | -CH₂-CH(CF₃)-CH₂- | | H | H | E | R | H | H | | H | H | 1 | | H | H | H | H | O |
| 2.29a | -CH₂-CH(CF₃)-CH₂- | | H | H | Z | R | H | H | | H | H | 1 | | H | H | H | H | O |
| 2.29b | -CH₂-CH(4-F-Ph)-CH₂- | | H | H | E | R | H | H | | H | H | 1 | | H | H | H | H | O |
| 2.30b | -CHz-CH(4-F-Ph)-CHz- | | H | H | Z | R | H | H | | H | H | 1 | | H | H | H | H | O |
| 2.31a | -CH₂-CH₂-CH₂- | | CH₃ | H | E | R | H | H | | H | H | 2 | | H | H | H | H | - |
| 2.31b | -CH₂-CH₂-CH₂- | | CH₃ | H | Z | R | H | H | | H | H | 2 | | H | H | H | H | - |
| 2.32a | -CH₂-CH₂-CH₂- | | CH₃ | H | E | R | H | H | | H | H | 1 | | H | H | H | H | O |
| 2.32b | -CH₂-CH₂-CH₂- | | CH₃ | H | Z | R | H | H | | H | H | 1 | | H | H | H | H | O |
| 2.33a | -CH₂-CH₂-CH₂- | | CF₃ | H | E | R | H | H | | H | H | 2 | | H | H | H | H | - |
| 2.33b | -CH₂-CH₂-CH₂- | | CF₃ | H | Z | R | H | H | | H | H | 2 | | H | H | H | H | - |
| 2.34a | -CH₂-CH₂-CH₂- | | H | H | E | R | H | H | | H | H | 2 | | H | H | CH₃ | H | - |
| 2.34b | -CH₂-CH₂-CH₂- | | H | H | Z | R | H | H | | H | H | 2 | | H | H | CH₃ | H | - |
| 2.35a | -CH₂-CH₂-CH₂- | | H | H | E | R | CH₃ | H | rac | H | H | 2 | | H | H | H | H | - |
| 2.35b | -CH₂-CH₂-CH₂- | | H | H | Z | R | CH₃ | H | rac | H | H | 2 | | H | H | H | H | - |
| 2.36a | -CH₂-CH₂-CH₂- | | H | H | E | R | H | H | | H | H | 1 | | CH₃ | H | CH₃ | H | -C(=O)- |
| 2.36b | -CH₂-CH₂-CH₂- | | H | H | Z | R | H | H | | H | H | 1 | | CH₃ | H | CH₃ | H | -C(=O)- |
| 2.37a | -CH₂-CH₂-CH₂- | | H | H | E | rac | H | H | | H | H | 1 | | H | H | H | H | -NMe- |
| 2.37b | -CH₂-CH₂-CH₂- | | H | H | Z | rac | H | H | | H | H | 1 | | H | H | H | H | -NMe- |
| 2.38a | -CH₂-CH₂-CH₂- | | H | H | E | R | H | H | | H | H | 1 | | H | H | H | H | - |
| 2.38b | -CH₂-CH₂-CH₂- | | H | H | Z | R | H | H | | H | H | 1 | | H | H | H | H | - |
| 2.39a | -CH₂-CHMe-CH₂- | | H | H | E | R | H | H | | H | H | 1 | | H | H | H | H | - |
| 2.39b | -CH₂-CHMe-CH₂- | | H | H | Z | R | H | H | | H | H | 1 | | H | H | H | H | - |
| 2.40a | -CH₂-CH(CF₃)-CH₂- | | H | H | E | R | H | H | | H | H | 1 | | H | H | H | H | - |
| 2.40b | -CH₂-CH(CF₃)-CH₂- | | H | H | Z | R | H | H | | H | H | 1 | | H | H | H | H | - |
| 2.41a | -CH₂-CH₂-CH₂- | | H | H | E | R | H | H | | H | H | 1 | | H | H | CH₃ | H | - |
| 2.41b | -CH₂-CH₂-CH₂- | | H | H | Z | R | H | H | | H | H | 1 | | H | H | CH₃ | H | - |
| 2.42a | -CH₂-CH₂-CH₂- | | H | H | E | rac | H | H | | H | H | 1 | | H | H | F | H | - |
| 2.42b | -CH₂-CH₂-CH₂- | | H | H | Z | rac | H | H | | H | H | 1 | | H | H | F | H | - |
| 2.43a | -CH₂-CH₂-CH₂- | | H | H | E | R | CH₃ | H | S | H | H | 1 | | H | H | CH₃ | H | - |
| 2.43b | -CH₂-CH₂-CH₂- | | H | H | Z | R | CH₃ | H | S | H | H | 1 | | H | H | CH₃ | H | - |
| 2.44a | -CH₂-CHMe-CH₂- | | H | H | E | R | CH₃ | H | S | H | H | 1 | | H | H | CH₃ | H | - |
| 2.44b | -CH₂-CHMe-CH₂- | | H | H | Z | R | CH₃ | H | S | H | H | 1 | | H | H | CH₃ | H | - |
| 2.45a | -CH₂-CMe₂-CH₂- | | H | H | E | R | CH₃ | H | S | H | H | 1 | | H | H | CH₃ | H | - |
| 2.45b | -CH₂-CMe₂-CH₂- | | H | H | Z | R | CH₃ | H | S | H | H | 1 | | H | H | CH₃ | H | - |
| 2.46a | -CH₂-CH(CF₃)-CH₂- | | H | H | E | R | CH₃ | H | S | H | H | 1 | | H | H | CH₃ | H | - |
| 2.46b | -CH₂-CH(CF₃)-CH₂- | | H | H | Z | R | CH₃ | H | s | H | H | 1 | | H | H | CH₃ | H | - |
| 2.47a | -CH₂-C(=O)-CH₂- | | H | H | E | R | CH₃ | H | S | H | H | 1 | | H | H | CH₃ | H | - |
| 2.47b | -CH₂-C(=O)-CH₂- | | H | H | Z | R | CH₃ | H | S | H | H | 1 | | H | H | CH₃ | H | - |
| 2.48a | -CH₂-CH(Cycloprop)-CH₂- | | H | H | E | R | CH₃ | H | S | H | H | 1 | | H | H | CH₃ | H | - |
| 2.48b | -CH₂-CH(Cycloprop)-CH₂- | | H | H | Z | R | CH₃ | H | S | H | H | 1 | | H | H | CH₃ | H | - |
| 2.49a | -CH₂-CHPh-CH₂- | | H | H | E | R | CH₃ | H | S | H | H | 1 | | H | H | CH₃ | H | - |
| 2.49b | -CH₂-CHPh-CH₂- | | H | H | Z | R | CH₃ | H | S | H | H | 1 | | H | H | CH₃ | H | - |
| 2.50a | -CH₂-CH(2-F-Ph)-CH₂- | | H | H | E | R | CH₃ | H | S | H | H | 1 | | H | H | CH₃ | H | - |
| 2.50b | -CH₂-CH(2-F-Ph)-CH₂- | | H | H | Z | R | CH₃ | H | S | H | H | 1 | | H | H | CH₃ | H | - |
| 2.51a | -CH₂-CH(2,4-di-F-Ph)-CH₂- | | H | H | E | R | CH₃ | H | S | H | H | 1 | | H | H | CH₃ | H | - |
| 2.51b | -CH₂-CH(2,4-di-F-Ph)-CH₂- | | H | H | Z | R | CH₃ | H | S | H | H | 1 | | H | H | CH₃ | H | - |
| 2.52a | -CH₂-CH(4-F-Ph)-CH₂- | | H | H | E | R | CH₃ | H | S | H | H | 1 | | H | H | CH₃ | H | - |
| 2.52b | -CH₂-CH(4-F-Ph)-CH₂- | | H | H | Z | R | CH₃ | H | S | H | H | 1 | | H | H | CH₃ | H | - |
| 2.53a | -CH₂-CH₂-CH₂- | | CH₂CH₃ | H | E | R | CH₃ | H | S | H | H | 1 | | H | H | CH₃ | H | - |
| 2.53b | -CH₂-CH₂-CH₂- | | CH₂CH₃ | H | Z | R | CH₃ | H | S | H | H | 1 | | H | H | CH₃ | H | - |
| 2.54a | -CH₂-S-CH₂- | | H | H | E | R | CH₃ | H | S | H | H | 1 | | H | H | CH₃ | H | - |
| 2.54b | -CH₂-S-CH₂- | | H | H | Z | R | CH₃ | H | S | H | H | 1 | | H | H | CH₃ | H | |
| 2.55a | -CH₂-S(=O)-CH₂- | | H | H | E | R | CH₃ | H | S | H | H | 1 | | H | H | CH₃ | H | - |
| 2.55b | -CH₂-S(-O)-CH₂- | | H | H | Z | R | CH₃ | H | S | H | H | 1 | | H | H | CH₃ | H | - |
| 2.56a | -CH₂-S(=O)₂-CH₂- | | H | H | E | R | CH₃ | H | S | H | H | 1 | | H | H | CH₃ | H | - |
| 2.56b | -CH₂-S(=O)₂-CH₂- | | H | H | Z | R | CH₃ | H | S | H | H | 1 | | H | H | CH₃ | H | - |
| 2.57a | -CH₂-CH₂-CH₂- | | H | H | E | R | CH₃ | H | S | H | H | 1 | | CH₃ | H | CH₃ | H | - |
| 2.57b | -CH₂-CH₂-CH₂- | | H | H | Z | R | CH₃ | H | S | H | H | 1 | | CH₃ | H | CH₃ | H | - |
| 2.58a | -CH₂-CH₂-CH₂- | | H | H | E | rac | H | H | | | | O | | H | H | H | H | O |
| 2.58b | -CH₂-CH₂-CH₂- | | H | H | Z | rac | H | H | | | | O | | H | H | H | H | O |
| 2.59a | -CH₂-CH₂-CH₂- | | H | H | E | rac | H | H | | H | H | 1 | | H | H | H | H | S |
| 2.59b | -CH₂-CH₂-CH₂- | | H | H | Z | rac | H | H | | H | H | 1 | | H | H | H | H | S |
| 2.60a | -CH₂-CH₂-CH₂- | | H | H | E | rac | H | H | | | | 0 | | H | H | H | H | -CH₂-O- |
| 2.60b | -CH₂-CH₂-CH₂- | | H | H | Z | rac | H | H | | | | 0 | | H | H | H | H | -CH₂-O- |
| 2.61a | -CH₂-CH₂-CH₂- | | H | H | E | rac | H | H | | | | 0 | | H | H | H | H | -CH₂-S- |
| 2.61b | -CH₂-CH₂-CH₂- | | H | H | Z | rac | H | H | | | | 0 | | H | H | H | H | -CH₂-S- |
| 2.62a | -CH₂-CH₂-CH₂- | | H | H | E | rac | H | H | | H | H | 1 | | H | H | CH₃ | H | -NMe- |
| 2.62b | -CH₂-CH₂-CH₂- | | H | H | Z | rac | H | H | | H | H | 1 | | H | H | CH₃ | H | -NMe- |
| 2.63a | -CH₂-CH₂-CH₂- | | H | H | E | rac | H | H | | H | H | 1 | | H | H | CH₃ | H | -C(=O)- |
| 2.63b | -CH₂-CH₂-CH₂- | | H | H | Z | rac | H | H | | H | H | 1 | | H | H | CH₃ | H | -C(=O)- |
| 2.64a | -CH₂-CH₂-CH₂- | | H | H | E | rac | H | H | | CH₃ | CH₃ | 1 | | H | H | CH₃ | H | -CH₂- |
| 2.64b | -CH₂-CH₂-CH₂- | | H | H | Z | rac | H | H | | CH₃ | CH₃ | 1 | | H | H | CH₃ | H | -CH₂- |
| 2.65a | -CH₂-CH₂-CH₂- | | H | H | E | rac | CH₂C H₃ | H | rac | H | H | 2 | | H | H | H | H | - |
| 2.65b | -CH₂-CH₂-CH₂- | | H | H | Z | rac | CH₂C H₃ | H | rac | H | H | 2 | | H | H | H | H | - |
| 2.66a | -CH₂-CH₂-CH₂- | | H | H | E | rac | H | H | | H | H | 2 | | H | H | OCH₂CH₃ | H | - |
| 2.66b | -CH₂-CH₂-CH₂- | | H | H | Z | rac | H | H | | H | H | 2 | | H | H | OCH₂CH₃ | H | - |
| 2.67a | -CH₂-CH₂-CH₂- | | H | H | E | rac | H | H | | H | H | 1 | | H | H | Cl | H | - |
| 2.67b | -CH₂-CH₂-CH₂- | | H | H | Z | rac | H | H | | H | H | 1 | | H | H | Cl | H | - |
| 2.68a | -CH₂-CH₂-CH₂- | | H | H | E | rac | H | H | | H | H | 1 | | H | H | OCH₃ | H | - |
| 2.68b | -CH₂-CH₂-CH₂- | | H | H | Z | rac | H | H | | H | H | 1 | | H | H | OCH₃ | H | - |
| 2.69a | -CH₂-CH₂-CH₂- | | H | H | E | rac | H | H | | H | H | 1 | | H | H | CN | H | - |
| 2.69b | -CH₂-CH₂-CH₂- | | H | H | Z | rac | H | H | | H | H | 1 | | H | H | CN | H | - |
| 2.70a | -CH₂-CH₂-CH₂- | | H | H | E | rac | H | H | | H | H | 1 | | H | H | Br | H | - |
| 2.70b | -CH₂-CH₂-CH₂- | | H | H | Z | rac | H | H | | H | H | 1 | | H | H | Br | H | - |
| 2.71a | -CH₂-CH₂-CH₂- | | H | H | E | rac | H | H | | H | H | 1 | | H | H | I | H | - |
| 2.71b | -CH₂-CH₂-CH₂- | | H | H | Z | rac | H | H | | H | H | 1 | | H | H | I | H | - |
| 2.72a | -CH₂-CH₂-CH₂- | | H | H | E | rac | H | H | | H | H | 1 | | H | H | CH=CH₂ | H | - |
| 2.72b | -CH₂-CH₂-CH₂- | | H | H | Z | rac | H | H | | H | H | 1 | | H | H | CH=CH₂ | H | - |
| 2.73a | -CH₂-CH₂-CH₂- | | H | H | E | rac | H | H | | H | H | 1 | | H | H | CCH | H | - |
| 2.73b | -CH₂-CH₂-CH₂- | | H | H | Z | rac | H | H | | H | H | 1 | | H | H | CCH | H | - |
| 2.74a | -CH₂-CH₂-CH₂- | | H | H | E | rac | H | H | | H | H | 1 | | H | H | C≡CCH₃ | H | - |
| 2.74b | -CH₂-CH₂-CH₂- | | H | H | Z | rac | H | H | | H | H | 1 | | H | H | C≡CCH₃ | H | - |
| 2.75a | -CH₂-CH₂-CH₂- | | H | H | E | rac | H | H | | H | H | 1 | | H | H | C≡CPh | H | - |
| 2.75b | -CH₂-CH₂-CH₂- | | H | H | Z | rac | H | H | | H | H | 1 | | H | H | C≡CPh | H | - |
| 2.76a | -CH₂-CH₂-CH₂- | | H | H | E | rac | H | H | | H | H | 1 | | H | H | C≡CCH₂OH | H | - |
| 2.76b | -CH₂-CH₂-CH₂- | | H | H | Z | rac | H | H | | H | H | 1 | | H | H | C≡CCH₂OH | H | - |
| 2.77a | -CH₂-CH₂-CH₂- | | H | H | E | rac | H | H | | H | H | 1 | | H | H | C≡CCH₂OCH₃ | H | - |
| 2.77b | -CH₂-CH₂-CH₂- | | H | H | Z | rac | H | H | | H | H | 1 | | H | H | C≡CCH₂OCH₃ | H | - |
| 2.78a | -CH₂-CH₂-CH₂- | | H | H | E | rac | H | H | | H | H | 1 | | H | Cl | Cl | H | - |
| 2.78b | -CH₂-CH₂-CH₂- | | H | H | Z | rac | H | H | | H | H | 1 | | H | Cl | Cl | H | - |
| 2.79a | -CH₂-CH₂-CH₂- | | H | H | E | rac | H | H | | H | H | 1 | | H | -O-CH₂-O- | -O-CH₂-O- | H | - |
| 2.79b | -CH₂-CH₂-CH₂- | | H | H | Z | rac | H | H | | H | H | 1 | | H | -O-CH₂-O- | -O-CH₂-O- | H | - |
| 2.80a | -CH₂-CH₂-CH₂- | | H | H | E | rac | H | H | | H | H | 1 | | H | H | CF₃ | H | - |
| 2.80b | -CH₂-CH₂-CH₂- | | H | H | Z | rac | H | H | | H | H | 1 | | H | H | CF₃ | H | - |
| 2.81a | -CH₂-CH₂-CH₂- | | H | H | E | rac | H | H | | H | H | 1 | | H | H | H | CH₃ | - |
| 2.81b | -CH₂-CH₂-CH₂- | | H | H | Z | rac | H | H | | H | H | 1 | | H | H | H | CH₃ | - |
| 2.82a | -CH₂-CH₂-CH₂- | | H | H | E | rac | H | H | | H | H | 1 | | CH₃ | H | H | CH₃ | - |
| 2.82b | -CH₂-CH₂-CH₂- | | H | H | Z | rac | H | H | | H | H | 1 | | CH₃ | H | H | CH₃ | - |
| 2.83a | -CH₂-CH₂-CH₂- | | H | H | E | rac | H | H | | H | H | 1 | | CH₃ | H | H | H | O |
| 2.83b | -CH₂-CH₂-CH₂- | | H | H | Z | rac | H | H | | H | H | 1 | | CH₃ | H | H | H | O |
| 2.84a | -CH₂-CH₂-CH₂- | | H | H | E | rac | H | H | | H | H | 1 | | Cl | H | H | H | O |
| 2.84b | -CH₂-CH₂-CH₂- | | H | H | Z | rac | H | H | | H | H | 1 | | Cl | H | H | H | O |
| 2.85a | -CH₂-CH₂-CH₂- | | H | H | E | rac | H | H | | H | H | 1 | | Cl | H | CH₃ | H | O |
| 2.85b | -CH₂-CH₂-CH₂- | | H | H | Z | rac | H | H | | H | H | 1 | | Cl | H | CH₃ | H | O |
| 2.86a | -CH₂-CH₂-CH₂- | | H | H | E | rac | H | H | | H | H | 1 | | Ph | H | H | H | O |
| 2.86b | -CH₂-CH₂-CH₂- | | H | H | Z | rac | H | H | | H | H | 1 | | Ph | H | H | H | O |
| 2.87a | -CH₂-CH₂-CH₂- | | H | H | E | rac | CH₃ | H | rac | H | H | 1 | | H | H | H | H | O |
| 2.87b | -CH₂-CH₂-CH₂- | | H | H | Z | rac | CH₃ | H | rac | H | H | 1 | | H | H | H | H | O |
| 2.88a | -CH₂-CH₂-CH₂- | | H | H | E | rac | CH₃ | H | rac | H | H | 1 | | Cl | H | Cl | H | O |
| 2.88b | -CH₂-CH₂-CH₂- | | H | H | Z | rac | CH₃ | H | rac | H | H | 1 | | Cl | H | Cl | H | O |
| 2.89a | -CH₂-CH₂-CH₂- | | H | H | E | rac | H | H | | CH₃ | CH₃ | 1 | | H | H | H | H | O |
| 2.89b | -CH₂-CH₂-CH₂- | | H | H | Z | rac | H | H | | CH₃ | CH₃ | 1 | | H | H | H | H | O |
| 2.90a | -CH₂-CH₂-CH₂- | | H | H | E | rac | H | H | | Ph | H | 1 | | H | H | H | H | O |
| 2.90b | -CH₂-CH₂-CH₂- | | H | H | Z | rac | H | H | | Ph | H | 1 | | H | H | H | H | O |
| 2.91a | -CH₂-CH₂-CH₂- | | H | H | E | rac | H | H | | H | H | 1 | | H | H | H | H | - |
| 2.91b | -CH₂-CH₂-CH₂- | | H | H | Z | rac | H | H | | H | H | 1 | | H | H | H | H | - |
| 2.92a | -CH₂-CH(4-F-Ph)-CH₂- | | H | H | E | R | H | H | | H | H | 2 | | H | H | H | H | - |
| 2.92b | -CH₂-CH(4-F-Ph)-CH₂- | | H | H | Z | R | H | H | | H | H | 2 | | H | H | H | H | - |
| 2.93a | -CH₂-CH(2-F-Ph)-CH₂- | | H | H | E | R | H | H | | H | H | 2 | | H | H | H | H | - |
| 2.93b | -CH₂-CH(2-F-Ph)-CH₂- | | H | H | Z | R | H | H | | H | H | 2 | | H | H | H | H | - |
| 2.94a | -CH₂-CMe₂-CH₂- | | H | H | E | rac | H | H | | H | H | 1 | | H | H | H | H | O |
| 2.94b | -CH₂-CMe₂-CH₂- | | H | H | Z | rac | H | H | | H | H | 1 | | H | H | H | H | O |
| 2.95a | -CH₂-CMe₂-CH₂- | | H | H | E | rac | CH₃ | H | rac | H | H | 2 | | H | H | H | H | - |
| 2.95b | -CH₂-CMe₂-CH₂- | | H | H | Z | rac | CH₃ | H | rac | H | H | 2 | | H | H | H | H | - |
| 2.96a | -CH₂-CH(CF₃)-CH₂- | | H | H | E | R | H | H | | H | H | 2 | | H | H | H | H | - |
| 2.96b | -CH₂-CH(CF₃)-CH₂- | | H | H | Z | R | H | H | | H | H | 2 | | H | H | H | H | - |
| 2.97a | -CH₂-O-CH₂- | | H | H | E | R | CH₃ | H | S | H | H | 1 | | H | H | CH₃ | H | - |
| 2.97b | -CH₂-O-CH₂- | | H | H | Z | R | CH₃ | H | S | H | H | 1 | | H | H | CH₃ | H | - |
| 2.98a | -CH₂-CH₂-CH₂- | | CF₃ | H | E | R | CH₃ | H | S | H | H | 1 | | H | H | CH₃ | H | - |
| 2.98b | -CH₂-CH₂-CH₂- | | CF₃ | H | Z | R | CH₃ | H | S | H | H | 1 | | H | H | CH₃ | H | - |
| 2.99a | -CH₂-Ring5*-CH₂- | | H | H | E | R | CH₃ | H | S | H | H | 1 | | H | H | CH₃ | H | - |
| 2.99b | -CH₂-Ring5*-CH₂- | | H | H | Z | R | CH₃ | H | S | H | H | 1 | | H | H | CH₃ | H | - |
| 2.100a | -CH₂-CH₂-CH₂- | | H | H | E | R | H | H | | H | H | 1 | | CH₃ | H | H | H | O |
| 2.100b | -CH₂-CH₂-CH₂- | | H | H | Z | R | H | H | | H | H | 1 | | CH₃ | H | H | H | O |
| 2.101a | -CH₂-CH₂-CH₂- | | H | H | E | R | H | H | | H | H | 2 | | H | F | H | H | - |
| 2.101b | -CH₂-CH₂-CH₂- | | H | H | Z | R | H | H | | H | H | 2 | | H | F | H | H | - |
| 2.102a | -CH₂-CHipr-CH₂- | | H | H | E | R | H | H | | H | H | 2 | | H | H | H | H | - |
| 2.102b | -CH₂-CHipr-CH₂- | | H | H | Z | R | H | H | | H | H | 2 | | H | H | H | H | - |
| 2.103a | -CH₂-CHipr-CH₂- | | H | H | E | R | H | H | | H | H | 1 | | H | H | H | H | - |
| 2.103b | -CH₂-CHipr-CH₂- | | H | H | Z | R | H | H | | H | H | 1 | | H | H | H | H | - |
| 2.104a | -CH₂-CHipr-CH₂- | | H | H | E | R | CH₃ | H | S | H | H | 1 | | H | H | CH₃ | H | - |
| 2.104b | -CH₂-CHipr-CH₂- | | H | H | Z | R | CH₃ | H | S | H | H | 1 | | H | H | CH₃ | H | - |
| 2.105a | -CH₂-Ring4*-CH₂- | | H | H | E | R | H | H | | H | H | 1 | | H | H | CH₃ | H | - |
| 2.105b | -CH₂-Ring4*-CH₂- | | H | H | Z | R | H | H | | H | H | 1 | | H | H | CH₃ | H | - |
| 2.106a | -CH₂-CH₂-CH₂- | | H | H | E | rac | CH₂-CH₃ | H | rac | H | H | 1 | | H | H | CH₃ | H | - |
| 2.106b | -CH₂-CH₂-CH₂- | | H | H | Z | rac | CH₂-CH₃ | H | rac | H | H | 1 | | H | H | CH₃ | H | - |
| 2.107a | -CH₂-CH₂-CH₂- | | H | H | E | rac | H | H | | H | H | 1 | | H | H | i-Pr | H | - |
| 2.107b | -CH₂-CH₂-CH₂- | | H | H | Z | rac | H | H | | H | H | 1 | | H | H | i-Pr | H | - |
| 2.108a | -CH₂-CH₂-CH₂- | | H | H | E | rac | CH₃ | H | rac | H | H | 1 | | H | Cl | H | H | - |
| 2.108b | -CH₂-CH₂-CH₂- | | H | H | Z | rac | CH₃ | H | rac | H | H | 1 | | H | Cl | H | H | - |
| 2.109a | -CH₂-CH₂-CH₂- | | H | H | E | rac | H | H | | H | H | 2 | | H | OCH₃ | H | H | - |
| 2.109b | -CH₂-CH₂-CH₂- | | H | H | Z | rac | H | H | | H | H | 2 | | H | OCH₃ | H | H | - |
| 2.110a | -CH₂-Ring4*-CH₂- | | H | H | E | R | H | H | | H | H | 2 | | H | H | H | H | - |
| 2.110b | -CH₂-Ring4*-CH₂- | | H | H | Z | R | H | H | | H | H | 2 | | H | H | H | H | - |
| 2.111a | -CH₂-CH₂-CH₂- | | H | H | E | rac | H | H | | H | H | 1 | | H | H | H | H | CHCH₃ |
| 2.111b | -CH₂-CH₂-CH₂- | | H | H | Z | rac | H | H | | H | H | 1 | | H | H | H | H | CHCH₃ |
| 2.112a | -CH₂-Ring5*-CH₂- | | H | H | E | R | H | H | | H | H | 1 | | H | H | CH₃ | H | - |
| 2.112b | -CH₂-Ring5*-CH₂- | | H | H | Z | R | H | H | | H | H | 1 | | H | H | CH₃ | H | - |
| 2.113a | -CH₂-CH₂-CH₂- | | H | H | E | rac | H | H | | H | H | 1 | | H | H | i-Pr | H | O |
| 2.113b | -CH₂-CH₂-CH₂- | | H | H | Z | rac | H | H | | H | H | 1 | | H | H | i-Pr | H | O |
| 2.114a | -CH₂-CH₂-CH₂- | | H | H | E | rac | H | H | | H | H | 1 | | H | H | F | H | S |
| 2.114b | -CH₂-CH₂-CH₂- | | H | H | Z | rac | H | H | | H | H | 1 | | H | H | F | H | S |
| 2.115a | -CH₂-CH₂-CH₂- | | H | H | E | rac | H | H | | H | H | 1 | | CF₃ | H | H | H | - |
| 2.115b | -CH₂-CH₂-CH₂- | | H | H | Z | rac | H | H | | H | H | 1 | | CF₃ | H | H | H | - |
| 2.116a | -CH₂-CH₂-CH₂- | | H | H | E | rac | H | H | | CH₃ | CH₃ | 1 | | H | O-CH₃ | H | H | O |
| 2.116b | -CH₂-CH₂-CH₂- | | H | H | Z | rac | H | H | | CH₃ | CH₃ | 1 | | H | O-CH₃ | H | H | O |
| 2.117a | -CH₂-CH₂-CH₂- | | H | H | E | rac | H | H | | | | 0 | | H | H | CH₃ | H | O |
| 2.117b | -CH₂-CH₂-CH₂- | | H | H | Z | rac | H | H | | | | 0 | | H | H | CH₃ | H | O |
| 2.118a | -CH₂-CH₂-CH₂- | | H | H | E | R | CH₃ | H | S | H | H | 1 | | H | H | CH₃ | H | - |
| 2.118b | -CH₂-CH₂-CH₂- | | H | H | Z | R | CH₃ | H | S | H | H | 1 | | H | H | CH₃ | H | - |
| 2.119a | -CH₂-CH₂- | | H | H | E | R | CH₃ | H | S | H | H | 1 | | H | H | CH₃ | H | - |
| 2.119b | -CH₂-CH₂- | | H | H | Z | R | CH₃ | H | S | H | H | 1 | | H | H | CH₃ | H | - |
| 2.120a | -CH₂-CH₂- | | H | H | E | R | H | H | | H | H | 1 | | H | H | CH₃ | H | - |
| 2.120b | -CH₂-CH₂- | | H | H | Z | R | H | H | | H | H | 1 | | H | H | CH₃ | H | - |
| 2.121a | -CH₂-CH₂- | | H | H | E | R | CH₃ | H | S | H | H | 1 | | H | H | H | H | - |
| 2.121b | -CH₂-CH₂- | | H | H | Z | R | CH₃ | H | S | H | H | 1 | | H | H | H | H | - |
| 2.122a | -CH₂-CH₂- | | H | H | E | R | H | H | | H | H | 2 | | H | H | H | H | - |
| 2.122b | -CH₂-CH₂- | | H | H | Z | R | H | H | | H | H | 2 | | H | H | H | H | - |
| 2.123a | -CH₂-CH₂-CH₂- | | H | CH₃ | E | R | CH₃ | H | S | H | H | 1 | | H | H | CH₃ | H | - |
| 2.123b | -CH₂-CH₂-CH₂- | | H | CH₃ | Z | R | CH₃ | H | S | H | H | 1 | | H | H | CH₃ | H | - |
| 2.124a | -CH₂-CH₂-CH₂- | | H | CH₃ | E | R | H | H | | H | H | 1 | | H | H | H | H | - |
| 2.124b | -CH₂-CH₂-CH₂- | | H | CH₃ | Z | R | H | H | | H | H | 1 | | H | H | H | H | - |
| 2.125a | -CH₂-CH₂-CH₂- | | H | CH₂CH₃ | E | R | CH₃ | H | S | H | H | 1 | | H | H | CH₃ | H | - |
| 2.125b | -CH₂-CH₂-CH₂- | | H | CH₂CH₃ | Z | R | CH₃ | H | S | H | H | 1 | | H | H | CH₃ | H | - |
| 2.126a | -CH₂-CH₂-CH₂- | | H | CH₂CH₃ | E | R | H | H | | H | H | 2 | | H | H | H | H | - |
| 2.126b | -CH₂-CH₂-CH₂- | | H | CH₂CH₃ | Z | R | H | H | | H | H | 2 | | H | H | H | H | - |
| 2.127a | -CH₂-CH₂-CH₂- | | H | CH₃ | E | R | H | H | | H | H | 2 | | H | H | H | H | - |
| 2.127b | -CH₂-CH₂-CH₂- | | H | CH₃ | Z | R | H | H | | H | H | 2 | | H | H | H | H | - |
| 2.128a | -CH₂-CH₂-CH₂- | | H | CH₂CH₃ | E | R | H | H | | H | H | 1 | | H | H | H | H | - |
| 2.128b | -CH₂-CH₂-CH₂- | | H | CH₂CH₃ | Z | R | H | H | | H | H | 1 | | H | H | H | H | - |
| 2.129a | -CH₂-CH₂-CH₂- | | H | C(CH₃)₃ | E | R | H | H | | H | H | 1 | | H | H | H | H | - |
| 2.129b | -CH₂-CH₂-CH₂- | | H | C(CH₃)₃ | Z | R | H | H | | H | H | 1 | | H | H | H | H | - |
| 2.130a | -CH₂-CH₂-CH₂- | | H | CH₃ | E | R | H | H | | H | H | 1 | | H | H | CH₃ | H | - |
| 2.130b | ₋CH₂-CH₂-CH₂- | | H | CH₃ | Z | R | H | H | | H | H | 1 | | H | H | CH₃ | H | - |
| 2.131a | -CH₂-CH₂-CH₂- | | H | CH(Rin g-CH₂)₄ | E | R | H | H | | H | H | 1 | | H | H | H | H | - |
| 2.131b | -CH₂-CH₂-CH₂- | | H | CH(Rin g-CH₂)₄ | Z | R | H | H | | H | H | 1 | | H | H | H | H | - |
| 2.132a | -CH₂-CH₂-CH₂- | | H | C(CH₃)₃ | E | R | H | H | | H | H | 1 | | H | H | CH₃ | H | - |
| 2.132b | -CH₂-CH₂-CH₂- | | H | C(CH₃)₃ | Z | R | H | H | | H | H | 1 | | H | H | CH₃ | H | - |
| 2.133a | -CH₂-CH₂-CH₂- | | H | CH₂CH₃ | E | R | H | H | | H | H | 2 | | H | H | CH₃ | H | - |
| 2.133b | -CH₂-CH₂-CH₂- | | H | CH₂CH₃ | Z | R | H | H | | H | H | 2 | | H | H | CH₃ | H | - |
| 2.134a | -CH₂-Ring4*-CH₂- | | H | CH₃ | E | R | H | H | | H | H | 2 | | H | H | H | H | - |
| 2.134b | -CH₂-Ring4*-CH₂- | | H | CH₃ | Z | R | H | H | | H | H | 2 | | H | H | H | H | - |
| 2.135a | -CH₂-CH₂-CH₂- | | H | CH₂CH( CH₃)₂ | E | R | H | H | | H | H | 2 | | H | H | H | H | - |
| 2.135b | -CH₂-CH₂-CH₂- | | H | CH₂CH( CH₃)₂ | Z | R | H | H | | H | H | 2 | | H | H | H | H | - |
| 2.136a | -CH₂-CH₂-CH₂- | | H | CH₃ | E | R | H | H | | H | H | 2 | | H | H | CH₃ | H | - |
| 2.136b | -CH₂-CH₂-CH₂- | | H | CH₃ | Z | R | H | H | | H | H | 2 | | H | H | CH₃ | H | - |
| 2.137a | -CH₂-CH₂-CH₂- | | H | CH₂CH (CH₃)₂ | E | R | H | H | | H | H | 1 | | H | H | H | H | - |
| 2.137b | -CH₂-CH₂-CH₂- | | H | CH₂CH (CH₃)₂ | Z | R | H | H | | H | H | 1 | | H | H | H | H | - |
| 2.138a | -CH₂-Ring4*-CH₂- | | H | CH₂CH₃ | E | R | H | H | | H | H | 2 | | H | H | H | H | - |
| 2.138b | -CH₂-Ring4*-CH₂- | | H | CH₂CH₃ | Z | R | H | H | | H | H | 2 | | H | H | H | H | - |
| 2.139a | -CH₂-CH₂-CH₂- | | H | CH(Rin g-CH₂)₄ | E | R | H | H | | H | H | 2 | | H | H | H | H | - |
| 2.139b | -CH₂-CH₂-CH₂- | | H | CH(Rin g-CH₂)₄ | Z | R | H | H | | H | H | 2 | | H | H | H | H | - |
| 2.140a | -CH₂-CH₂-CH₂- | | H | CH₂CH (CH₃)₂ | E | R | H | H | | H | H | 2 | | H | H | CH₃ | H | - |
| 2.140b | -CH₂-CH₂-CH₂- | | H | CH₂CH (CH₃)₂ | z | R | H | H | | H | H | 2 | | H | H | CH₃ | H | - |
| 2.141a | -CH₂-Ring4*-CH₂- | | H | CH₂CH (CH₃)₂ | E | R | H | H | | H | H | 2 | | H | H | H | H | - |
| 2.141b | -CH₂-Ring4*-CH₂- | | H | CH₂CH (CH₃)₂ | Z | R | H | H | | H | H | 2 | | H | H | H | H | - |
| 2.142a | -CH₂-Ring4*-CH₂- | | H | C(CH₃)₃ | E | R | H | H | | H | H | 2 | | H | H | H | H | - |
| 2.142b | -CH₂-Ring4*-CH₂- | | H | C(CH₃)₃ | Z | R | H | H | | H | H | 2 | | H | H | H | H | - |
| 2.143a | -CH₂-CH₂-CH₂- | | H | CH₂CH₃ | E | R | H | H | | H | H | 1 | | H | H | CH₃ | H | - |
| 2.143b | -CH₂-CH₂-CH₂- | | H | CH₂CH₃ | Z | R | H | H | | H | H | 1 | | H | H | CH₃ | H | - |
| 2.144a | -CH₂-CH₂-CH₂- | | H | CH(Rin g-CH₂)₄ | E | R | H | H | | H | H | 1 | | H | H | CH₃ | H | - |
| 2.144b | -CH₂-CH₂-CH₂- | | H | CH(Rin g-CH₂)₄ | Z | R | H | H | | H | H | 1 | | H | H | CH₃ | H | - |
| 2.145a | -CH₂-CH₂-CH₂- | | H | CH₂CH( CH₃)₂ | E | R | H | H | | H | H | 2 | | H | H | H | H | - |
| 2.145b | -CH₂-CH₂-CH₂- | | H | CH₂CH( CH₃)₂ | Z | R | H | H | | H | H | 2 | | H | H | H | H | - |
| 2.146a | -CH₂-CH₂-CH₂- | | H | C(CH₃)₃ | E | R | H | H | | H | H | 2 | | H | H | H | H | - |
| 2.146b | -CH₂-CH₂-CH₂- | | H | C(CH₃)₃ | Z | R | H | H | | H | H | 2 | | H | H | H | H | - |

In Tabelle 2 stehen Ring4* und Ring5* in der Spalte R¹/R^{2a} für einen vier- bzw. fünfgliedrigen Spirocyclus mit -CH₂-CH₂-CH₂-CH₂- bzw. CH₂-CH₂-CH₂-CH₂--CH₂- als Ring.

Weiterer Gegenstand der vorliegenden Erfindung sind Verfahren zur Herstellung entsprechender Verbindungen der allgemeinen Formel (I) und/oder deren Salze und/oder deren agrochemisch verträglichen quaternierten Stickstoff-Derivate 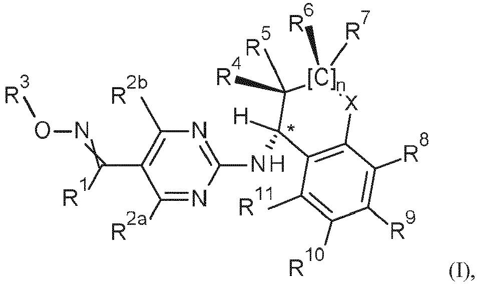 in welchen die Reste R¹ bis R¹¹ sowie X und n die vorstehenden Bedeutungen aufweisen, und wobei nach einem ersten Verfahren
a) eine Verbindung der allgemeinen Formel (II) 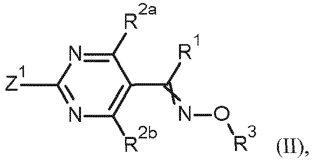 wobei R¹, R^{2a}, R^{2b} und R³ die vorstehende Bedeutung aufweisen und Z¹ für einen austauschfähigen Rest oder eine Abgangsgruppe steht,
   mit einem Amin der allgemeinen Formel (III) oder einem Säureadditionssalz des Amins der allgemeinen Formel (III) 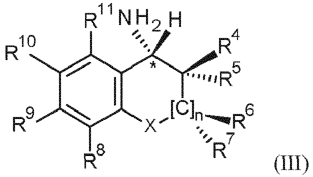 gegebenenfalls unter zur Hilfenahme unterstützender Reagenzien wie zusätzlichen Hilfsbasen wie z.B. Triethylamin oder N-Chlor-Succinimid umgesetzt wird,
   wobei die Reste R⁴ bis R¹¹ und X sowie die Laufzahl n die vorstehende Bedeutung aufweisen.

Der austauschfähige Rest Z¹, bzw. die Abgangsgruppe Z¹ steht für Fluor, Chlor, Brom, Jod, ein (C₁-C₄)-Alkylsulfanyl oder ein (C₁-C₄)-Alkylsulfinyl oder ein (C₁-C₄)-Alkylsulfonyl, ein unsubstituiertes oder ein einfach oder mehrfach mit Fluor, Chlor, Brom oder (C₁-C₄)-Alkyl oder (C₁-C₄)-Alkoxy substituiertes Phenyl-(C₁-C₄)-alkylsulfonyl oder ein (C₁-C₄)-Alkylphenyl-sulfonyl.

Gegebenenfalls kann ein Rest Z¹ in eine andere, besser austauschbare Gruppe überführt werden. So kann beispielsweise im Sinne eines Zweistufen-Eintopfverfahren ein (C₁-C₄)-Alkylsulfanyl mit einem Oxidationsmittel wie m-Chlorperbenzoesäure oder Oxone® in ein (C₁-C₄)-Alkylsulfinyl oder ein (C₁-C₄)-Alkylsulfonyl oder Mischungen davon überführt werden und anschließend mit einem Amin der allgemeinen Formel (III) oder einem Säureadditionssalz unter Verwendung einer Hilfsbase wie z.B. Triethylamin oder Kaliumcarbonat zur Reaktion gebracht werden.

Die Verbindungen der allgemeinen Formel (II) können nach bekannten Verfahren hergestellt werden aus den entsprechenden Ketonen hergestellt werden. In dem Fall, dass R³ für Wasserstoff steht kann auch nach Umsetzung mit dem Amin der allgemeinen Formel (III) das Wasserstoff -Atom durch z.B. eine Alkylierung in andere Moleküle der Formel (I) überführt werden.

Gegebenenfalls kann die Umsetzung auch durch unterschiedliche Hilfsstoffe katalysiert werden, wie beispielsweise durch die Reagenzien Kaliumphosphat, Kupfer(I)iodid und N,N-diethyl-2-hydroxybenzamide oder im Sinne der Buchwald-Hartwig-Kupplung durch spezielle Übergangsmetallkatalysatorsysteme.

Die Amine der allgemeinen Formel (III) oder die Säureadditionssalz davon sind kommerziell verfügbar oder deren Synthese ist in WO 2004/069814 A1 beschrieben.

In einer weiteren Ausführungsform werden die Verbindungen der allgemeinen Formel (I) und/oder deren agrochemisch verträgliche Salze und/oder deren agrochemisch verträgliche quaternierte Stickstoff-Derivate 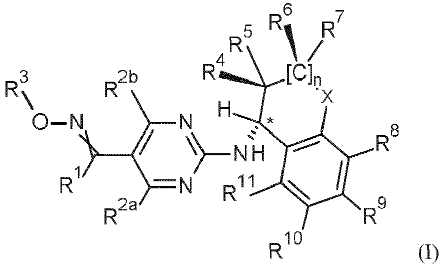 in welchen die Reste R¹ bis R¹¹ sowie X und n die vorstehenden Bedeutungen aufweisen, nach einem zweiten Verfahren
b) durch Umsetzung einer Verbindung der allgemeinen Formel (IIa) 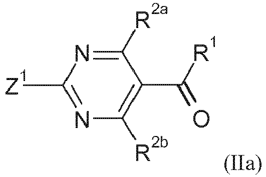 worin R¹ und R^{2a} sowie R^{2b} die vorstehende Bedeutung aufweisen und Z¹ für einen austauschfähigen Rest oder eine Abgangsgruppe steht,
mit einem Amin der allgemeinen Formel (III) oder einem Säureadditionssalz des Amins der allgemeinen Formel (III) 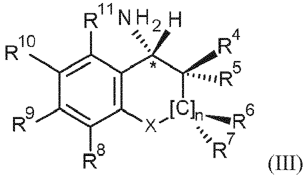 wobei die Reste R⁴ bis R¹¹ sowie X und n die vorstehende Bedeutung aufweisen, hergestellt.

Der austauschfähige Rest Z¹, bzw. die Abgangsgruppe Z¹ steht für Fluor, Chlor, Brom, Jod, ein (C₁-C₄)-Alkylsulfanyl oder ein (C₁-C₄)-Alkylsulfinyl oder ein (C₁-C₄)-Alkylsulfonyl, ein unsubstituiertes oder ein substituiertes Phenyl-(C₁-C₄)-alkylsulfonyl oder ein (C₁-C₄)-Alkylphenyl-sulfonyl.

Gegebenenfalls kann ein Rest Z¹ in eine andere, besser austauschbare Gruppe überführt werden. So kann beispielsweise im Sinne eines Zweistufen-Eintopfverfahren ein (C₁-C₄)-Alkylsulfanyl mit einem Oxidationsmittel wie m-Chlorperbenzoesäure oder Oxone® in ein (C₁-C₄)-Alkylsulfinyl oder ein (C₁-C₄)-Alkylsulfonyl oder Mischungen davon überführt werden und anschließend mit einem Amin der allgemeinen Formel (III) oder einem Säureadditionssalz unter Verwendung einer Hilfsbase wie z.B. Triethylamin oder Kaliumcarbonat zur Reaktion gebracht werden.

Die Verbindungen der allgemeinen Formel (IIa) sind kommerziell verfügbar oder können nach bekannten Verfahren hergestellt werden.

Gegebenenfalls kann die Umsetzung auch durch unterschiedliche Hilfsstoffe katalysiert werden, wie beispielsweise durch die Reagenzien Kaliumphosphat, Kupfer(I)iodid und N,N-diethyl-2-hydroxybenzamide oder im Sinne der Buchwald-Hartwig-Kupplung durch spezielle Übergangsmetallkatalysatorsysteme.

Die Amine der allgemeinen Formel (III) oder die Säureadditionssalz davon sind kommerziell verfügbar oder deren Synthese ist in WO 2004/069814 A1 beschrieben.

Die so hergestellten Ketone werden anschließend basenkatalysiert mit Hydroxylamin oder O-substituierten Hydroxylaminen (bzw. den aus den jeweiligen Salzen der Hydroxylamine in situ freigesetzten Reaktanten) zu den Verbindungen der Formel (I) umgesetzt.

Verbindungen der allgemeinen Formel (I) können auch dadurch hergestellt werden, dass zunächst eine Verbindung der allgemeinen Formel (IIa) analog den unter a. beschriebenen Verfahren mit einem Amin der Formel (III) oder einem Säureadditionssalz davon in ein Intermediat der Formel (Ia) 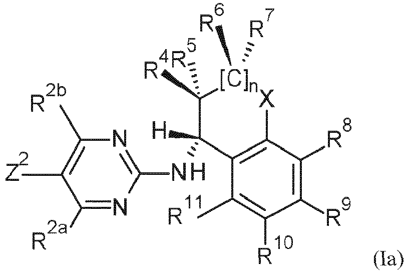 überführt werden und dann der Rest Z² in ein Keton überführt werden. So kann beispielsweise in dem Fall, dass Z² = (C₂-C₆)-Alkinyl ist, die (C₂-C₆)-Alkinyl-Gruppe in -C(=O)-CH₂-(C₁-C₄)-Alkinyl überführt werden oder wenn Z² = Trimethylsilylacetylene ist in Ethinyl überführt werden; beispielsweise kann der Rest Z² = COOH oder COO(C₁-C₆)-Alkyl nach in der Literatur bekannten Verfahren in C(=O)-R¹ überführt werden.

Gegebenenfalls kann auch ein Rest Z² in einen zunächst anderen Rest Z² überführt werden. So kann beispielsweise nach den zuvor beschriebenen Verfahren zunächst ein Intermediat des Typs (Ia) hergestellt werden, in dem der Rest Z² ein Halogen ist und das Halogen nach Literaturbekannten Verfahren in ein (C₂-C₆)-Alkinyl oder ein 1-(C₁-C₆)-Alkoxyl-(C₂-C₆)-Alkenyl überführt wird und dann der Rest Z² in eine C(=O)-R¹ umgewandelt wird.

Die so hergestellten Ketone der Formel (Ia) mit Z² = R³-CO- werden anschließend basenkatalysiert mit Hydroxylamin oder O-substituierten Hydroxylaminen (bzw. den aus den jeweiligen Salzen der Hydroxylamine in situ freigesetzten Reaktanden) zu den Verbindungen der Formel (I) umgesetzt.

Verbindungen der allgemeinen Formel (I) können auch hergestellt werden, indem Guanidine des Typs (IV) oder Säureadditionssalze davon 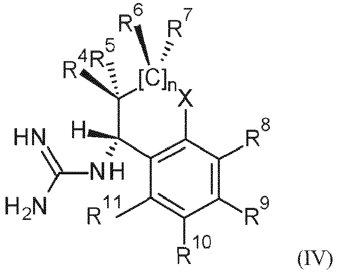 mit einem Keton der Formel (V) 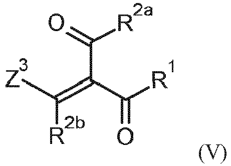 in der Rest Z³ für ein (C₁-C₆)-Alkoxy oder Di-(C₁-C₆)-Alkylamino steht, kondensiert werden. Die so hergestellten Ketone werden anschließend basenkatalysiert mit Hydroxylamin oder O-substituierten Hydroxylaminen (bzw. den aus den jeweiligen Salzen der Hydroxylamine in situ freigesetzten Reaktanden) zu den Verbindungen der Formel (I) umgesetzt.

Verbindungen der allgemeinen Formel (I) können schließlich auch in einer Drei-Komponenten-Reaktion hergestellt werden, indem Guanidine des Typs (IV) oder Säureadditionssalze 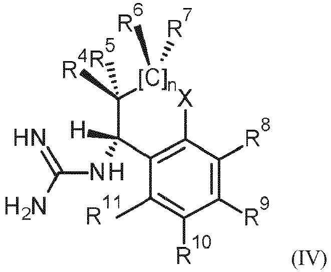 mit einem Keton der Formel (VI). 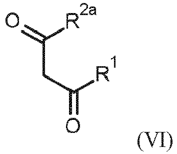 und mit einem Fragment (VII) in dem Z³ für ein (C₁-C₆)-Alkoxy oder Di-(C₁-C₆)-Alkylamino und Z⁴ für ein (C₁-C₆)-Alkox steht, 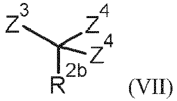 kondensiert werden.

Die so hergestellten Ketone werden anschließend basenkatalysiert mit Hydroxylamin oder O-substituierten Hydroxylaminen (bzw. den aus den jeweiligen Salzen der Hydroxylamine in situ freigesetzten Reaktanden) zu den Verbindungen der Formel (I) umgesetzt. Verbindungen der allgemeinen Formel (II), in denen der Rest Z¹ für (C₁-C₄)-Alkylsulfanyl steht können analog wie oben beschrieben hergestellt werden, wobei statt (IV) S-(C₁-C₄)-Alkylisothioharnstoffe oder deren Säureadditionssalze eingesetzt werden.

Verbindungen der allgemeinen Formel (I) können auch dadurch hergestellt werden, dass zunächst ein Intermediat der Formel (Ib) ensprechend den zuvor beschreibenenen Methoden hergestellt wird und dann die OH-Funktion am Oxim durch Alkylierung mit Verbindungen des Typs R³-Hal modifiziert werden.

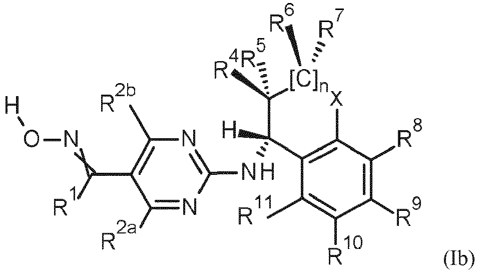

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Reaktionsstationen (reaction stations) der Firma Radleys, Shirehill, Saffron Walden, Essex, CB 11 3AZ, England oder MultiPROBE Automated Workstations der Firma Perkin Elmer, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma Teledyne ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Herbizide werden in landwirtschaftlich genutzten Kulturen während verschiedener Anbauphasen eingesetzt. So erfolgt die Applikation einiger Produkte schon vor oder während der Saat. Andere werden ausgebracht, bevor die Kulturpflanze aufläuft, d.h. bevor der Keimling die Erdoberfläche durchbricht (Vorauflauf-Herbizide). Nachauflauf-Herbizide schließlich werden verwendet, wenn von der Kulturpflanze entweder bereits die Keimblätter oder Laubblätter ausgebildet sind.

Die erfindungsgemäßen Verbindungen können dabei sowohl im Vorlauf als auch im Nachlauf angewendet werden, wobei eine Verwendung der erfindungsgemäßen Verbindungen im Vorlauf bevorzugt ist.

Die Vorauflauf-Behandlung schließt sowohl die Behandlung der Anbaufläche vor der Aussaat (ppi = pre plant incorporation) als auch die Behandlung der angesäten, aber noch nicht bewachsenen Anbauflächen ein.

Die erfindungsgemäßen Verbindungen der Formel (I) und deren Salze, im folgenden auch synonym zusammen auch als Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnem seien beispielhaft einige Vertreter der mono- und dikotylen Unkrautflora genannt, die durch die erfindungsgemäßen Verbindungen der allgemeinen Formel (I) kontrolliert werden können, ohne daß durch die Nennung eine Beschränkung auf bestimmte Arten erfolgen soll.

Auf der Seite der monokotylen Unkrautarten werden z. B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea, sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern. Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium beobachtet.

Werden die erfindungsgemäßen Verbindungen der allgemeinen Formel (I) vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe der allgemeinen Formel (I) auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle, Raps und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen der allgemeinen Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z. B. durch Auslösen von Desikkation und Wuchsstauchung eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z. B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP 0221044, EP 0131624). Beschrieben wurden beispielsweise in mehreren Fällen
- gentechnische Veränderungen von Kulturpflanzen zwecks Modifikation der in den Pflanzen synthetisierten Stärke (z. B. WO 92/011376, WO 92/014827, WO 91/019806),
- transgene Kulturpflanzen, welche gegen bestimmte Herbizide vom Typ Glufosinate (vgl. z. B. EP 0242236, EP 0242246) oder Glyphosate (WO 92/000377) oder der Sulfonylharnstoffe (EP 0257993, US 5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP 0142924, EP 0193259),
- transgene Kulturpflanzen mit modifizierter Fettsäurezusammensetzung (WO 91/013972),
- gentechnisch veränderte Kulturpflanzen mit neuen Inhalts- oder Sekundärstoffen z. B. neuen Phytoalexinen, die eine erhöhte Krankheitsresistenz verursachen (EP 0309862, EP 0464461),
- gentechnisch veränderte Pflanzen mit reduzierter Photorespiration, die höhere Erträge und höhere Stresstoleranz aufweisen (EP 0305398),
- transgene Kulturpflanzen, die pharmazeutisch oder diagnostisch wichtige Proteine produzieren ("molecular pharming"),
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) als Herbizide zur Bekämpfung von Schadpflanzen in transgenen Kulturpflanzen.

Die Verbindungen der allgemeinen Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösemittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z. B. Insektiziden, Akariziden, Herbiziden, Fungiziden, sowie mit Safenern, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-Dinaphylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösemittel z. B. Butanol, Cyclohexanon, Dimethylformamid, Xylol oder auch höhersiedenden Aromaten oder Kohlenwasserstoffen oder Mischungen der organischen Lösemittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z. B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rühren, Kolloidmühlen und/oder statischen Mischern unter Verwendung von wäßrigen organischen Lösemitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z. B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z. B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe u.a. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösemittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird anhand der nachfolgenden Beispiele näher erläutert, welche die Erfindung jedoch keinesfalls beschränken.

### A. Synthesebeispiele

### N-[(1R,2S)-2,6-Dimethyl-2,3-dihydro-1H-inden-1-yl]-4-ethyl-5-[(1E)-N-hydroxypropanimidoyl]pyrimidin-2-amin (Bsp.:1.40a) und N-[(1R,2S)-2,6-Dimethyl-2,3-dihydro-1H-inden-1-yl]-4-ethyl-5-[(1Z)-N-hydroxypropanimidoyl]pyrimidin-2-amin (Bsp.: 1.40b)

5,0 g (39 mmol) 3,5-Heptandion und 7,0 g (7.8 ml, 58,5 mmol) N,N-Dimethylformamiddimethylacetal werden in 10 ml Methanol beim Raumtemperatur zusammen gegeben. Die Mischung wird dann 120 min bei 65 - 70 °C gerührt, nach eingengen am Rotavapor unter vermindertem Druck werden 7,8 g 4-(dimethylaminomethylene)heptane-3,5-dione mit ca 90 %iger Reinheit erhalten, das ohne weitere Reinigung in die Folgestufe eingesetzt werden kann.

7,8 g (90 %, 38 mmol) 4-(dimethylaminomethylene)heptane-3,5-dione und 5,9 g (21 mmol) [amino(methylsulfanyl)methylene]ammonium sulfate werden in 10 ml Methanol MeOH vorgelegt, dann werden 9 g (9.1 ml, 50 mmol) 30%ige Natriummethylat in Methanol zugeben. Die Mischung wird 60 Minuten auf Rückfluß erhitzt. Das Rohgemisch wird auf Kieselgel aufgezogen und säulenchromatographisch mit Heptan/Essigester als Laufmittel gereinigt. Nach Einengen werden 1,9 g 1-(4-ethyl-2-methylsulfanyl-pyrimidin-5-yl)propan-1-one (fest, Schmelzpunkt 71,7 °C).

In 25 ml Chloroform werden 1,68 g (8 mmol) 1-(4-ethyl-2-methylsulfanyl-pyrimidin-5-yl)propan-1-one vorgelegt und im Eisbad auf 0-5 °C abgekühlt. Unter gutem Rühren werden portionsweisse 2,8 g (12 mmol) meta-Chlorperbenzoesaeure (73 %) zu gegeben. Diese Reaktionsmisschung wird auf Raumtemperatur kommen gelassen und zwei Stunden gerührt. Danach gibt man 4,3 g (5,9 ml, 40 mmol) Triethylamin und anschließend 1.55 g (9,6 mol) (1R,2S)-2,6-dimethyl-2,3-dihydro-1H-inden-1-amine hinzu und rührt 20 Stunden bei Raumtemperatur weiter. Die Reaktionsmischung wird auf Kieselgel aufgezogen und säulenchromatographisch mit Heptan/Essigester als Laufmittel gereinigt. Nach Einengen werden 2,3 g 1-[2-[[(1R,2S)-2,6-dimethylindan-1-yl]amino]-4-ethyl-pyrimidin-5-yl]propan-1-one (fest, Schmelzpunkt 61,5 °C) erhalten (Ausbeute 86 % bei 95 % Reinheit).

Zu 1,7 g (5 mmol) 1-[2-[[(1R,2S)-2,6-dimethylindan-1-yl]amino]-4-ethyl-pyrimidin-5-yl]propan-1-one in 10 ml Methanol werden 0.7 g (10 mmol) Hydroxylamin-Hydrochlorid 97% und dann 1,5 g (1,6 ml, 8,5 mmol) Natriummethylat 30%in Methanol zugeben. Die Mischung wird 240 min. auf leichten Rückfluss erhitzt. Das so erhaltene Rohgemisch wird auf Kieselgel aufgezogen und säulenchromatographisch mit Heptan/Essigester als Laufmittel gereinigt. Nach Einengen werden 0,96 g N-[(1R,2S)-2,6-Dimethyl-2,3-dihydro-1H-inden-1-yl]-4-ethyl-5-[(1Z)-N-hydroxypropanimidoyl]pyrimidin-2-amin (wachsartig) (Ausbeute 51 % bei 90 % Reinheit) und 0,53 g N-[(1R,2S)-2,6-Dimethyl-2,3-dihydro-1H-inden-1-yl]-4-ethyl-5-[(1E)-N-hydroxypropanimidoyl]pyrimidin-2-amin (wachsartig) (Ausbeute 28 % bei 90 % Reinheit) erhalten.

### 5-{(1Z)-N-[(4-Chlorbenzyl)oxy]propanimidoyl}-N-[(1R,2S)-2,6-dimethyl-2,3-dihydro-1H-inden-1-yl]-4-ethylpyrimidin-2-amin (Bsp.:1.115b)

0,15 g (0.44 mmol) N-[(1R,2S)-2,6-Dimethyl-2,3-dihydro-1H-inden-1-yl]-4-ethyl-5-[(1Z)-N-hydroxypropanimidoyl]pyrimidin-2-amin werden in Dioxan gelöst, 0,2 g (4,8 mmol) Natriumhydrid (60 % in Parrafinöl) werden zugeben (Wasserstoffentwicklung beachten), Nach 10 min rühren werden 0.109 g (0,53 mmol) 4-Chlorbenzylbromid zugeben und Reaktionsmischung wird 2 Stunden bei 60°C gerührt. Das so erhaltene Rohgemisch wird auf Kieselgel aufgezogen und säulenchromatographisch mit Heptan/Essigester als Laufmittel gereinigt. Nach Einengen werden 0,068 g 5-{(1Z)-N-[(4-Chlorbenzyl)oxy]propanimidoyl}-N-[(1R,2S)-2,6-dimethyl-2,3-dihydro-1H-inden-1-yl]-4-ethylpyrimidin-2-amin (wachsartig) (Ausbeute 32 % bei 95 % Reinheit) erhalten.

### N-[(1R,2S)-2,6-Dimethyl-2,3-dihydro-1H-inden-1-yl]-5-[(1E)-N-ethoxyethanimidoyl]pyrimidin-2-amin (Bsp.:1.103a) und N-[(1R,2S)-2,6-Dimethyl-2,3-dihydro-1H-inden-1-yl]-5-[(1Z)-N-ethoxyethanimidoyl]pyrimidin-2-amin (Bsp.:1.103b)

Zwei mal werden 2 g (12.8 mmol) 1-(2-chloropyrimidin-5-yl)ethanone, 2,7 g (16,6 mmol) (1R,2S)-2,6-dimethyl-2,3-dihydro-1H-inden-1-amine und 3,9 g (5,3 ml, 38,3 mmol) Triethylamin in 5 ml 1,4-Dioxan in einem Mikrowellenvial vorgelegt und nach Verschließen 45 min. bei 140 Grad in der Mikrowelle (Biotage Initiator, http://www.biotage.com/product-page/biotage-initiator) erhitzt. Die beiden Reaktionsgemische werden vereint, auf Wasser gegeben und dreimal mit Essigester extrahiert. Die organische Phase wird getrochnet und einrotiert. Die dann erhaltenen 9.4 g Rohgemsich werden auf Kieselgel aufgezogen und säulenchromatographisch mit Heptan/Essigester als Laufmittel gereinigt. Nach Einengen werden 5,1 g 1-[2-[[(1R,2S)-2,6-dimethylindan-1-yl]amino]pyrimidin-5-yl]ethanone (fest) erhalten (Ausbeute 68 % bei 95 % Reinheit).

Zu 0,25 g (0,84 mmol) 1-[2-[[(1R,2S)-2,6-dimethylindan-1-yl]amino]pyrimidin-5-yl]ethanone in 4 ml Methanol werden 0.1 g (1,7 mmol) O-Ethylhydroxylamin und dann 0,3 g (0,3 ml 1,7 mmol) Natriummethylat 30% in Methanol zugeben. Die Mischung wird 120 min. auf Rückfluss erhitzt. Dann werden nochmals 0,3 g (0,3 ml 1,7 mmol) Natriummethylat 30%in Methanol zugeben und die Mischung wird weitere 120 min. auf Rückfluss erhitzt. Das so erhaltene Rohgemisch wird auf Kieselgel aufgezogen und säulenchromatographisch mit Heptan/Essigester als Laufmittel gereinigt. Nach Einengen werden 0,076 g N-[(1R,2S)-2,6-Dimethyl-2,3-dihydro-1H-inden-1-yl]-5-[(1Z)-N-ethoxyethanimidoyl]pyrimidin-2-amin (wachsartig) (Ausbeute 26 % bei 95 % Reinheit) und 0,035 g N-[(1R,2S)-2,6-Dimethyl-2,3-dihydro-1H-inden-1-yl]-5-[(1E)-N-ethoxyethanimidoyl]pyrimidin-2-amin (wachsartig) (Ausbeute 12 % bei 95 % Reinheit) erhalten.

### N-[(1R,2S)-2,6-Dimethyl-2,3-dihydro-1H-inden-1-yl]-5-[(1E)-N-(4-fluorphenoxy)ethanimidoyl]pyrimidin-2-amin(Bsp.:1.101a) und N-[(1R,2S)-2,6-Dimethyl-2,3-dihydro-1H-inden-1-yl]-5-[(1Z)-N-(4-fluorphenoxy)ethanimidoyl]pyrimidin-2-amin (Bsp.:1.101b)

Zu 0,25 g (0,84 mmol) 1-[2-[[(1R,2S)-2,6-dimethylindan-1-yl]amino]pylimidin-5-yl]ethanone in 4 ml Methanol werden 0.277 g (1,7 mmol) (4-fluorophenoxy)ammonium chloride und dann 0,3 g (0,3 ml 1,7 mmol) Natriummethylat 30%in Methanol zugeben. Die Mischung wird 120 min. auf Rückfluss erhitzt. Dann werden nochmals 0,3 g (0,3 ml 1,7 mmol) Natriummethylat 30%in Methanol zugeben und die Mischung wird weitere 120 min. auf Rückfluss erhitzt. Das so erhaltene Rohgemisch wird auf Kieselgel aufgezogen und säulenchromatographisch mit Heptan/Essigester als Laufmittel gereinigt. Nach Einengen werden 0,057 g N-[(1R,2S)-2,6-Dimethyl-2,3-dihydro-1H-inden-1-yl]-5-[(1Z)-N-(4-fluorphenoxy)ethanimidoyl]pyrimidin-2-amin (wachsartig) (Ausbeute 16 % bei 90 % Reinheit) erhalten. Bei einer weiteren säulenchromatographischen Trennung einer Mischfraktion werden 0,026 g N-[(1R,2S)-2,6-Dimethyl-2,3-dihydro-1H-inden-1-yl]-5-[(1E)-N-(4-fluorphenoxy)ethanimidoyl]pyrimidin-2-amin (wachsartig) (Ausbeute 8 % bei 95 % Reinheit) und 0,154 g N-[(1R,2S)-2,6-Dimethyl-2,3-dihydro-1H-inden-1-yl]-5-[(1Z)-N-(4-fluorphenoxy)ethanimidoyl]pyrimidin-2-amin (wachsartig) (Ausbeute 42 % bei 90 % Reinheit) erhalten.

### N-[(1R,2S)-2,6-Dimethyl-2,3-dihydro-1H-inden-1-yl]-5-[(1E)-N-hydroxypentanimidoyl]pyrimidin-2-amin (Bsp.:1.85a) und N-[(1R,2S)-2,6-Dimethyl-2,3-dihydro-1H-inden-1-yl]-5-[(1Z)-N-hydroxypentanimidoyl] pyrimidin-2-amin (Bsp.: 1.85b)

2,5 g (12.9 mmol) 5-bromo-2-chloro-pyrimidine, 2,5 g (15,5 mmol) (1R,2S)-2,6-dimethyl-2,3-dihydro-1H-inden-1-amine und 2,6 g (3,6 ml, 25,8 mmol) Triethylamin werden in 10 ml 1,4-Dioxan in einem Mikrowellenvial vorgelegt und nach Verschließen 120 min. bei 120 Grad in der Mikrowelle (Biotage Initiator, http://www.biotage.com/product-page/biotage-initiator) erhitzt. Das Reaktionsgemische wird auf Wasser gegeben und dreimal mit Essigester extrahiert. Die organische Phase wird getrochnet und einrotiert. Das Rohgemsich wird säulenchromatographisch mit Heptan/Essigester als Laufmittel gereinigt. Nach Einengen werden 3,3 g 5-bromo-N-[(1R,2S)-2,6-dimethylindan-1-yl]pyrimidin-2-amine erhalten (Ausbeute 68 % bei 85 % Reinheit).

1,5 g (4,7 mmol) 5-bromo-N-[(1R,2S)-2,6-dimethylindan-1-yl]pyrimidin-2-amine, 0,64 g (9,4 mmol) 1-Pentyne, 0,2 g (0,28 mmol) Bis(triphenylphosphin)palladium(II)chlorid und 0.036 g (0,19 mmol) Kupfer(I)iodid werden in 10 ml Triethylamin 8 Stunden unter rühren auf 80 °C erhitzt. Die Reaktionslösung wird bis zum Rückstand eingeengt.

Der Rückstand wird säulenchromatographisch mit Heptan/Essigester als Laufmittel gereinigt. Nach Einengen werden 0,82 g N-[(1R,2S)-2,6-dimethylindan-1-yl]-5-pent-1-ynyl-pyrimidin-2-amine erhalten (Ausbeute 54 % bei 95 % Reinheit).

0,6 g (1,8 mmol) N-[(1R,2S)-2,6-dimethylindan-1-yl]-5-pent-1-ynyl-pyrimidin-2-amine und 0,06 g (0,35 mmol) 4-Toluolsulfonsaeure werden 0,5 ml Waser und 2 ml Ethanol in einem Mikrowellenvial vorgelegt und nach Verschließen 45 min. bei 120 Grad in der Mikrowelle (Biotage Initiator, http://www.biotage.com/product-page/biotage-initiator) erhitzt. Das Rohgemsich wird auf Kieselgel aufgezogen und säulenchromatographisch mit Heptan/Essigester als Laufmittel gereinigt. Nach Einengen werden 0,22 g 1-[2-[[(1R,2S)-2,6-dimethylindan-1-yl]amino]pyrimidin-5-yl]pentan-1-one erhalten (Ausbeute 36 % bei 95 % Reinheit).

Zu 0,33 g (90 %ig, 0,94 mmol) 1-[2-[[(1R,2S)-2,6-dimethylindan-1-yl]amino]pyrimidin-5-yl]pentan-1-one in 2 ml Methanol werden 0.097 g (1,7 mmol) Hydroxylamin-Hydrochlorid 97% und dann 0,22 g (0,22 ml 1,2 mmol) Natriummethylat 30% in Methanol zugeben. Die Mischung wird 120 min. auf Rückfluss erhitzt. Das so erhaltene Rohgemisch wird auf Kieselgel aufgezogen und säulenchromatographisch mit Heptan/Essigester als Laufmittel gereinigt. Nach Einengen werden 0,108 g N-[(1R,2S)-2,6-Dimethyl-2,3-dihydro-1H-inden-1-yl]-5-[(1Z)-N-hydroxypentanimidoyl]pyrimidin-2-amin (wachsartig) (Ausbeute 30 % bei 90 % Reinheit) und 0,034 N-[(1R,2S)-2,6-Dimethyl-2,3-dihydro-1H-inden-1-yl]-5-[(1E)-N-hydroxypentanimidoyl]pyrimidin-2-amin (wachsartig) (Ausbeute 10 % bei 90 % Reinheit) erhalten.

**Tabelle 3: Chemisch Physikalische Charakterisierung ausgewählter Synthesebeispiele**

| **Verbindung** | **Beschreibung** |
|---|---|
| **1.10a** | fest; logp (HCOOH): 3.00; 1H-NMR (CDCl₃, 400 MHZ, δ in ppm): 1.25 (d, 3H, CH₃); 2.20 (s, 3H, CH₃); 2.30 (s, 3H, CH₃); 2.35 (m, 1H, 1H von CH₂); 2.50 (m, 1H, 1H von CH₂); 3.05 (m, 1H, 1H von CH); 5.25 (t, 1H, CH); 5.80 (br, 1H, NH); 7.00 - 7.10 (m, 3H, Ar-H); 7.80 (br, 1H, OH); 8.55 (br, 2H, Pyr-4H, Pyr-6H); |
| **1.26a** | oelig; logp (HCOOH): 2.93; 1H-NMR (CDCl₃, 400 MHZ, δ in ppm): 1.25 (d, 3H, CH₃); 2.05 (s, 3H, CH₃); 2.30 (m+s, 7H, 1H von CH_{2;} 2*CH₃); 2.50 (m, 1H, 1H von CH₂); 3.05 (m, 1H, 1H von CH); 5.25 (t, 1H, CH); 5.90 (br, 1H, NH); 6.95 - 7.10 (m, 3H, Ar-H); 8.00 (br, 1H, OH); 8.70 (br, 1H, Pyr-6H); |
| **1.26b** | oelig; logp (HCOOH): 3.07; 1H-NMR (CDCl₃, 400 MHZ, δ in ppm): 1.25 (d, 3H, CH₃); 2.20 (m+s, 4H, 1H von CH_{2;} CH₃); 2.30 (s, 3H, CH₃); 2.35 (s, 3H, CH₃); 2.50 (m, 1H, 1H von CH₂); 3.00 (m, 1H, 1H von CH); 5.25 (t, 1H, CH); 5.85 (br, 1H, NH); 6.95 - 7.10 (m, 3H, Ar-H); 8.15 (br, 1H, OH); 8.90 (br, 1H, Pyr-6H); |
| **1.40a** | fest; logp (HCOOH): 3.71; 1H-NMR (CDCl₃, 400 MHZ, δ in ppm): 1.10 (t, 3H, CH₃); 1.25 (t, 3H, CH₃); 1.30 (d, 3H, CH₃); 2.30 (m+s, 4H, 1H von CH_{2;}CH₃); 2.45 (q, 2H, CH₂); 2.50 (m, 3H, CH₂, 1H von CH₂); 3.05 (m, 1H, 1H von CH); 5.25 (t, 1H, CH); 5.60 (br, 1H, NH); 6.95 - 7.10 (m, 3H, Ar-H); 7.80 (br, 1H, OH); 8.00 (br, 1H, Pyr-6H); |
| **1.40b** | fest; logp (HCOOH): 3.86; 1H-NMR (CDCl₃, 400 MHZ, δ in ppm): 1.10 (t, 3H, CH₃); 1.20 (t, 3H, CH₃); 1.25 (d, 3H, CH₃); 2.25 (m, 1H, 1H von CH₂); 2.50 (m, 1H, 1H von CH2); 2.65 (q, 2H, CH₂); 2.70 (q, 2H, CH₂); 3.05 (m, 1H, 1H von CH); 5.25 (t, 1H, CH); 5.80 (br, 1H, NH); 6.95 - 7.10 (m, 3H, Ar-H); 8.00 (br, 1H, OH); 8.70 (br, 1H, Pyr-6H); |
| **1.85a** | oelig; logp (HCOOH): 3.86; 1H-NMR (CDCl₃, 400 MHZ, δ in ppm): ); 0.85 (t, 3H, CH₃); 1.25 (d+m, 5H, CH_{2;} CH₃); 1.40 (m, 2H, CH₂); 2.30 (m+s, 4H, 1H von CH_{2;} CH₃); 2.45 (m, 2H, CH₂); 2.50 (m, 1H, 1H von CH₂); 3.05 (m, 1H, 1H von CH); 5.30 (t, 1H, CH); 6.10 (br, 1H, NH); 6.95 - 7.10 (m, 3H, Ar-H); 8.30 (br, 1H, OH); 8.65 (br, 2H, Pyr-4H, Pyr-6H); |
| **1.85b** | oelig; logp (HCOOH): 4.10; 1H-NMR (CDCl₃, 400 MHZ, δ in ppm):); 0.85 (t, 3H, CH₃); 1.25 (d, 3H, CH_{2;} CH₃); 1.40 (m, 2H, CH₂); 1.50 (m, 2H, CH₂); 2.30 (m+s, 4H, 1H von CH_{2;}CH₃); 2.50 (m, 1H, 1H von CH₂); 2.70 (m, 2H, CH₂); 3.05 (m, 1H, 1H von CH); 5.25 (t, 1H, CH); 5.80 (br, 1H, NH); 6.95 - 7.10 (m, 3H, Ar-H); 8.15 (br, 1H, OH); 8.50 (br, 2H, Pyr-4H, Pyr-6H); |
| **1.97b** | oelig; logp (HCOOH): 4.60; 1H-NMR (CDCl₃, 400 MHZ, δ in ppm): 1.25 (d, 3H, CH₃); 2.15 (s, 3H, CH₃); 2.25 (m, 1H, 1H von CH₂); 2.30 (s, 3H, CH₃); 2.40 (s, 3H, CH₃); 2.50 (m, 1H, 1H von CH2); 3.05 (m, 1H, 1H von CH); 3.95 (s, 3H, OCH₃); 5.30 (br, 2H, CH, NH); 6.95 - 7.10 (m, 3H, Ar-H); 8.15 (br, 1H, Pyr-6H); |
| **1.98a** | oelig; logp (HCOOH): 4.51; 1H-NMR (CDCl₃, 400 MHZ, δ in ppm): 1.25 (d, 3H, CH₃); 2.15 (s, 3H, CH₃); 2.25 (m+s, 4H, 1H von CH₂, CH₃); 2.50 (m, 1H, 1H von CH2); 3.05 (m, 1H, 1H von CH); 3.95 (s, 3H, OCH₃); 5.25 (t, 1H, CH); 5.50 (br, 1H, NH); 6.95 - 7.10 (m, 3H, Ar-H); 8.55 (br, 2H, Pyr-4H, Pyr-6H); |
| **1.98b** | oelig; logp (HCOOH): 4.12; 1H-NMR (CDCl₃, 400 MHZ, δ in ppm): 1.25 (d, 3H, CH₃); 2.20 (s, 3H, CH₃); 2.30 (m+s, 4H, 1H von CH₂, CH₃); 2.50 (m, 1H, 1H von CH2); 3.05 (m, 1H, 1H von CH); 3.90 (s, 3H, OCH₃); 5.25 (t, 1H, CH); 5.50 (br, 1H, NH); 6.95 - 7.10 (m, 3H, Ar-H); 8.70 (br, 2H, Pyr-4H, Pyr-6H); |
| **1.99a** | oelig; logp (HCOOH): 4.01; 1H-NMR (CDCl₃, 400 MHZ, δ in ppm): 1.25 (d, 3H, CH₃); 2.20 (s, 3H, CH₃); 2.25 (m+s, 4H, 1H von CH₂, CH₃); 2.45 (s, 1H, CH); 2.50 (m, 1H, 1H von CH2); 3.05 (m, 1H, 1H von CH); 4.70 (s, 2H, OCH₂); 5.25 (t, 1H, CH); 5.50 (br, 1H, NH); 6.95 - 7.10 (m, 3H, Ar-H); 8.75 (br, 2H, Pyr-4H, Pyr-6H); |
| **1.99b** | oelig; logp (HCOOH): 4.26; 1H-NMR (CDCl₃, 400 MHZ, δ in ppm): 1.25 (d, 3H, CH₃); 2.20 (s, 3H, CH₃); 2.25 (m+s, 4H, 1H von CH₂, CH₃); 2.45 (s, 1H, CH); 2.50 (m, 1H, 1H von CH2); 3.05 (m, 1H, 1H von CH); 4.75 (s, 2H, OCH₂); 5.25 (t, 1H, CH); 5.45 (br, 1H, NH); 6.95 - 7.10 (m, 3H, Ar-H); 8.60 (br, 2H, Pyr-4H, Pyr-6H); |
| **1.101a** | oelig; logp (HCOOH): 5.34; 1H-NMR (CDCl₃, 400 MHZ, δ in ppm): 1.25 (d, 3H, CH₃2.30 (m+s, 7H, 1H von CH_{2;}2^{∗}CH₃); 2.55 (s, 1H, CH); 3.05 (m, 1H, 1H von CH); 5.30 (t, 1H, CH); 5.70 (br, 1H, NH); 6.95 - 7.20 (m, 7H, Ar-H); 8.75 (br, 2H, Pyr-4H, Pyr-6H); |
| **1.101b** | oelig; logp (HCOOH): 5.56; 1H-NMR (CDCl₃, 400 MHZ, δ in ppm): 1.25 (d, 3H, CH₃); 2.30 (m+s, 4H, 1H von CH₂); 2.35 (s, 3H, CH₃); 2.55 (s, 1H, CH); 3.05 (m, 1H, 1H von CH); 5.30 (t, 1H, CH); 5.60 (br, 1H, NH); 6.95 - 7.25 (m, 7H, Ar-H); 8.65 (br, 2H, Pyr-4H, Pyr-6H); |
| **1.102a** | oelig; logp (HCOOH): 5.43; 1H-NMR (CDCl₃, 400 MHZ, δ in ppm): 0.95 (d, 6H, CH₃); 1.25 (d, 3H, CH₃); 2.05 (m, 1H, CH); 2.20 (s, 3H, CH₃); 2.30 (m+s, 4H, 1H von CH₂,CH₃); 2.50 (m, 1H, 1H von CH2); 3.05 (m, 1H, 1H von CH); 3.90 (d, 2H, OCH₂); 5.25 (t, 1H, CH); 5.50 (br, 1H, NH); 6.95 - 7.10 (m, 3H, Ar-H); 8.75 (br, 2H, Pyr-4H, Pyr-6H); |
| **1.102b** | oelig; logp (HCOOH): 5.99; 1H-NMR (CDCl₃, 400 MHZ, δ in ppm): 0.95 (d, 6H, CH₃); 1.25 (d, 3H, CH₃); 2.05 (m, 1H, CH); 2.15 (s, 3H, CH₃); 2.30 (m+s, 4H, 1H von CH₂, CH₃); 2.50 (m, 1H, 1H von CH2); 3.05 (m, 1H, 1H von CH); 3.90 (d, 2H, OCH₂); 5.25 (t, 1H, CH); 5.50 (br, 1H, NH); 6.95 - 7.10 (m, 3H, Ar-H); 8.50 (br, 2H, Pyr-4H, Pyr-6H); |
| **1.103a** | oelig; 1H-NMR (CDCl₃, 400 MHZ, δ in ppm): 1.25 (d+t, 6H, 2^{∗}CH₃); 2.15 (s, 3H, CH₃); 2.30 (m+s, 4H, 1H von CH₂,CH₃); 2.50 (m, 1H, 1H von CH2); 3.05 (m, 1H, 1H von CH); 4.15 (q, 2H, OCH₂); 5.30 (t, 1H, CH); 5.50 (br, 1H, NH); 6.95 - 7.10 (m, 3H, Ar-H); 8.75 (br, 2H, Pyr-4H, Pyr-6H); |
| **1.103b** | oelig; logp (HCOOH): 4.92; 1H-NMR (CDCl₃, 400 MHZ, δ in ppm): 1.25 (d, 3H, CH₃); 1.30 (t, 3H, CH₃); 2.15 (s, 3H, CH₃); 2.30 (m+s, 4H, 1H von CH₂,CH₃); 2.50 (m, 1H, 1H von CH2); 3.05 (m, 1H, 1H von CH); 4.20 (q, 2H, OCH₂); 5.30 (t, 1H, CH); 5.50 (br, 1H, NH); 6.95 - 7.10 (m, 3H, Ar-H); 8.50 (br, 2H, Pyr-4H, Pyr-6H); |
| **1.104a** | oelig; logp (HCOOH): 5.00; 1H-NMR (CDCl₃, 400 MHZ, δ in ppm): 1.25 (2^{∗}d, 9H, 3^{∗}CH₃); 2.20 (s, 3H, CH₃); 2.30 (m+s, 4H, 1H von CH₂, CH₃); 2.50 (m, 1H, 1H von CH2); 3.05 (m, 1H, 1H von CH); 4.35 (m, 1H, OCH); 5.30 (t, 1H, CH); 5.45 (br, 1H, NH); 6.95 - 7.10 (m, 3H, Ar-H); 8.80 (br, 2H, Pyr-4H, Pyr-6H); |
| **1.104b** | oelig; logp (HCOOH): 5.51; 1H-NMR (CDCl₃, 400 MHZ, δ in ppm): 1.25 (2^{∗}d, 9H, 3^{∗}CH₃); 2.15 (s, 3H, CH₃); 2.30 (m+s, 4H, 1H von CH₂, CH₃); 2.50 (m, 1H, 1H von CH2); 3.05 (m, 1H, 1H von CH); 4.40 (m, 1H, OCH); 5.30 (t, 1H, CH); 5.45 (br, 1H, NH); 6.95 - 7.10 (m, 3H, Ar-H); 8.50 (br, 2H, Pyr-4H, Pyr-6H); |
| **1.105a** | oelig; logp (HCOOH): 5.71; 1H-NMR (CDCl₃, 400 MHZ, δ in ppm): 1.25 (d, 3H, CH₃); 2.15 (s, 3H, CH₃); 2.30 (m+s, 4H, 1H von CH₂, CH₃); 2.50 (m, 1H, 1H von CH2); 3.05 (m, 1H, 1H von CH); 5.15 (2, 2H, OCH₂); 5.30 (t, 1H, CH); 5.55 (br, 1H, NH); 6.95 - 7.30 (m, 7H, Ar-H); 8.75 (br, 2H, Pyr-4H, Pyr-6H); |
| **1.105b** | oelig; logp (HCOOH): 6.05; 1H-NMR (CDCl₃, 400 MHZ, δ in ppm): 1.25 (d, 3H, CH₃); 2.15 (s, 3H, CH₃); 2.30 (m+s, 4H, 1H von CH₂, CH₃); 2.50 (m, 1H, 1H von CH2); 3.05 (m, 1H, 1H von CH); 5.10 (2, 2H, OCH₂); 5.30 (t, 1H, CH); 5.55 (br, 1H, NH); 6.95 - 7.30 (m, 7H, Ar-H); 8.55 (br, 2H, Pyr-4H, Pyr-6H); |
| **1.106a** | oelig; logp (HCOOH): 4.47; 1H-NMR (CDCl₃, 400 MHZ, δ in ppm): 1.25 (d, 3H, CH₃); 2.20 (s, 3H, CH₃); 2.30 (m+s, 4H, 1H von CH₂, CH₃); 2.50 (m, 1H, 1H von CH2); 3.05 (m, 1H, 1H von CH); 5.20 (2, 2H, OCH₂); 5.30 (t, 1H, CH); 5.65 (br, 1H, NH); 6.95 - 7.10 (m, 3H, Ar-H); 7.25 (m, 1H, Ar-H); 7.70 (m, 1H, Ar-H); 8.30 (m, 1H, Ar-H); 8.75 (br, 2H, Pyr-4H, Pyr-6H); |
| **1.106b** | oelig; logp (HCOOH): 4.81; 1H-NMR (CDCl₃, 400 MHZ, δ in ppm): 1.25 (d, 3H, CH₃); 2.20 (s, 3H, CH₃); 2.25 (m+s, 4H, 1H von CH₂, CH₃); 2.50 (m, 1H, 1H von CH2); 3.05 (m, 1H, 1H von CH); 5.25 (t, 1H, CH); 5.30 (2, 2H, OCH₂); 5.65 (br, 1H, NH); 6.95 - 7.10 (m, 3H, Ar-H); 7.25 (m, 1H, Ar-H); 7.70 (m, 1H, Ar-H); 8.30 (m, 1H, Ar-H); 8.50 (br, 2H, Pyr-4H, Pyr-6H); |
| **1.107a** | oelig; logp (HCOOH): 5.65; 1H-NMR (CDCl₃, 400 MHZ, δ in ppm): 1.25 (d, 3H, CH₃); 2.15 (s, 3H, CH₃); 2.25 (m+s, 4H, 1H von CH₂, CH₃); 2.30 (s, 3H, CH₃); 2.50 (m, 1H, 1H von CH₂); 3.05 (m, 1H, 1H von CH); 5.10 (2, 2H, OCH₂); 5.25 (t, 1H, CH); 5.50 (br, 1H, NH); 6.95 - 7.30 (m, 7H, Ar-H); 8.75 (br, 2H, Pyr-4H, Pyr-6H); |
| **1.107b** | oelig; logp (HCOOH): 6.01; 1H-NMR (CDCl₃, 400 MHZ, δ in ppm): 1.25 (d, 3H, CH₃); 2.15 (s, 3H, CH₃); 2.25 (m+s, 4H, 1H von CH₂, CH₃); 2.35 (s, 3H, CH₃); 2.50 (m, 1H, 1H von CH2); 3.05 (m, 1H, 1H von CH); 5.15 (2, 2H, OCH₂); 5.25 (t, 1H, CH); 5.50 (br, 1H, NH); 6.95 - 7.30 (m, 7H, Ar-H); 8.50 (br, 2H, Pyr-4H, Pyr-6H); |
| **1.108a** | oelig; logp (HCOOH): 5.46; 1H-NMR (CDCl₃, 400 MHZ, δ in ppm): 1.25 (d, 3H, CH₃); 2.15 (s, 3H, CH₃); 2.25 (m+s, 4H, 1H von CH₂, CH₃); 2.50 (m, 1H, 1H von CH2); 3.05 (m+t, 3H, 1H von CH₂, CH₂); 4.30 (t, 2H, CH₂); 5.25 (t, 1H, CH); 5.50 (br, 1H, NH); 6.95 - 7.35 (m, 8H, Ar-H); 8.70 (br, 2H, Pyr-4H, Pyr-6H); |
| **1.108b** | oelig; logp (HCOOH): 5.91; 1H-NMR (CDCl₃, 400 MHZ, δ in ppm): 1.25 (d, 3H, CH₃); 2.10 (s, 3H, CH₃); 2.25 (m+s, 4H, 1H von CH₂, CH₃); 2.50 (m, 1H, 1H von CH2); 3.05 (m+t, 3H, 1H von CH₂, CH₂); 4.35 (t, 2H, CH₂); 5.25 (t, 1H, CH); 5.50 (br, 1H, NH); 6.95 - 7.35 (m, 8H, Ar-H); 8.50 (br, 2H, Pyr-4H, Pyr-6H); |
| **1.109a** | oelig; logp (HCOOH): 5.86; 1H-NMR (CDCl₃, 400 MHZ, δ in ppm): 1.25 (d, 3H, CH₃); 2.20 (s, 3H, CH₃); 2.25 (m+s, 4H, 1H von CH₂, CH₃); 2.50 (m, 1H, 1H von CH₂); 3.00 (t, 2H, CH₂); 3.05 (m, 1H, 1H von CH₂); 4.30 (t, 2H, CH₂); 5.25 (t, 1H, CH); 5.60 (br, 1H, NH); 6.95 - 7.25 (m, 7H, Ar-H); 8.65 (br, 2H, Pyr-4H, Pyr-6H); |
| **1.109b** | oelig; logp (HCOOH): 6.28; 1H-NMR (CDCl₃, 400 MHZ, δ in ppm): 1.25 (d, 3H, CH₃); 2.20 (s, 3H, CH₃); 2.30 (m+s, 4H, 1H von CH₂, CH₃); 2.50 (m, 1H, 1H von CH₂); 3.00 (t, 2H, CH₂); 3.05 (m, 1H, 1H von CH₂); 4.35 (t, 2H, CH₂); 5.25 (t, 1H, CH); 5.50 (br, 1H, NH); 6.95 - 7.25 (m, 7H, Ar-H); 8.55 (br, 2H, Pyr-4H, Pyr-6H); |
| **1.111a** | oelig; logp (HCOOH): 5.65; 1H-NMR (CDCl₃, 400 MHZ, δ in ppm): 1.25 (d, 3H, CH₃); 1.30 (s, 9H, 3^{∗}CH₃); 2.20 (s, 3H, CH₃); 2.25 (m+s, 4H, 1H von CH₂, CH₃); 2.50 (m, 1H, 1H von CH2); 3.05 (m, 1H, 1H von CH₂); 5.25 (t, 1H, CH); 5.50 (br, 1H, NH); 6.95 - 7.10 (m, 3H, Ar-H); 8.80 (br, 2H, Pyr-4H, Pyr-6H); |
| **1.111b** | oelig; logp (HCOOH): 6.05; 1H-NMR (CDCl₃, 400 MHZ, δ in ppm): 1.25 (d, 3H, CH₃); 1.35 (s, 9H, 3^{∗}CH₃); 2.15 (s, 3H, CH₃); 2.25 (m+s, 4H, 1H von CH₂, CH₃); 2.50 (m, 1H, 1H von CH2); 3.05 (m, 1H, 1H von CH₂); 5.25 (t, 1H, CH); 5.45 (br, 1H, NH); 6.95 - 7.10 (m, 3H, Ar-H); 8.60 (br, 2H, Pyr-4H, Pyr-6H); |
| **1.112a** | oelig; logp (HCOOH): 4.47; 1H-NMR (CDCl₃, 400 MHZ, δ in ppm): 1.25 (d+t, 6H, 2^{∗}CH₃); 1.50 (s, 6H, 2^{∗}CH₃); 2.20 (s, 3H, CH₃); 2.25 (m+s, 4H, 1H von CH₂, CH₃); 2.50 (m, 1H, 1H von CH2); 3.05 (m, 1H, 1H von CH₂); 3.75 (s, 2H von CH₂); 4.10 (br, 2H von CH₂); 5.25 (t, 1H, CH); 5.50 (br, 1H, NH); 6.95 - 7.10 (m, 3H, Ar-H); 8.80 (br, 2H, Pyr-4H, Pyr-6H); |
| **1.112b** | oelig; logp (HCOOH): 4.66; 1H-NMR (CDCl₃, 400 MHZ, δ in ppm): 1.25 (d+t, 6H, 2^{∗}CH₃); 1.50 (s, 6H, 2^{∗}CH₃); 2.20 (s, 3H, CH₃); 2.25 (m+s, 4H, 1H von CH₂, CH₃); 2.50 (m, 1H, 1H von CH2); 3.05 (m, 1H, 1H von CH₂); 3.75 (s, 2H von CH₂); 4.10 (br, 2H von CH₂); 5.25 (t, 1H, CH); 5.50 (br, 1H, NH); 6.95 - 7.10 (m, 3H, Ar-H); 8.50 (br, 2H, Pyr-4H, Pyr-6H); |
| **1.119a** | fest; logp (HCOOH): 2.49; 1H-NMR (CDCl₃, 400 MHZ, δ in ppm): 1.85 (m, 3H, 1H von CH₂, CH₂); 2.05 (s, 3H, CH₃); 2.10 (m, 1H, 1H von CH2); 2.30 (s, 3H, CH₃); 2.80 (m, 2H, CH₂); 5.25 (br, 1H, CH); 5.75 (br, 1H, NH); 6.85 (m, 1H, Ar-H); 7.05 (m, 2H, Ar-H); 7.90 (br, 1H, =N-OH); 8.05 (br, 1H, Pyr-6H); |
| **1.119b** | fest; logp (HCOOH): 2.60; 1H-NMR (CDCl₃, 400 MHZ, δ in ppm): 1.85 (m, 3H, 1H von CH₂, CH₂); 2.10 (m, 1H, 1H von CH₂); 2.20 (s, 3H, CH₃); 2.40 (s, 3H, CH₃); 2.80 (m, 2H, CH₂); 5.25 (br, 1H, CH); 5.60 (br, 1H, NH); 6.85 (m, 1H, Ar-H); 7.05 (m, 2H, Ar-H); 8.15 (br, 1H, Pyr-6H); 8.70 (br, 1H, =N-OH); |
| **1.120a** | fest; logp (HCOOH): 3.65; 1H-NMR (CDCl₃, 400 MHZ, δ in ppm): 1.85 (m, 3H, 1H von CH₂, CH₂); 2.10 (m, 1H, 1H von CH2); 2.15 (s, 3H, CH₃); 2.30 (s, 3H, CH₃); 2.80 (m, 2H, CH₂); 3.85 (s, 3H, CH₃); 5.30 (br, 1H, CH); 5.45 (br, 1H, NH); 6.85 (m, 1H, Ar-H); 7.05 (m, 2H, Ar-H); 8.00 (br, 1H, Pyr-6H); |
| **1.120b** | fest; logp (HCOOH): 4.02; 1H-NMR (CDCl₃, 400 MHZ, δ in ppm): 1.85 (m, 3H, 1H von CH₂, CH₂); 2.05 (m, 1H, 1H von CH2); 2.10 (s, 3H, CH₃); 2.40 (s, 3H, CH₃); 2.80 (m, 2H, CH₂); 3.95 (s, 3H, CH₃); 5.35 (br, 2H, CH, NH); 6.85 (m, 1H, Ar-H); 7.05 (m, 2H, Ar-H); 8.20 (br, 1H, Pyr-6H); |
| **2.34a** | oelig; logp (HCOOH): 3.14; 1H-NMR (CDCl₃, 400 MHZ, δ in ppm): 1.85 (m, 5H, 1H von CH₂,2^{∗}CH₂); 2.10 (m, 1H, 1H von CH₂); 2.30 (s, 3H, CH₃); 2.80 (m, 6H, 3^{∗}CH₂); 5.30 (br, 1H, CH); 5.55 (br, 1H, NH); 6.85 (m, 2H, Ar-H); 7.05 (s, 1H, Ar-H); 7.60 (br, 1H, =N-OH); 8.85 (br, 1H, Pyr-6H); |
| **2.123a** | oelig; logp (HCOOH): 4.56; 1H-NMR (CDCl₃, 400 MHZ, δ in ppm): 1.25 (d, 3H, CH₃); 2.00 (m, 2H, 2H von CH₂); 2.30 (m+s, 4H, 1H von CH₂, CH₃); 2.50 (m, 3H, 1H von CH₂, CH₂); 2.75 (m, 2H, CH₂); 3.05 (m, 1H, 1H von CH); 3.95 (s, 3H, OCH₃); 5.30 (br, 1H, CH); 5.40 (br, 1H, NH); 6.95 - 7.10 (m, 3H, Ar-H); 9.50 (br, 1H, Pyr-6H); |
| **2.123b** | oelig; logp (HCOOH): 5.03; 1H-NMR (CDCl₃, 400 MHZ, δ in ppm): 1.25 (d, 3H, CH₃); 1.85 (m, 2H, 2H von CH₂); 2.25 (m+s, 4H, 1H von CH₂, CH₃); 2.50 (m, 1H, 1H von CH₂); 2.75 (m, 4H, 2^{∗}CH₂); 3.05 (m, 1H, 1H von CH); 3.95 (s, 3H, OCH₃); 5.30 (br, 1H, CH); 5.40 (br, 1H, NH); 6.95 - 7.10 (m, 3H, Ar-H); 8.80 (br, 1H, Pyr-6H); |
| **2.124b** | oelig; logp (HCOOH): 3.85; 1H-NMR (CDCl₃, 400 MHZ, δ in ppm): 1.85 (m, 3H, 1H von CH₂, CH₂); 2.75 (m, 5H, 1H von CH₂,2^{∗}CH₂); 2.90 (m, 1H, 1H von CH); 3.05 (m, 1H, 1H von CH); 3.95 (s, 3H, OCH₃); 5.45 (br, 1H, NH); 5.65 (m, 1H, CH); 7.15 - 7.30 (m, 4H, Ar-H); 8.80 (br, 1H, Pyr-6H); |
| **2.125a** | oelig; logp (HCOOH): 5.08; 1H-NMR (CDCl₃, 400 MHZ, δ in ppm): 1.25 (d, 3H, CH₃); 1.30 (t, 3H, CH₃); 2.00 (m, 2H, 2H von CH₂); 2.30 (m+s, 4H, 1H von CH₂, CH₃); 2.50 (m, 3H, 1H von CH₂, CH₂); 2.75 (m, 2H, CH₂); 3.05 (m, 1H, 1H von CH); 4.15 (q, 2H, OCH₂); 5.35 (br, 2H, CH, NH); 6.95 - 7.10 (m, 3H, Ar-H); 9.50 (br, 1H, Pyr-6H); |
| **2.125b** | oelig; logp (HCOOH): 5.56; 1H-NMR (CDCl₃, 400 MHZ, δ in ppm): 1.25 (d, 3H, CH₃); 1.30 (t, 3H, CH₃); 1.85 (m, 2H, 2H von CH₂); 2.30 (m+s, 4H, 1H von CH₂, CH₃); 2.50 (m, 1H, 1H von CH₂); 2.70 (m, 2H, CH₂); 2.75 (m, 2H, CH₂); 3.05 (m, 1H, 1H von CH); 4.20 (q, 2H, OCH₂); 5.35 (br, 2H, CH, NH); 6.95 - 7.10 (m, 3H, Ar-H); 8,95 (br, 1H, Pyr-6H); |
| **2.126b** | oelig; logp (HCOOH): 4.90; 1H-NMR (CDCl₃, 400 MHZ, δ in ppm): 1.30 (t, 3H, CH₃); 1.85 (m, 5H, 1H von CH₂, 2∗CH₂); 2.15 (m, 1H, 1H von CH₂); 2.70 (m, 6H, 3^{∗}CH₂); 4.20 (q, 2H, OCH₂); 5.35 (br, 2H, CH, NH); 7.05 - 7.35 (m, 4H, Ar-H); 8.95 (br, 1H, Pyr-6H); |
| **2.127a** | oelig; logp (HCOOH): 4.39; 1H-NMR (CDCl₃, 400 MHZ, δ in ppm): 1.85 (m, 5H, 1H von CH₂,2∗CH₂); 2.10 (m, 1H, 1H von CH₂); 2.70 (m, 6H, 3^{∗}CH₂); 3.95 (s, 3H, OCH₃); 5.35 (br, 1H, CH); 5.50 (br, 1H, NH); 7.05 - 7.20 (m, 3H, Ar-H); 7.35 (m, 3H, Ar-H); 8.95 (br, 1H, Pyr-6H); |
| **2.128a** | oelig; logp (HCOOH): 4.03; 1H-NMR (CDCl₃, 400 MHZ, δ in ppm): 1.35 (t, 3H, CH₃); 1.85 (m, 1H, 1H von CH₂); 1.90 (m, 2H, CH₂); 2.50 (m, 2H, CH₂); 2.65 (m, 1H, 1H von CH₂); 2.75 (m, 2H, CH₂); 2.90 (m, 1H, 1H von CH); 3.00 (m, 1H, 1H von CH); 4.15 (q, 2H, OCH₂); 5.50 (br, 1H, NH); 5.65 (m, 1H, CH); 7.15 - 7.30 (m, 4H, Ar-H); 9.50 (br, 1H, Pyr-6H); |
| **2.128b** | oelig; logp (HCOOH): 4.46; 1H-NMR (CDCl₃, 400 MHZ, δ in ppm): 1.35 (t, 3H, CH₃); 1.85 (m, 3H, 1H von CH₂, CH₂); 2.70 (m, 7H, 1H von CH, 3^{∗}CH₂); 2.90 (m, 1H, 1H von CH); 3. 00 (m, 1H, 1H von CH); 4.15 (q, 2H, OCH₂); 5.50 (br, 1H, NH); 5.65 (m, 1H, CH); 7.15 - 7.30 (m, 4H, Ar-H); 8.85 (br, 1H, Pyr-6H); |
| **2.130a** | oelig; logp (HCOOH): 4.41; 1H-NMR (CDCl₃, 400 MHZ, δ in ppm): 1.85 (m, 3H, 1H von CH₂, CH₂); 2.30 (s, 3H, CH₃); 2.70 (m, 5H, 1H von CH, 2^{∗}CH₂); 2.85 (m, 1H, 1H von CH); 3.00 (m, 1H, 1H von CH); 3.95 (s, 3H, OCH₃); 5.35 (br, 1H, NH); 5.60 (m, 1H, CH); 7.15 - 7.30 (m, 4H, Ar-H); 8.85 (br, 1H, Pyr-6H); |
| **2.130b** | oelig; logp (HCOOH): 5.36; 1H-NMR (CDCl₃, 400 MHZ, δ in ppm): 1.85 (m, 1H, 1H von CH₂); 1.95 (m, 2H, CH₂); 2.30 (s, 3H, CH₃); 2.50 (m, 2H, CH₂); 2.65 (m, 1H, 1H von CH₂); 2.70 (m, 2H, CH₂); 2.85 (m, 1H, 1H von CH); 3.00 (m, 1H, 1H von CH); 3.95 (s, 3H, OCH₃); 5.45 (br, 1H, NH); 5.65 (m, 1H, CH); 7.15 - 7.30 (m, 4H, Ar-H); 9.50 (br, 1H, Pyr-6H); |
| **2.131a** | oelig; logp (HCOOH): 5.90; 1H-NMR (CDCl₃, 400 MHZ, δ in ppm): 1.60 (m, 2H, 2^{∗}1H von CH₂), 1.70 (m, 2H, 2^{∗}1H von CH₂),1.85 (m, 7H, 1H von CH₂, 3^{∗}CH₂); 2.70 (m, 5H, 1H von CH, 2^{∗}CH₂); 2.85 (m, 1H, 1H von CH); 3.00 (m, 1H, 1H von CH); 4.25 (m, 1H, 1H von CHRing5); 5.45 (br, 1H, NH); 5.60 (m, 1H, CH); 7.15 - 7.30 (m, 4H, Ar-H); 8.85 (br, 1H, Pyr-6H); |
| **2.131b** | oelig; logp (HCOOH): 5.29; 1H-NMR (CDCl₃, 400 MHZ, δ in ppm): 1.60 (m, 2H, 2^{∗}1H von CH₂), 1.70 (m, 2H, 2^{∗}1H von CH₂),1.85 (m, 8H, 2^{∗}1H von CH₂, 3^{∗}CH₂); 2.50 (m, 2H, CH₂); 2.65 (m, 1H, 1H von CH₂); 2.70 (m, 2H, CH₂); 2.85 (m, 1H, 1H von CH); 3.00 (m, 1H, 1H von CH); 4.20 (m, 1H, 1H von CHRing5); 5.45 (br, 1H, NH); 5.60 (m, 1H, CH); 7.15 - 7.30 (m, 4H, Ar-H); 9.50 (br, 1H, Pyr-6H); |
| **2.134a** | oelig; logp (HCOOH): 5.44; 1H-NMR (CDCl₃, 400 MHZ, δ in ppm): 1.80 - 1.95 (m, 9H, 1H von CH₂,4^{∗}CH₂), 2.10 (m, 1H, 1H von CH₂), 2.70 (m, 6H, 3^{∗}CH₂); 3.95 (s, 3H, OCH₃); 5.45 (m, 1H, CH); 5.50 (br, 1H, NH); 7.10 - 7.20 (m, 3H, Ar-H); 7.35 (m, 3H, Ar-H) 8.85 (br, 1H, Pyr-6H); |
| **2.134b** | oelig; logp (HCOOH): 5.13; 1H-NMR (CDCl₃, 400 MHZ, δ in ppm): 1.80 - 1.95 (m, 9H, 1H von CH₂, 4^{∗}CH₂), 2.10 (m, 1H, 1H von CH₂), 2.50 (s, 2H, CH₂); 2.70 (m, 6H, 2^{∗}CH₂); 3.95 (s, 3H, OCH₃); 5.45 (m, 1H, CH); 5.50 (br, 1H, NH); 7.10 - 7.20 (m, 3H, Ar-H); 7.35 (m, 3H, Ar-H) 9.50 (br, 1H, Pyr-6H); |
| **2.135a** | oelig; logp (HCOOH): 6.16; 1H-NMR (CDCl₃, 400 MHZ, δ in ppm): 0.95 (d, 6 H, 2^{∗}CH₃); 1.80 - 1.95 (m, 5H, 1H von CH₂,2^{∗}CH₂), 2.05 (m, 2H, 1H von CH₂, 1H von CH), 2.65 (s, 2H, CH₂); 2.70 (m, 2H, CH₂); 2.80 (m, 2H, 2^{∗}1H von CH₂); 3.95 (d, 2H, OCH₂); 5.45 (m, 1H, CH); 5.50 (br, 1H, NH); 7.10 - 7.20 (m, 3H, Ar-H); 7.35 (m, 3H, Ar-H) 8.80 (br, 1H, Pyr-6H); |
| **2.137a** | oelig; logp (HCOOH): 5.84; 1H-NMR (CDCl₃, 400 MHZ, δ in ppm): 0.95 (d, 6 H, 2^{∗}CH₃); 1.80 - 1.95 (m, 3H, 1H von CH₂, CH₂), 2.05 (m, 1H, 1H von CH₂), 2.70 (m, 5H, 2^{∗}1H von CH₂, 1H von CH-ipr, CH₂); 3.95 (d, 2H, OCH₂); 5.45 (m, 1H, CH); 5.50 (br, 1H, NH); 7.10 - 7.20 (m, 3H, Ar-H); 7.35 (m, 3H, Ar-H) 8.80 (br, 1H, Pyr-6H); |
| **2.137b** | oelig; logp (HCOOH): 5.34; 1H-NMR (CDCl₃, 400 MHZ, δ in ppm): 0.95 (d, 6 H, 2^{∗}CH₃); 1.80 - 1.95 (m, 3H, 1H von CH₂, CH₂), 2.05 (m, 1H, 1H von CH₂), 2.50 (m, 2H, CH₂); 2.70 (m, 4H, 3^{∗}1H von CH₂, 1H von CH-ipr); 3.95 (d, 2H, OCH₂); 5.45 (m, 1H, CH); 5.50 (br, 1H, NH); 7.10 - 7.20 (m, 3H, Ar-H); 7.35 (m, 3H, Ar-H) 9.50 (br, 1H, Pyr-6H); |

### B. Formulierungsbeispiele

Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277° C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I) und/oder deren Salze,
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Versuchsbeschreibung

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

In den nachfolgenden Tabellen werden folgende Abkürzungen verwendet:

**Unerwünschte Pflanzen / Weeds:**

| | | | |
|---|---|---|---|
| ABUTH: | *Abutilon theophrasti* | ALOMY: | *Alopecurus myosuroides* |
| AMARE: | *Amaranthus retroflexus* | AVEFA: | *Avena fatua* |
| CYPES: | *Cyperus esculentus* | ECHCG: | *Echinochloa crus-galli* |
| LOLMU: | *Lolium multiflorum* | MATIN: | *Matricaria inodora* |
| PHBPU: | *Ipomoea purpurea* | POLCO: | *Polygonum convolvulus* |
| SETVI: | *Setaria viridis* | STEME: | *Stellaria media* |
| VERPE: | *Veronica persica* | VIOTR: | *Viola tricolor* |

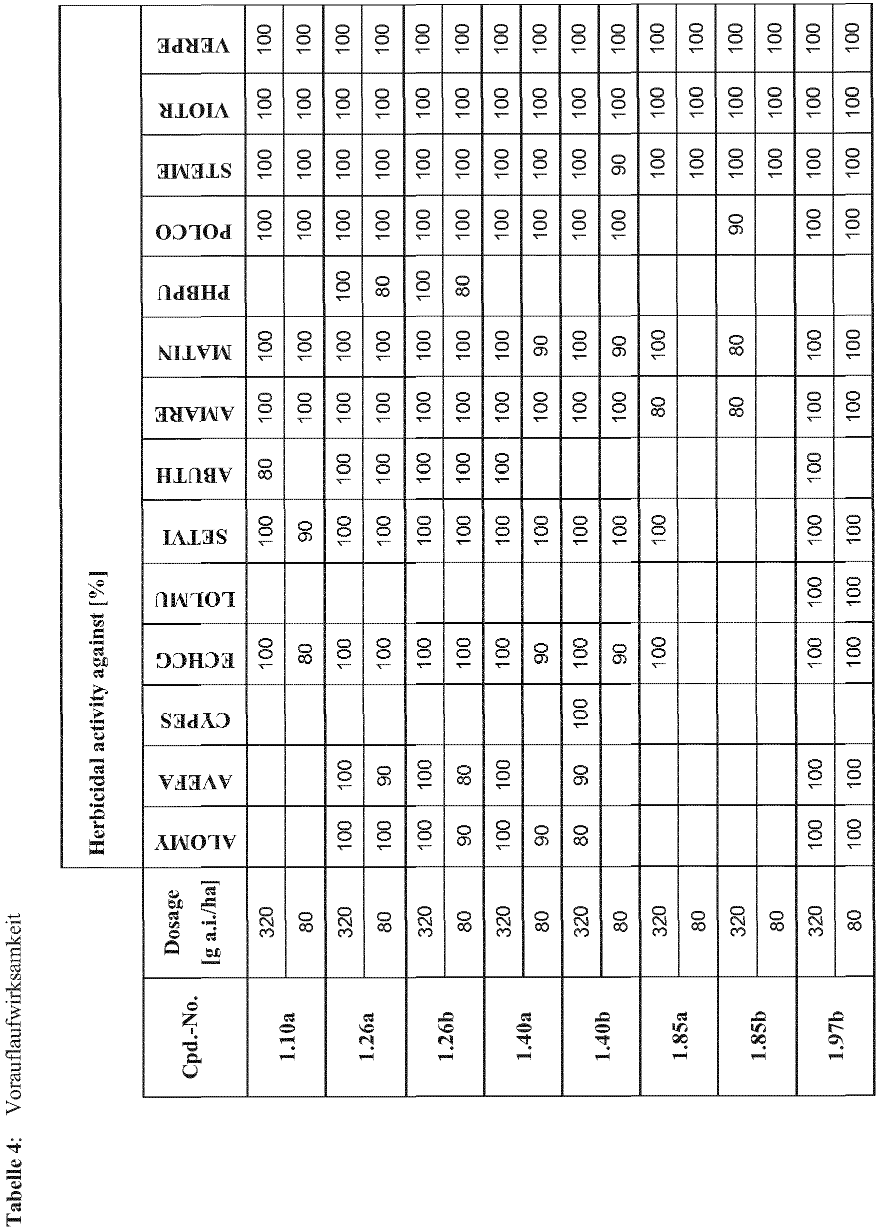

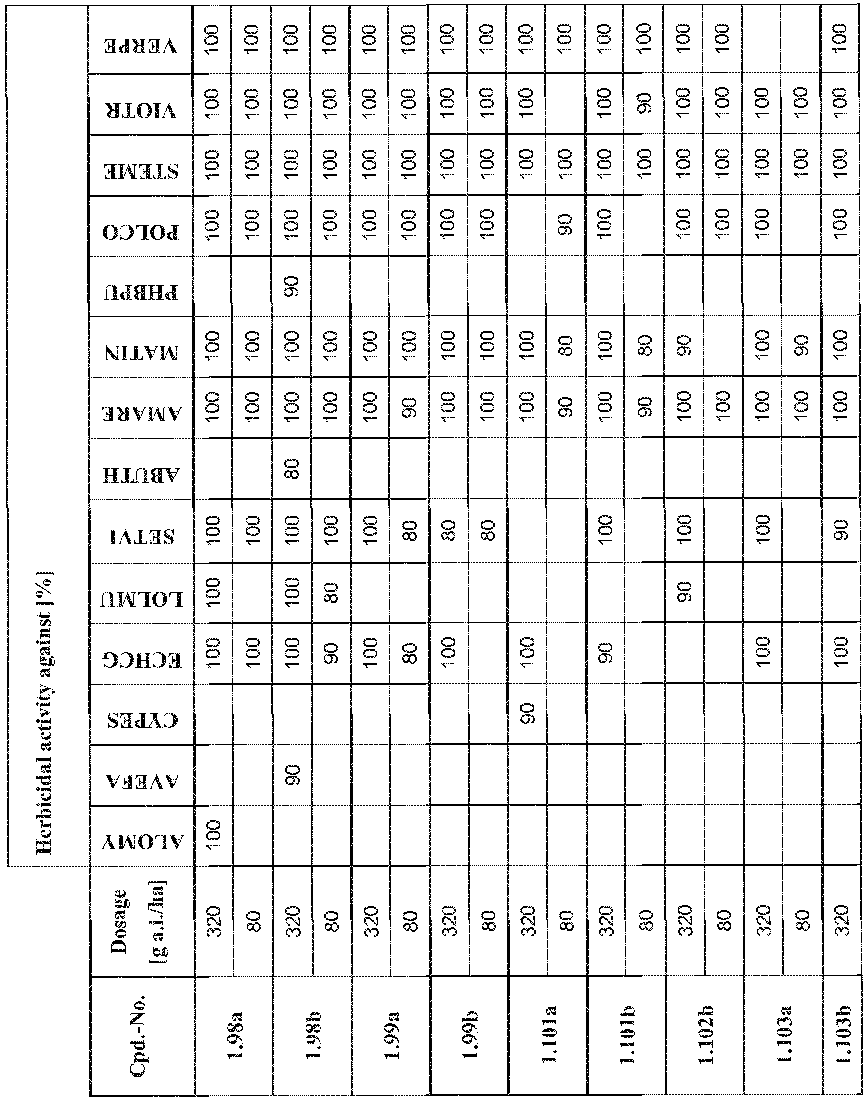

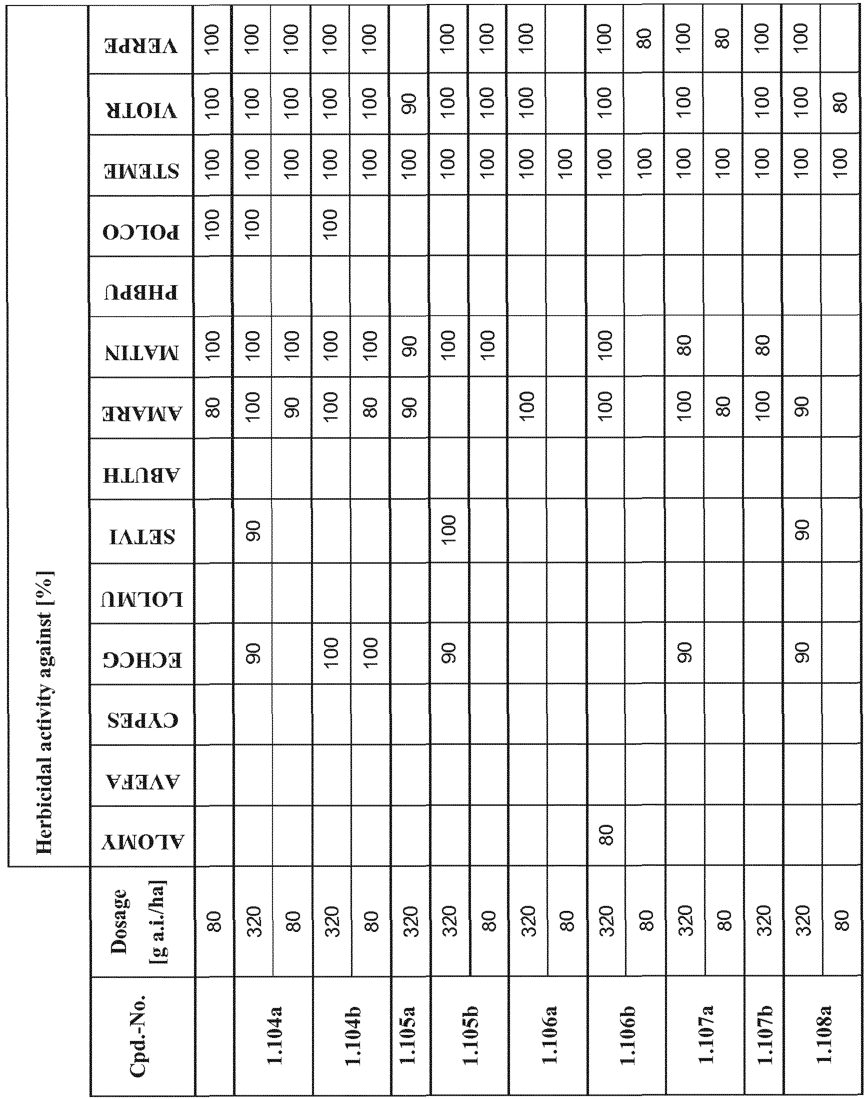

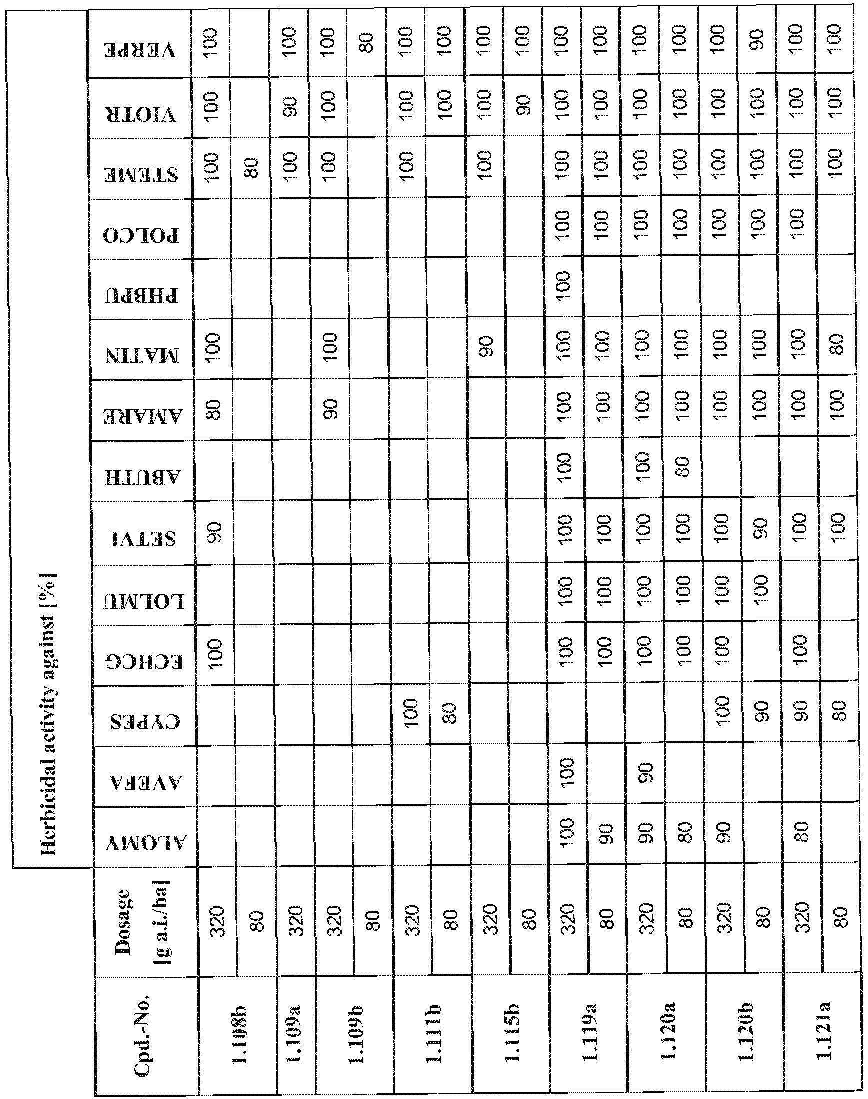

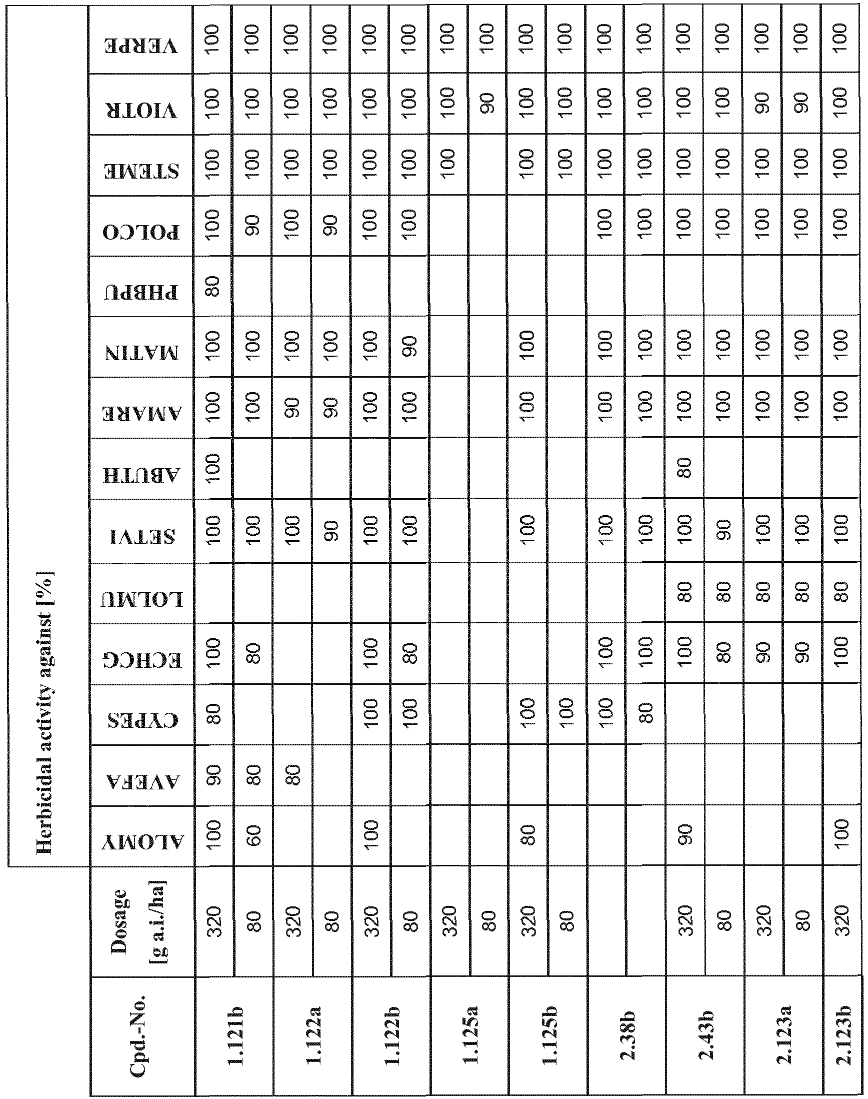

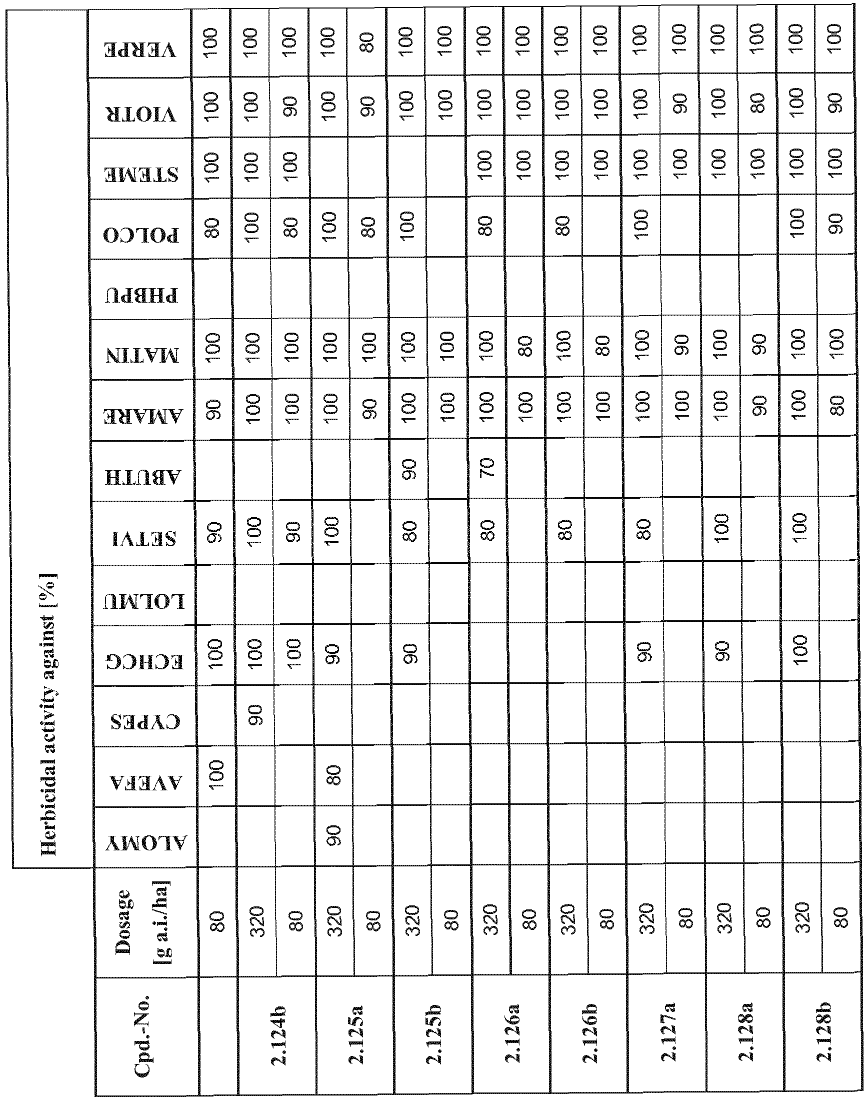

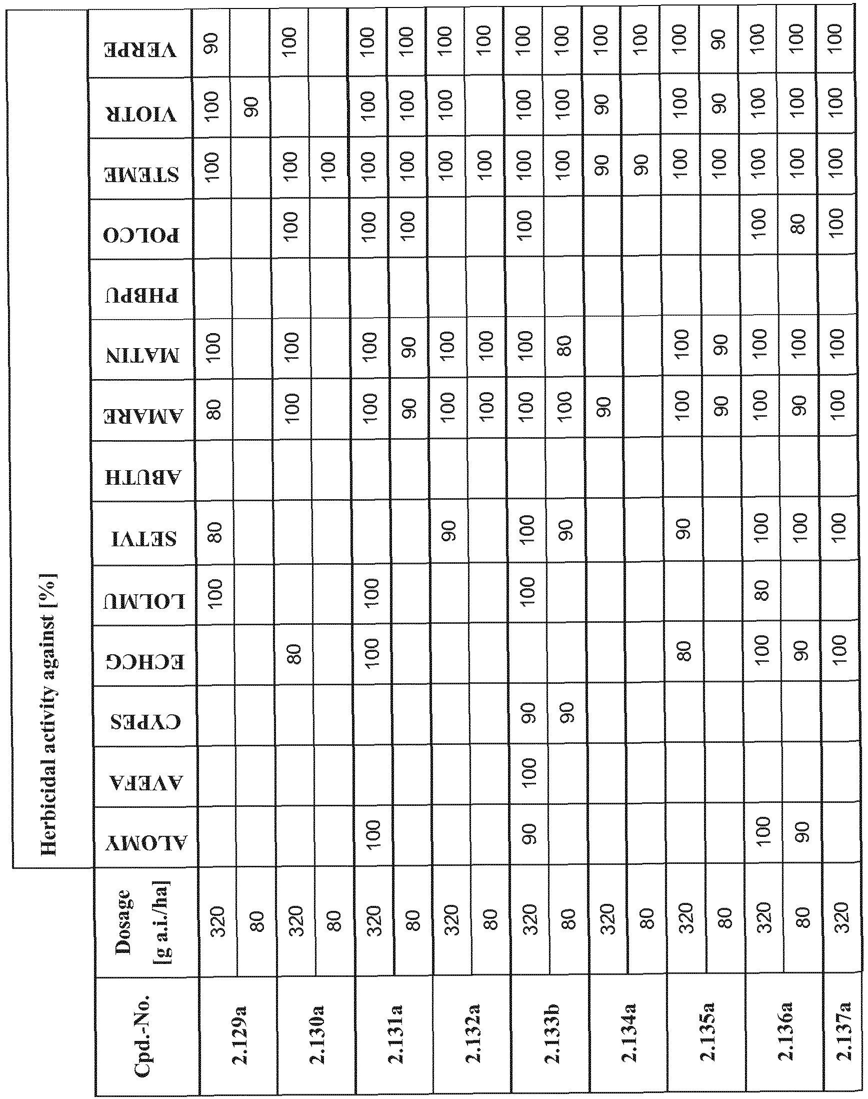

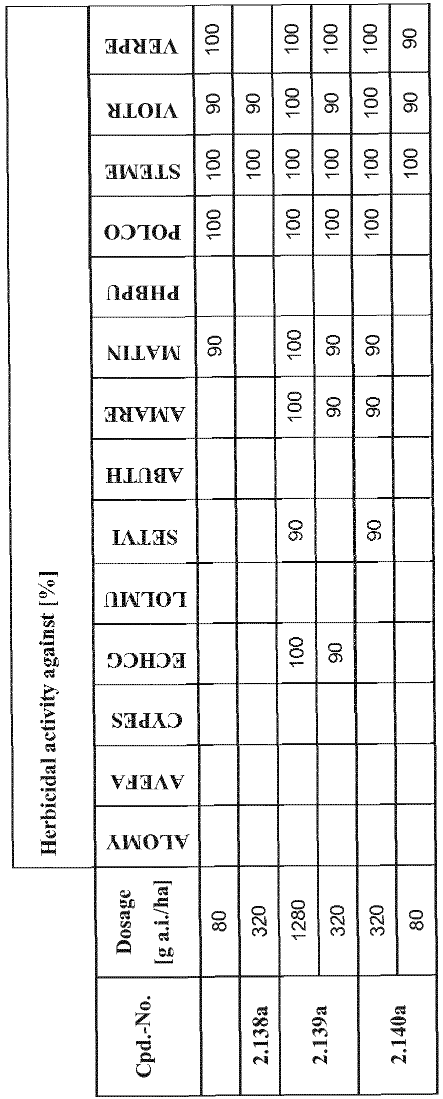

Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Verbindungen aus Tabelle 1 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Vorauflaufverfahren bei einer Aufwandmenge von 0,32 kg und weniger Aktivsubstanz pro Hektar. Die erfindungsgemäßen Verbindungen eignen sich deshalb im Vorauflaufverfahren zur Bekämpfung von unerwünschtem Pflanzenwuchs.

### 2. Herbizide Wirkung bzw. Kulturpflanzenverträglichkeit im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wässrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

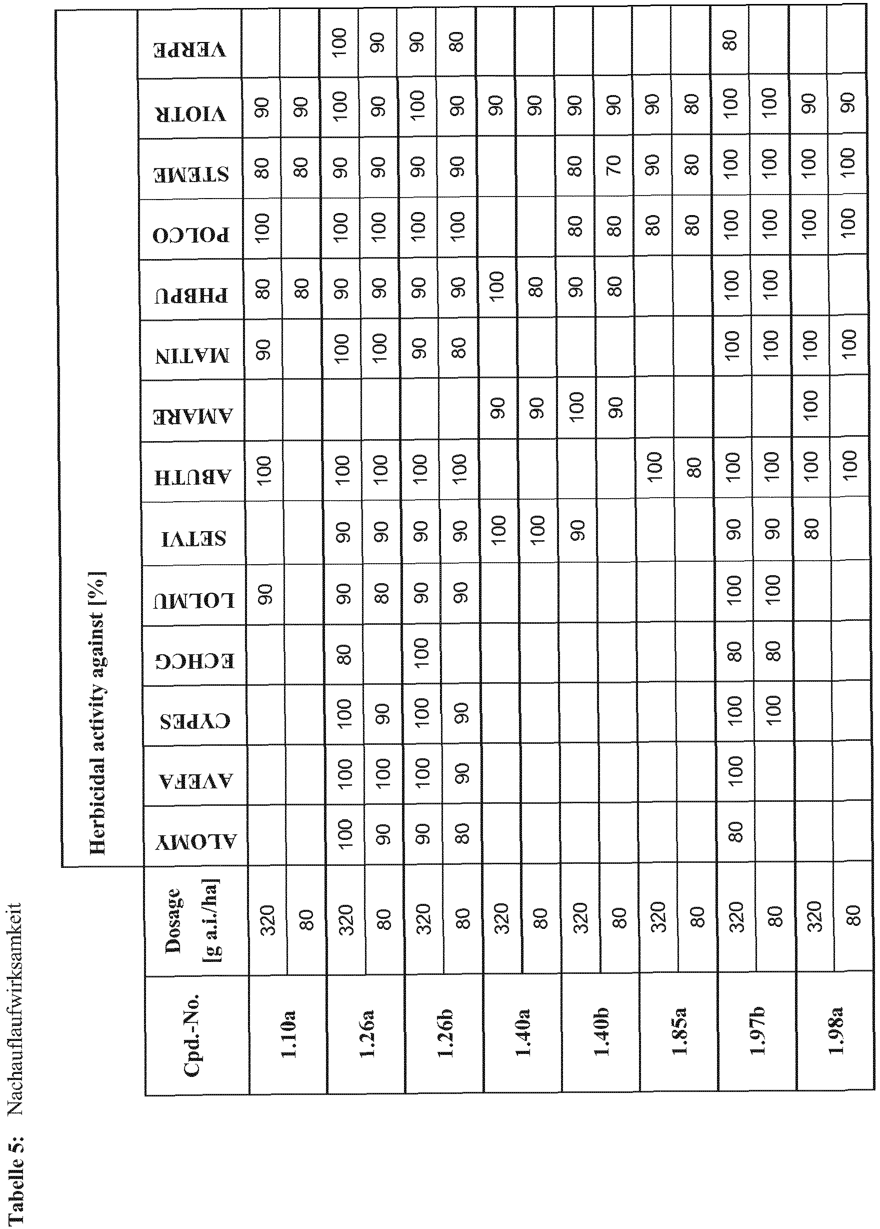

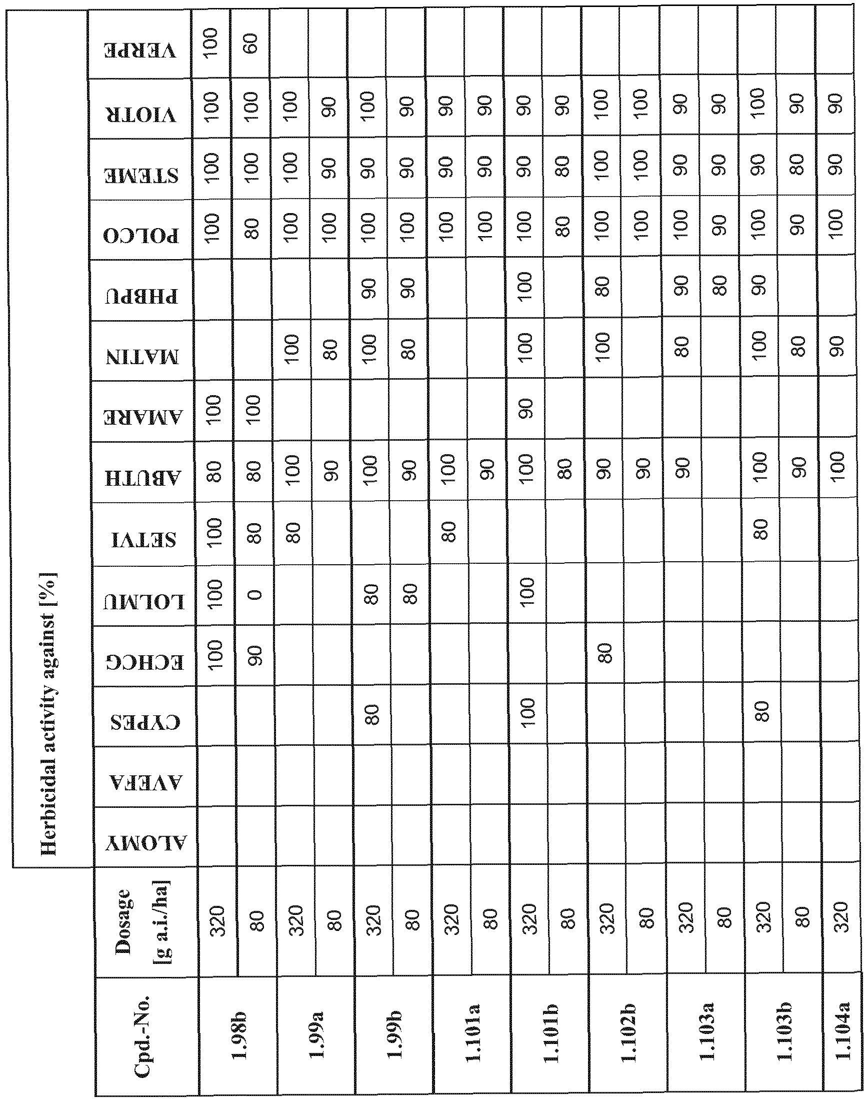

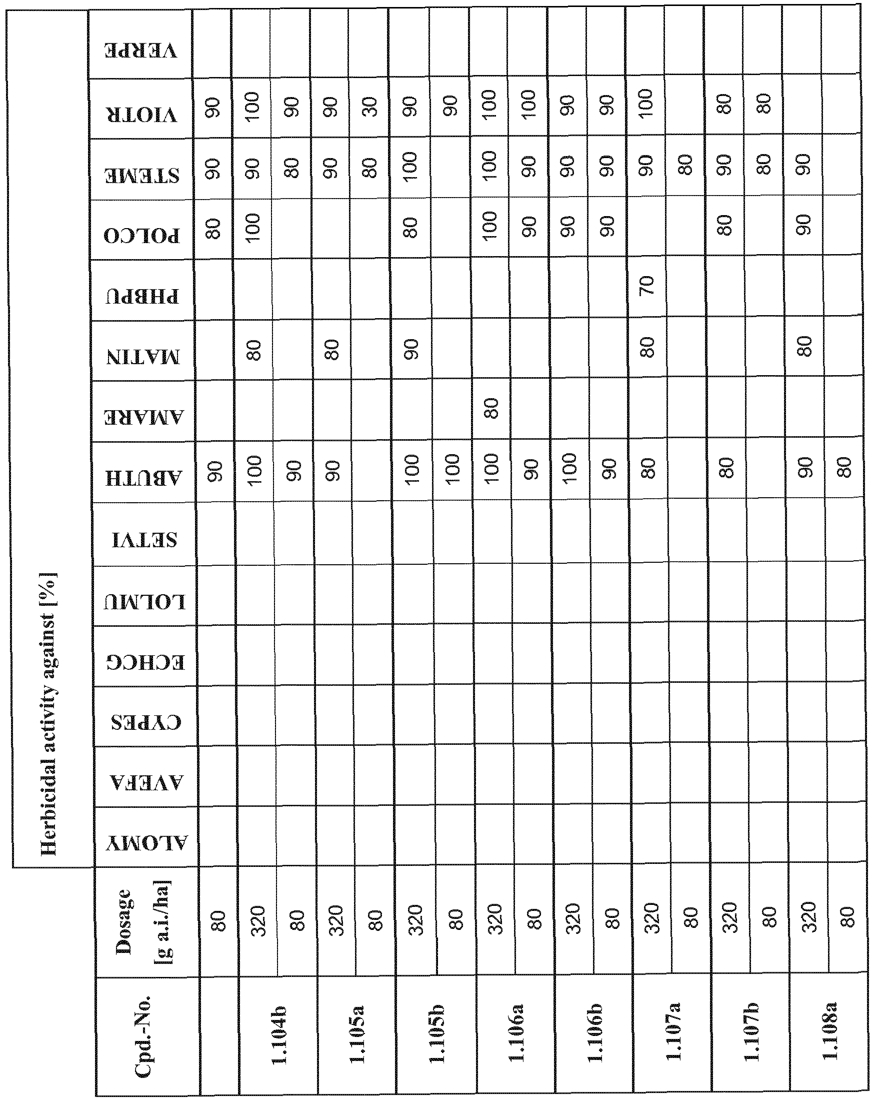

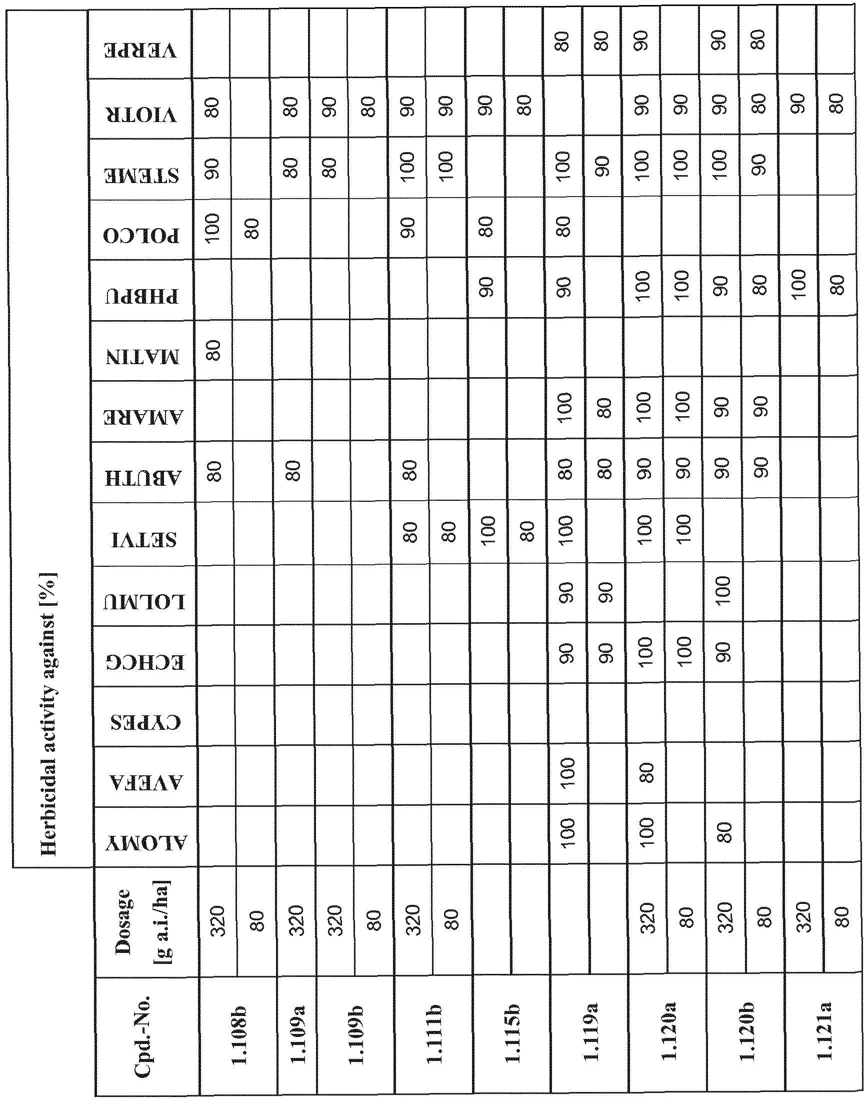

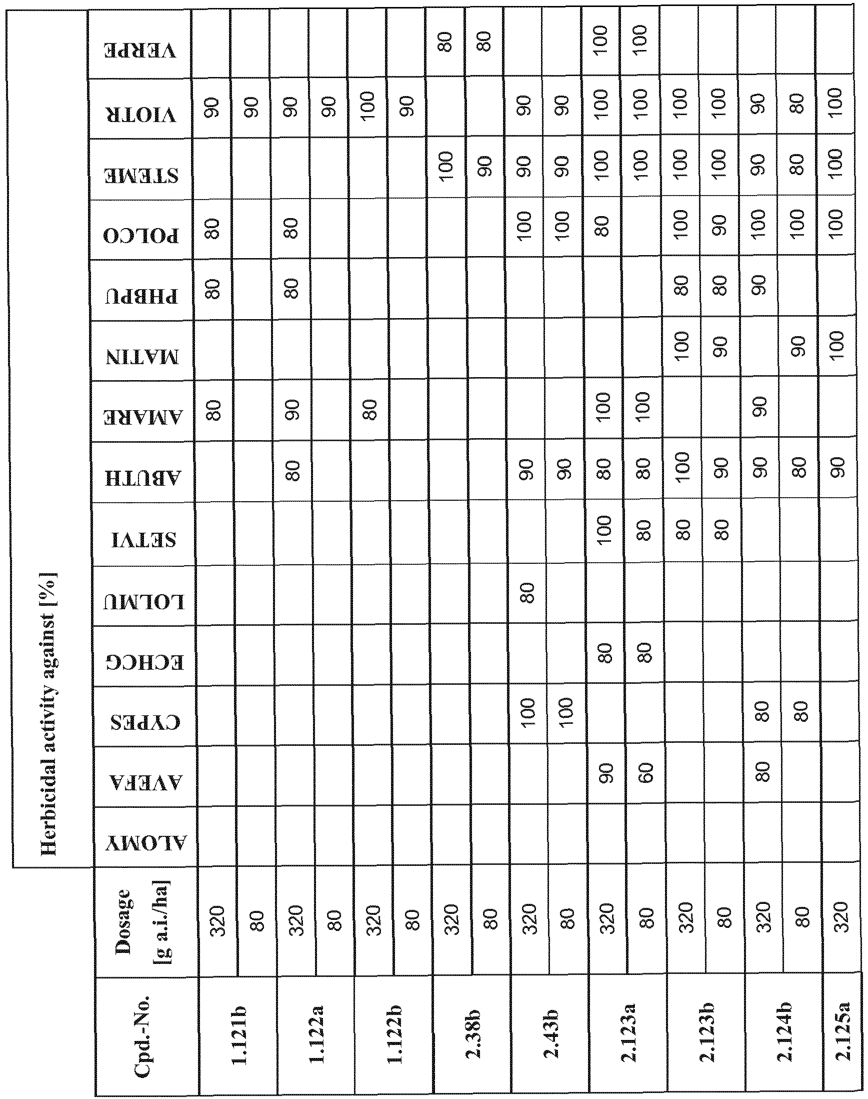

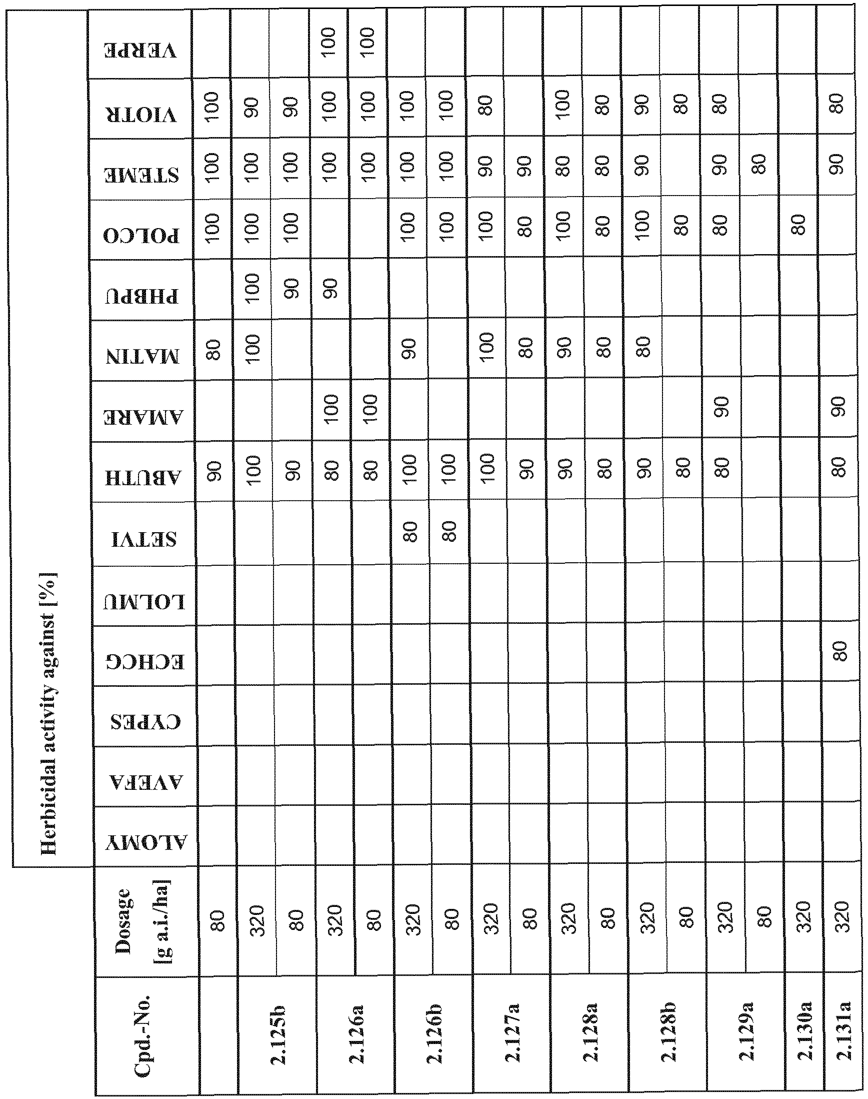

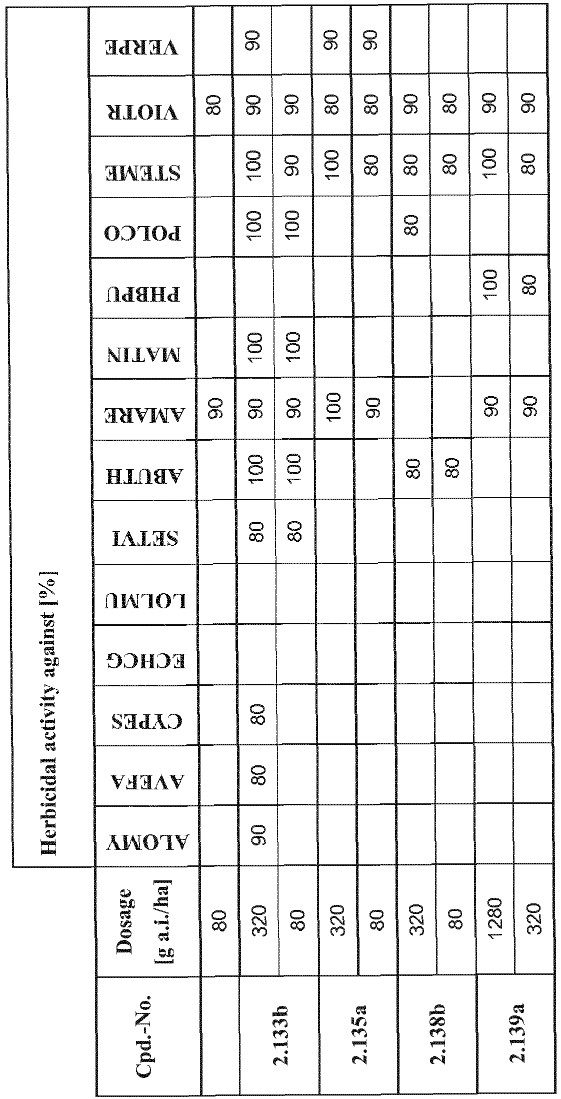

Wie die Ergebnisse zeigen, weisen erfindungsgemäße Verbindungen eine gute herbizide Nachauflaufwirksamkeit gegen ein breites Spektrum von Ungräsem und Unkräutern auf. Beispielsweise haben die Verbindungen aus Tabelle 2 sehr gute herbizide Wirkung gegen Schadpflanzen wie Avena fatua, Stellaria media, Echinochloa crus-galli, Lolium multiflorum, Setaria viridis, Abutilon theophrasti, Amaranthus retroflexus und Alopecurus myosuroides im Nachauflaufverfahren bei einer Aufwandmenge von 0,32 kg und weniger Aktivsubstanz pro Hektar. Die erfindungsgemäßen Verbindungen eignen sich deshalb im Nachauflaufverfahren zur Bekämpfung von unerwünschtem Pflanzenwuchs.

## Patentansprüche

1. Verbindungen der allgemeinen Formel (I) 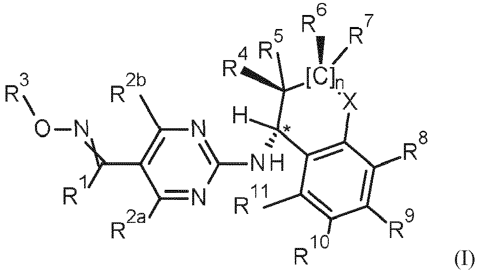 und deren agrochemisch verträglichen Salze, in welchen
R¹ ausgewählt ist aus der Gruppe, bestehend aus
- (C₁-C₆)-Alkyl, (C₁-C₆)-Halogenalkyl, (C₁-C₆)-Alkylcarbonyl-(C₁-C₄)-alkyl;
- (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-hatogenalkyl;
- (C₂-C₆)-Alkenyl, (C₂-C₆)-Halogenalkenyl;
- (C₂-C₆)-Alkinyl, (C₂-C₆)-Halogenalkinyl; Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl;
- Aminocarbonyl-(C₁-C₆)-alkyl;
- (C₁-C₆)-Alkoxy-(C₁-C₆)-alkyl;
- (C₃-C₈)-Cycloalkyl, welches unsubstituiert oder einfach oder mehrfach am Cycloalkylrest durch (C₁-C₆)-Alkyl und/oder Halogen substituiert ist; (C₃-C₈)-Cycloalkyl-(C₁-C₆)-alkyl, (C₃-C₈)-Cycloalkyl-(C₁-C₆)-haloalkyl;
- (C₃-C₈)-Cycloalkenyl, (C₃-C₈)-Cycloalkenyl-(C₁-C₆)-alkyl, (C₃-C₈)-Cycloalkenyl-(C₁-C₆)-halogenalkyl;
- Hydroxy-(C₁-C₆)-alkyl, Cyano-(C₁-C₆)-alkyl;
- (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl;
R^{2a} und R^{2b}, jeweils unabhängig voneinander, ausgewählt sind aus der Gruppe, bestehend aus
- Wasserstoff, Halogen, Hydroxy, Cyano, C(O)OH, C(O)NH₂;
- (C₁-C₆)-Alkyl, (C₁-C₆)-Halogenalkyl, (C₁-C₆)-Alkylcarbonyl, (C₁-C₆)-Haloalkylcarbonyl, (C₁-C₆)-Alkylcarbonyloxy, (C₁-C₆)-Halogenalkylcarbonyloxy, (C₁-C₆)-Alkylcarbonyl-(C₁-C₄)-alkyl;
- (C₁-C₆)-Alkoxy, (C₁-C₆)-Halogenalkoxy, (C₁-C₆)-Alkoxycarbonyl, (C₁-C₆)-Haloalkoxycarbonyl, (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₂-C₆)-Alkenyl, (C₂-C₆)-Halogenalkenyl, (C₂-C₆)-Alkenylcarbonyl, (C₂-C₆)-Haloalkenylcarbonyl, (C₂-C₆)-Alkenyloxy, (C₂-C₆)-Haloalkenyloxy, (C₂-C₆)-Alkenyloxycarbonyl, (C₂-C₆)-Haloalkenyloxycarbonyl;
- (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl; Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₆-C₁₄)-Aryl, (C₆-C₁₄)-Aryloxy, (C₆-C₁₄)-Arylcarbonyl und (C₆-C₁₄)-Aryloxycarbonyl, welche jeweils am Arylteil mit Halogen, (C₁-C₆)-Alkyl und/oder (C₁-C₆)-Haloalkyl substituiert sein können;
- (C₆-C₁₄)-Aryl-(C₁-C₆)-alkyl, (C₆-C₁₄)-Aryl-(C₁-C₆)-alkoxy, (C₆-C₁₄)-Aryl-(C₁-C₆)-alkyl-carbonyl, (C₆-C₁₄)-Aryl-(C₁-C₆)-alkyl-carbonyloxy, (C₆-C₁₄)-Aryl-(C₁-C₆)-alkoxycarbonyl, (C₆-C₁₄)-Aryl-(C₁-C₆)-alkoxycarbonyloxy;
- Aminocarbonyl-(C₁-C₆)-alkyl, Di-(C₁-C₆)-Alkylaminocarbonyl-(C₁-C₆)-alkyl;
- N-((C₁-C₆)-Haloalkanoyl)-amino-carbonyl, Mono-((C₆-C₁₄)-aryl)-amino-carbonyl, Di-((C₆-C₁₄)-aryl)-amino-carbonyl;
- (C₁-C₆)-Alkoxy-(C₁-C₆)-alkyl, (C₁-C₆)-Alkoxy-(C₁-C₆)-alkoxy, (C₁-C₆)-Alkoxycarbonyl-(C₁-C₆)-alkoxy;
- (C₃-C₈)-Cycloalkyl, welches unsubstituiert oder einfach oder mehrfach am Cycloalkylrest durch (C₁-C₆)-Alkyl und/oder Halogen substituiert ist; (C₃-C₈)-Cycloalkoxy, (C₃-C₈)-Cycloalkyl-(C₁-C₆)-alkyl, (C₃-C₈)-Cycloalkyl-(C₁-C₆)-haloalkyl, (C₃-C₈)-Cycloalkyl-(C₁-C₆)-alkoxy, (C₃-C₈)-Cycloalkyl-(C₁-C₆)-haloalkoxy, (C₃-C₈)-Cycloalkylcarbonyl, (C₃-C₈)-Cycloalkoxycarbonyl, (C₃-C₈)-Cycloalkyl-(C₁-C₆)-alkylcarbonyl, (C₃-C₈)-Cycloalkyl-(C₁-C₆)-haloalkylcarbonyl, (C₃-C₈)-Cycloalkyl-(C₁-C₆)-alkoxycarbonyl, (C₃-C₈)-Cycloalkyl-(C₁-C₆)-haloalkoxycarbonyl, (C₃-C₈)-Cycloalkylcarbonyloxy, (C₃-C₈)-Cycloalkoxycarbonyloxy, (C₃-C₈)-Cycloalkyl-(C₁-C₆)-alkylcarbonyloxy, (C₃-C₈)-Cycloalkyl-(C₁-C₆)-halogenalkylcarbonyloxy, (C₃-C₈)-Cycloalkyl-(C₁-C₆)-alkoxycarbonyloxy, (C₃-C₈)-Cycloalkyl-(C₁-C₆)-haloalkoxycarbonyloxy;
- (C₃-C₈)-Cycloalkenyl, (C₃-C₈)-Cycloalkenyloxy, (C₃-C₈)-Cycloalkenyl-(C₁-C₆)-alkyl, (C₃-C₈)-Cycloalkenyl-(C₁-C₆)-halogenalkyl, (C₃-C₈)-Cycloalkenyl-(C₁-C₆)-alkoxy, (C₃-C₈)-Cycloalkenyl-(C₁-C₆)-halogenalkoxy, (C₃-C₈)-Cycloalkenylcarbonyl, (C₃-C₈)-Cycloakenyloxycarbonyl, (C₃-C₈)-Cycloalkenyl-(C₁-C₆)-alkylcarbonyl, (C₃-C₈)-Cycloalkenyl-(C₁-C₆)-halogenalkylcarbonyl, (C₃-C₈)-Cycloalkenyl-(C₁-C₆)-alkoxycarbonyl, (C₃-C₈)-Cycloalkenyl-(C₁-C₆)-haloalkoxycarbonyl, (C₃-C₈)-Cycloalkenylcarbonyloxy, (C₃-C₈)-Cycloalkenyloxycarbonyloxy, (C₃-C₈)-Cycloalkenyl-(C₁-C₆)-alkylcarbonyloxy, (C₃-C₈)-Cycloalkenyl-(C₁-C₆)-halogenalkylcarbonyloxy, (C₃-C₈)-Cycloalkenyl-(C₁-C₆)-alkoxycarbonyloxy, (C₃-C₈)-Cycloalkenyl-(C₁-C₆)-haloalkoxycarbonyloxy;
- Hydroxy-(C₁-C₆)-alkyl, Hydroxy-(C₁-C₆)-alkoxy Cyano-(C₁-C₆)-alkoxy, Cyano-(C₁-C₆)-alkyl;
- (C₁-C₆)-Alkylsulfonyl, (C₁-C₆)-Alkylthio, (C₁-C₆)-Alkylsulfinyl, (C₁-C₆)-Haloalkylsulfonyl, (C₁-C₆)-Halogenalkylthio, (C₁-C₆)-Halogenalkylsulfinyl, (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl, (C₁-C₆)-Alkylsulfonyloxy, (C₁-C₆)-Halogenalkylsulfonyloxy, (C₁-C₆)-Alkylthiocarbonyl, (C₁-C₆)-Haloalkylthiocarbonyl, (C₁-C₆)-Alkylthiocarbonyloxy, (C₁-C₆)-Haloalkylthiocarbonyloxy, (C₁-C₆)-Alkylthio-(C₁-C₆)-alkyl, (C₁-C₆)-Alkylthio-(C₁-C₆)-alkoxy, (C₁-C₆)-Alkylthio-(C₁-C₆)-alkylcarbonyl, (C₁-C₆)-_{A}lkylthio-(C₁-C₆)-alkylcarbonyloxy; (C₄-C₁₄)-Arylsulfonyl, (C₆-C₁₄)-Arylthio, (C₆-C₁₄)-Arylsulfinyl, (C₃-C₈)-Cycloalkylthio, (C₃-C₈)-Alkenylthio, (C₃-C₈)-Cycloalkenylthio und (C₃-C₆)-Alkinylthio;
R¹ mit R^{2a} über eine Bindung miteinander verbunden sind, so dass zusammen mit den Kohlenstoffen 4 und 5 des Pyrimidins und der Carbonylgruppe in der 5-Positon des Pyrimidins sich ein 5 bis 7 gliedriger teilhydrierter Carbo- oder Heterocyclus ergibt, worin die Heteroatome ausgewählt sind aus der Gruppe bestehend aus Sauerstoff oder Schwefel, wobei anstatt des Schwefels eine Sulfoxidgruppe oder eine Sulfonylgruppe vorliegen kann, und der Carbo- oder Heterocyclus unsubstituiert oder substituiert ist mit einem oder mehreren Substituenten ausgewählt aus der Gruppe bestehend aus (C₁-C₆)-Alkyl, (C₁-C₆)-Haloalkyl, (C₃-C₆)-Cycloalkyl, spiro-(C₂-C₅)-Cycloalkyl und (C₆-C₁₄)-Aryl, welches mit einem oder mehreren Substituenten ausgewählt aus Fluor, Chlor, Brom oder Iod substituiert sein kann;
R³ ausgewählt ist aus der Gruppe, bestehend aus
- Wasserstoff;
(C₁-C₆)-Alkyl, (C₁-C₆)-Halogenalkyl, (C₁-C₆)-Alkylcarbonyl-(C₁-C₄)-alkyl;
- (C₂-C₆)-Alkenyl, (C₂-C₆)-Halogenalkenyl;
- (C₂-C₆)-Alkinyl, (C₂-C₆)-Halogenalkinyl; Tri-(C₁-C₆)-alkylsilyl-(C₂-C₆)-alkinyl, Di-(C₁-C₆)-alkylsilyl-(C₂-C₆)-alkinyl, Mono-(C₁-C₆)-alkylsilyl-(C₂-C₆)-alkinyl; Phenylsilyl-(C₂-C₆)-alkinyl;
- (C₁-C₆)-Alkoxycarbonyl-(C₁-C₆)-alkyl, (C₁-C₆)-Haloalkoxycarbonyl-(C₁-C₆)-alkyl, (C₁-C₆)-Alkoxycarbonyl-(C₁-C₆)-halogenalkyl, (C₁-C₆)-Halogenalkoxycarbonyl-(C₁-C₆)-halogenalkyl;
- (C₆-C₁₄)-Aryl, welche jeweils am Arylteil mit Halogen, (C₁-C₆)-Alkyl und/oder (C₁-C₆)-Haloalkyl substituiert sein können;
- (C₂-C₁₄)-Het-Aryl, welche jeweils am Het-Arylteil mit Halogen, (C₁-C₆)-Alkyl und/oder (C₁-C₆)-Haloalkyl substituiert sein können;
- (C₆-C₁₄)-Aryl-(C₁-C₆)-alkyl;
- Aminocarbonyl-(C₁-C₆)-alkyl;
- (C₁-C₆)-Alkoxy, (C₁-C₆)-Alkoxy-(C₁-C₆)-alkyl;
- (C₃-C₈)-Cycloalkyl, welches unsubstituiert oder einfach oder mehrfach am Cycloalkylrest durch (C₁-C₆)-Alkyl und/oder Halogen substituiert sein kann; (C₃-C₈)-Cycloalkyl-(C₁-C₆)-alkyl, (C₃-C₈)-Cycloalkyl-(C₁-C₆)-haloalkyl;
- (C₃-C₈)-Cycloalkenyl-(C₁-C₆)-alkyl, (C₃-C₈)-Cycloalkenyl-(C₁-C₆)-halogenalkyl, Hydroxy-(C₁-C₆)-alkyl, Cyano-(C₁-C₆)-alkyl;
- (C₁-C₆)-Alkylsulfonyl-(C₁-C₆)-alkyl, (C₁-C₆)-Alkylthio-(C₁-C₆)-alkyl, (C₁-C₆)-Alkylsulfinyl-(C₁-C₆)-alkyl, (C₁-C₆)-Haloalkylsulfonyl-(C₁-C₆)-alkyl, (C₁-C₆)-Haloalkylthio-(C₁-C₆)-alkyl, (C₁-C₆)-Haloalkylsulfinyl-(C₁-C₆)-alkyl, (C₁-C₆)-Alkylsulfonyl-(C₁-C₆)-haloalkyl, (C₁-C₆)-Alkylthio-(C₁-C₆)-haloalkyl, (C₁-C₆)-Alkylsulfinyl-(C₁-C₆)-haloalkyl, (C₁-C₆)-Haloalkylsulfonyl-(C₁-C₆)-haloalkyl, (C₁-C₆)-Haloalkylthio-(C₁-C₆)-haloalkyl, (C₁-C₆)-Haloalkylsulfinyl-(C₁-C₆)-haloalkyl;
R⁴ und R⁵ jeweils unabhängig voneinander, ausgewählt sind aus der Gruppe, bestehend aus Wasserstoff, (C₁-C₆)-Alkyl, (C₁-C₆)-Haloalkyl, Hydroxy, (C₁-C₆)-Alkoxy und (C₁-C₆)-Halogenalkoxy; oder die Reste R⁴ und R⁵ zusammen mit dem Kohlenstoffatom, an welchem sie gebunden sind, einen drei- bis siebengliedrigen Ring bilden;
R⁶ und R⁷ jeweils unabhängig voneinander, ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Halogenalkoxy, (C₆-C₁₄)-Aryl, (C₆-C₁₄)-Aryloxy, (C₆-C₁₄)-Arylcarbonyl und (C₆-C₁₄)-Aryloxycarbonyl; oder die Reste R⁶ und R⁷ zusammen eine (C₁-C₇)-Alkylengruppe bilden, die ein oder mehrere Sauerstoff- und/oder Schwefelatome enthalten können, wobei die (C₁-C₇)-Alkylengruppe durch Halogen einfach oder mehrfach substituiert sein kann und die jeweilgen Halogensubstituenten gleich oder verschieden sein können;
n die Laufzahl 0, 1 oder 2 ist;
R⁸, R⁹, R¹⁰ und R¹¹ jeweils unabhängig voneinander ausgewählt sind aus der Gruppe, bestehend aus Wasserstoff, Halogen, Cyano, C(O)OH, C(O)NH₂, (C₁-C₆)-Alkyl, (C₁-C₆)-Alkylcarbonyl, (C₁-C₆)-Alkyloxycarbonyl, (C₁-C₆)-Alkylaminocarbonyl, (C₁-C₆)-di-Alkylaminocarbonyl, (C₁-C₆)-Haloalkyl, (C₁-C₆)-Alkoxy, (C₁-C₆)-Halogenalkoxy, (C₂-C₆)-Alkenyl, (C₂-C₆)-Alkinyl, (C₂-C₆)-Halogenalkinyl, (C₂-C₆)-Alkinylcarbonyl, (C₂-C₆)-Haloalkinylcarbonyl, (C₂-C₆)-Alkinyloxy, (C₂-C₆)-Haloalkinyloxy, (C₂-C₆)-Alkinyloxycarbonyl, (C₂-C₆)-Halogenalkinyloxycarbonyl und Nitro, wobei die Reste R⁹ und R¹⁰ ringbildend durch eine -O-CH₂-O- Gruppe verbunden sein können;
X für eine chemische Bindung, CH₂, O, S, Carbonyl, NH, CR¹²R¹³,NR¹⁴,CH₂O, oder CH₂S steht, wobei bei den beiden letztgenannten Gruppen das Kohlenstoffatom an den aromatischen Teil und das Heteroatom O oder S an den teilhydrierten Teil des Amins gebunden ist,;
R¹² und R¹³ jeweils unabhängig voneinander, ausgewählt sind aus der Gruppe, bestehend aus Wasserstoff, (C₁-C₆)-Alkyl und (C₁-C₆)-Haloalkyl; und
R¹⁴ ausgewählt ist aus der Gruppe, bestehend aus Wasserstoff, (C₁-C₆)-Alkyl und (C₁-C₆)-Haloalkyl.

2. Verbindungen der allgemeinen Formel (I) nach Anspruch 1, **dadurch gekennzeichnet, dass** R¹ ausgewählt ist aus der Gruppe, bestehend aus (C₁-C₆)-Alkyl, (C₁-C₆)-Haloalkyl, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist.

3. Verbindungen der allgemeinen Formel (I) nach Anspruch 1 oder 2, **dadurch gekennzeichnet, dass** R^{2a} und R^{2b} jeweils unabhängig voneinander ausgewählt sind aus der Gruppe, bestehend aus Wasserstoff, (C₁-C₆)-Alkyl, (C₁-C₆)-Haloalkyl, (C₁-C₆)-Alkoxy, (C₁-C₆)-Alkoxy-(C₁-C₆)-alkyl, (C₃-C₈)-Cycloalkyl, (C₃-C₈)-Cycloalkyl-(C₁-C₆)-alkyl und (C₃-C₈)-Cycloalkyl-(C₁-C₆)-haloalkyl, wobei der Cycloalkylrest jeweils unsubstituiert ist oder durch (C₁-C₆)-Alkyl und/oder Halogen substituiert ist.

4. Verbindungen der allgemeinen Formel (I) nach einem der Ansprüche 1 bis 3, **dadurch gekennzeichnet, dass** R¹ mit R^{2a} über eine Bindung miteinander verbunden sind, so dass sich zusammen mit den Kohlenstoffen 4 und 5 des Pyrimidins und der Carbonylgruppe in der 5-Positon des Pyrimidins ein 5- bis 7-gliedriger teilhydrierter Carbo- oder Heterocyclus ergibt, worin die Heteroatome ausgewählt sind aus der Gruppe bestehend aus Sauerstoff oder Schwefel, wobei anstatt des Schwefels eine Sulfoxidgruppe oder eine Sulfonylgruppe vorliegen kann, und der Carbo- oder Heterocyclus unsubstituiert ist oder substituiert ist mit einem oder mehreren Substituenten ausgewählt aus der Gruppe bestehend aus (C₁-C₃)-Alkyl, (C₁-C₃)-Haloalkyl, (C₃-C₆)-Cycloalkyl, und Phenyl (C₆H₅), welches einfach oder mehrfach mit Fluor, Chlor, Brom oder Iod substituiert sein kann.

5. Verbindungen der allgemeinen Formel (I) nach einem der Ansprüche 1 bis 4, **dadurch gekennzeichnet, dass** der R³ ausgewählt ist aus der Gruppe bestehend Wasserstoff, (C₁-C₆)-Alkyl, (C₁-C₆)-Halogenalkyl, (C₁-C₆)-Alkoxy, (C₁-C₆)-Alkoxy-(C₁-C₆)-alkyl, (C₂-C₆)-Alkenyl, (C₂-C₆)-Halogenalkenyl, (C₂-C₆)-Alkinyl, und (C₂-C₆)-Halogenalkinyl und (C₆-C₁₄)-Aryl-(C₁-C₆)-alkyl.

6. Verbindungen der allgemeinen Formel (I) nach einem der Ansprüche 1 bis 5, **dadurch gekennzeichnet, dass** R⁴ und R⁵ jeweils unabhängig voneinander ausgewählt sind aus der Gruppe, bestehend aus Wasserstoff, Hydroxy, (C₁-C₆)-Alkyl, (C₁-C₆)-Alkylphenyl und (C₁-C₆)-Alkoxy.

7. Verbindungen der allgemeinen Formel (I) nach einem der Ansprüche 1 bis 6, **dadurch gekennzeichnet, dass** R⁶ und R⁷ unabhängig voneinander, ausgewählt sind aus der Gruppe, bestehend aus Wasserstoff, (C₁-C₆)-Alkyl und (C₆-C₁₄)-Aryl.

8. Verbindungen der allgemeinen Formel (I) nach einem der Ansprüche 1 bis 7, **dadurch gekennzeichnet, dass** R⁸ ausgewählt ist aus der Gruppe, bestehend aus aus Wasserstoff, Halogen, (C₁-C₆)-Alkyl, (C₁-C₆)Alkoxy, und (C₆-C₁₄)-Aryl.

9. Verbindungen der allgemeinen Formel (I) nach einem der Ansprüche 1 bis 8, **dadurch gekennzeichnet, dass** R⁹ ausgewählt ist aus der Gruppe, bestehend aus Wasserstoff, Halogen, (C₁-C₆)-Alkyl und (C₁-C₆)-Alkoxy.

10. Verbindungen der allgemeinen Formel (I) nach einem der Ansprüche 1 bis 9, **dadurch gekennzeichnet, dass** R¹⁰ ausgewählt ist aus der Gruppe, bestehend aus aus Wasserstoff, Halogen, Cyano, Aminocarbonyl, Hydroxycarbonyl, (C₁-C₆)-Alkyl, (C₁-C₆)-Haloalkyl, (C₁-C₆)-Alkoxy, (C₂-C₆)-Alkenyl, (C₂-C₆)-Alkinyl, (C₁-C₆)-Alkyl-(C₂-C₆)-alkinyl, Hydroxy-(C₁-C₆)-Alkyl-(C₂-C₆)-alkinyl, (C₁-C₆)-Alkoxy-(C₂-C₆)-Alkinyl und Aryl-(C₂-C₆)-Alkinyl.

11. Verbindungen der allgemeinen Formel (I) nach einem der Ansprüche 1 bis 10, **dadurch gekennzeichnet, dass** R¹¹ ausgewählt ist aus der Gruppe, bestehend aus Wasserstoff und (C₁-C₆)-Alkyl.

12. Verbindungen der allgemeinen Formel (I) nach einem der Ansprüche 1 bis 11, **dadurch gekennzeichnet, dass** X ausgewählt ist aus der Gruppe bestehend aus einer chemischen Bindung, CH₂, O, S, Carbonyl, NH, CH(C₁-C₆)Alkyl, N(C₁-C₆)Alkyl, OCH₂, und SCH₂, wobei bei den beiden letztgenannten Gruppen das Kohlenstoffatom an den aromatischen Teil und das Heteroatom O oder S an den teilhydrierten Teil des Amins gebunden ist.

13. Verbindungen der allgemeinen Formel (I) nach einem der Ansprüche 1 bis 12, **dadurch gekennzeichnet, dass** R⁹ und R¹⁰ ringbildend durch eine -O-CH₂-O- Gruppe verbunden sind.

14. Verbindungen der allgemeinen Formel (I) nach einem der Ansprüche 1 bis 13, **dadurch gekennzeichnet, dass** die Laufzahl n 0 oder 1 beträgt.

15. Verbindungen der allgemeinen Formel (I) 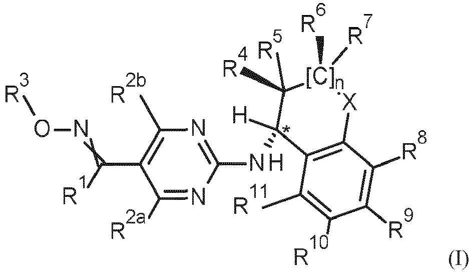 nach einem der Ansprüche 1 bis 14, **dadurch gekennzeichnet, dass** das chirale Kohlenstoffatom mit der Kennzeichnung (*) eine (R)-Konfiguration aufweist.

16. Verbindungen der allgemeinen Formel (I) 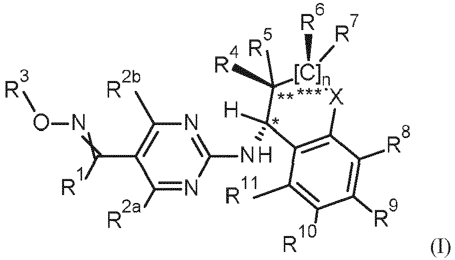 nach einem der Ansprüche 1 bis 15, **dadurch gekennzeichnet, dass** das chirale Kohlenstoffatom mit der Kennzeichnung (*) eine (R)-Konfiguration und das chirale Kohlenstoffatom mit der Kennzeichnung (**) eine (S)-Konfiguration aufweist.

17. Verfahren zur Herstellung von Verbindungen der allgemeinen Formel (I) und/oder deren agrochemisch verträgliche Salze und/oder deren agrochemisch verträgliche quaternierte Stickstoff-Derivate 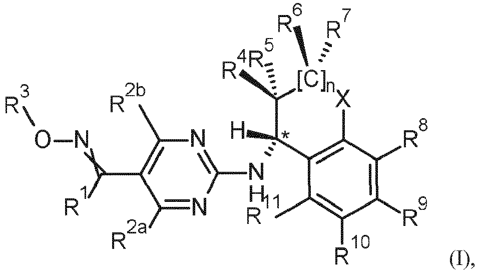 wobei die Reste R¹ bis R¹¹ sowie X und n gemäß einem der Ansprüche 1 bis 14 definiert sind, und wobei
eine Verbindung der allgemeinen Formel (II) 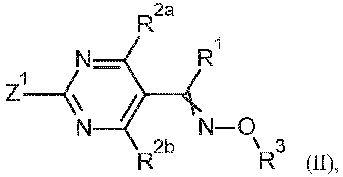 wobei R¹, R^{2a}, R^{2b} und R³ gemäß einem der Ansprüche 1 bis 14 definiert sind, und Z¹ für einen austauschfähigen Rest oder eine Abgangsgruppe steht, ausgewählt aus der Gruppe bestehend aus Fluor, Chlor, Brom, Jod, ein (C₁-C₄)-Alkylsulfanyl, (C₁-C₄)-Alkylsulfinyl, (C₁-C₄)-Alkylsulfonyl, ein unsubstituertes oder einfach oder mehrfach mit Fluor, Chlor, Brom oder (C₁-C₄)-Alkyl oder (C₁-C₄)-Alkoxy substituiertes Phenyl-(C₁-C₄)-alkylsulfonyl oder ein (C₁-C₄)-Alkylphenyl-sulfonyl,
umgesetzt wird mit einem Amin der allgemeinen Formel (III) oder mit einem Säureadditionssalz des Amins der allgemeinen Formel (III) 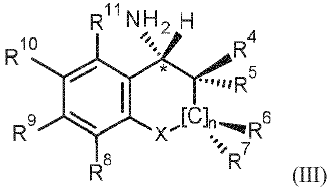 wobei die Reste R⁴ bis R¹¹ sowie X und n wie in einem der Ansprüche 1 bis 14 definiert sind.

18. Verfahren zur Herstellung von Verbindungen der allgemeinen Formel (I) und/oder deren agrochemisch verträgliche Salze und/oder deren agrochemisch verträgliche quaternierte Stickstoff-Derivate 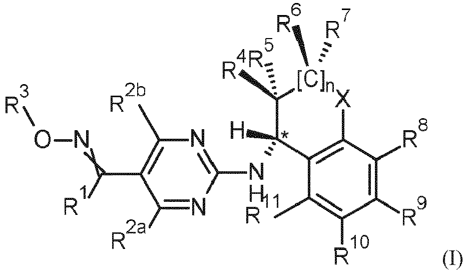 wobei die Reste R¹ bis R¹¹ sowie X und n gemäß einem der Ansprüche 1 bis 14 definiert sind,
durch Kondensation eines Guanidins des Typs (IV) oder eines Säureadditionssalzes davon 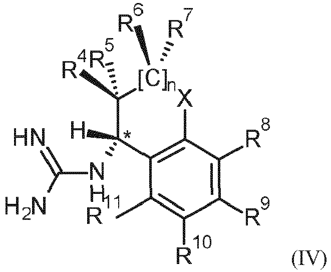 mit einem Keton der Formel (V) 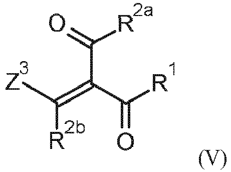 wobei der Rest Z³ für ein (C₁-C₆)-Alkoxy oder Di-(C₁-C₆)-Alkylamino steht,
und anschließend basenkatalysierte Umsetzung der erhaltenen Ketone der Formel (Ia), wobei Z² für R³-CO steht, 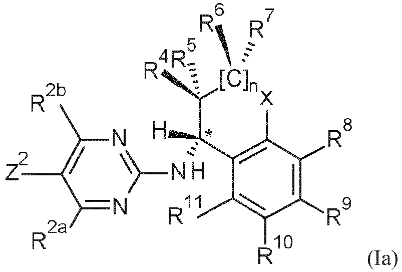 mit Hydroxylamin oder O-substituierten Hydroxylaminen zu den Verbindungen der allgemeinen Formel (I).

19. Herbizides Mittel oder pflanzenwachstumsregulierendes Mittel, **dadurch gekennzeichnet, dass** es eine oder mehrere Verbindungen der allgemeinen Formel (I) oder deren Salze nach einem der Ansprüche 1 bis 16 enthält.

20. Verfahren zur Bekämpfung von Schadpflanzen oder zur Wachstumsregulierung von Pflanzen, **dadurch gekennzeichnet, dass** man eine wirksame Menge von einer oder mehreren Verbindungen der allgemeinen Formel (I) oder deren Salzen nach einem der Ansprüche 1 bis 16 auf Pflanzen, Pflanzenteile, Pflanzensamen oder auf eine Anbaufläche appliziert.

21. Verwendung von Verbindungen der allgemeinen Formel (I) oder deren Salzen nach einem der Ansprüche 1 bis 16 als Herbizide oder als Pflanzenwachstumsregulatoren.

22. Verwendung nach Anspruch 21, **dadurch gekennzeichnet, dass** die Verbindungen der allgemeinen Formel (I) oder deren Salze zur Bekämpfung von Schadpflanzen oder zur Wachstumsregulierung von Pflanzen in Kulturen von Nutz- oder Zierpflanzen eingesetzt werden.

23. Verwendung nach den Ansprüchen 21 oder 22, **dadurch gekennzeichnet, dass** die Kulturpflanzen transgene Kulturpflanzen sind.

## Claims

1. Compounds of the general formula (I) 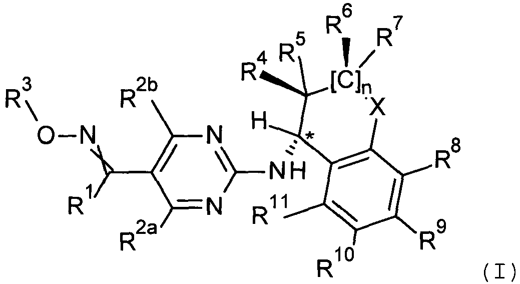 and the agrochemically acceptable salts thereof in which
R¹ is selected from the group consisting of
- (C₁-C₆)-alkyl, (C₁-C₆)-haloalkyl, (C₁-C₆)-alkylcarbonyl-(C₁-C₄)-alkyl;
- (C₁-C₆)-alkoxycarbonyl-(C₁-C₆) -alkyl, (C₁-C₆)-haloalkoxycarbonyl-(C₁-C₆) -alkyl, (C₁-C₆)-alkoxycarbonyl-(C₁-C₆)-haloalkyl, (C₁-C₆)-haloalkoxycarbonyl-(C₁-C₆)-haloalkyl;
- (C₂-C₆)-alkenyl, (C₂-C₆)-haloalkenyl;
- (C₂-C₆)-alkynyl, (C₂-C₆)-haloalkynyl; tri-(C₁-C₆)-alkylsilyl-(C₂-C₆)-alkynyl, di-(C₁-C₆)-alkylsilyl-(C₂-C₆)-alkynyl, mono- (C₁-C₆)-
- alkylsilyl-(C₂-C₆)-alkynyl; phenylsilyl-(C₂-C₆)-alkynyl; (C₆-C₁₄)-aryl which may be substituted on the aryl moiety in each case by halogen, (C₁-C₆)-alkyl and/or (C₁-C₆)-haloalkyl;
- (C₆-C₁₄)-aryl-(C₁-C₆)-alkyl;
- aminocarbonyl-(C₁-C₆)-alkyl;
- (C₁-C₆)-alkoxy- (C₁-C₆)-alkyl;
- (C₃-C₈)-cycloalkyl which is unsubstituted or mono- or polysubstituted on the cycloalkyl radical by (C₁-C₆)-alkyl and/or halogen; (C₃-C₈)-cycloalkyl-(C₁-C₆)-alkyl, (C₃-C₈)-cycloalkyl-(C₁-C₆)-haloalkyl;
- (C₃-C₈)-cycloalkenyl, (C₃-C₈)-cycloalkenyl-(C₁-C₆)-alkyl, (C₃-C₈)-cycloalkenyl-(C₁-C₆)-haloalkyl;
- hydroxy-(C₁-C₆)-alkyl, cyano-(C₁-C₆)-alkyl;
- (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆₎-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl;
R^{2a} and R^{2b} are each independently selected from the group consisting of
- hydrogen, halogen, hydroxyl, cyano, C(O)OH, C(O)NH₂;
- (C₁-C₆)-alkyl, (C₁-C₆)-haloalkyl, (C₁-C₆)-alkylcarbonyl, (C₁-C₆)-haloalkylcarbonyl, (C₁-C₆)-alkylcarbonyloxy, (C₁-C₆)-haloalkylcarbonyloxy, (C₁-C₆)-alkylcarbonyl-(C₁-C₄)-alkyl;
- (C₁-C₆)-alkoxy, (C₁-C₆)-haloalkoxy, (C₁-C₆)-alkoxycarbonyl, (C₁-C₆)-haloalkoxycarbonyl, (C₁-C₆)-alkoxycarbonyl-(C₁-C₆)-alkyl, (C₁-C₆)-haloalkoxycarbonyl-(C₁-C₆)-alkyl, (C₁-C₆)-alkoxycarbonyl-(C₁-C₆)-haloalkyl, (C₁-C₆)-haloalkoxycarbonyl-(C₁-C₆)-haloalkyl;
- (C₂-C₆)-alkenyl, (C₂-C₆)-haloalkenyl, (C₂-C₆)-alkenylcarbonyl, (C₂-C₆)-haloalkenylcarbonyl, (C₂-C₆)-alkenyloxy, (C₂-C₆)-haloalkenyloxy, (C₂-C₆)-alkenyloxycarbonyl, (C₂-C₆)-haloalkenyloxycarbonyl;
- (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl; tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₆-C₁₄)-aryl, (C₆-C₁₄)-aryloxy, (C₆-C₁₄)-arylcarbonyl and (C₆-C₁₄)-aryloxycarbonyl, each of which may be substituted on the aryl moiety by halogen, (C₁-C₆)-alkyl and/or (C₁-C₆)-haloalkyl;
- (C₆-C₁₄)-aryl-(C₁-C₆)-alkyl, (C₆-C₁₄)-aryl-(C₁-C₆)-alkoxy, (C₆-C₁₄)-aryl-(C₁-C₆)-alkylcarbonyl, (C₆-C₁₄)-aryl-(C₁-C₆)-alkylcarbonyloxy, (C₆-C₁₄)-aryl-(C₁-C₆)-alkoxycarbonyl, (C₆-C₁₄)-aryl-(C₁-C₆)-alkoxycarbonyloxy;
- aminocarbonyl-(C₁-C₆)-alkyl, di-(C₁-C₆)-alkylaminocarbonyl-(C₁-C₆)-alkyl;
- N-((C₁-C₆)-haloalkanoyl)-aminocarbonyl, mono-((C₆-C₁₄)-aryl) -aminocarbonyl, di-((C₆-C₁₄)-aryl)-aminocarbonyl;
- (C₁-C₆)-alkoxy-(C₁-C₆)-alkyl, (C₁-C₆)-alkoxy-(C₁-C₆)-alkoxy, (C₁-C₆)-alkoxycarbonyl-(C₁-C₆)-alkoxy;
- (C₃-C₈)-cycloalkyl which is unsubstituted or mono- or polysubstituted on the cycloalkyl radical by (C₁-C₆)-alkyl and/or halogen; (C₃-C₈)-cycloalkoxy, (C₃-C₈)-cycloalkyl-(C₁-C₆)-alkyl, (C₃-C₈)-cycloalkyl-(C₁-C₆)-haloalkyl, (C₃-C₈)-cycloalkyl-(C₁-C₆)-alkoxy, (C₃-C₈)-cycloalkyl-(C₁-C₆)-haloalkoxy, (C₃-C₈)-cycloalkylcarbonyl, (C₃-C₈)-cycloalkoxycarbonyl, (C₃-C₈)-cycloalkyl-(C₁-C₆)-alkylcarbonyl, (C₃-C₈)-cycloalkyl-(C₁-C₆)-haloalkylcarbonyl, (C₃-C₈)-cycloalkyl-(C₁-C₆)-alkoxycarbonyl, (C₃-C₈)-cycloalkyl-(C₁-C₆)-haloalkoxycarbonyl, (C₃-C₈)-cycloalkylcarbonyloxy, (C₃-C₈)-cycloalkoxycarbonyloxy, (C₃-C₈)-cycloalkyl-(C₁-C₆)-alkylcarbonyloxy, (C₃-C₈)-cycloalkyl-(C₁-C₆)-haloalkylcarbonyloxy, (C₃-C₈)-cycloalkyl- (C₁-C₆) -alkoxycarbonyloxy, (C₃-C₈)-cycloalkyl-(C₁-C₆)-haloalkoxycarbonyloxy;
- (C₃-C₈)-cycloalkenyl, (C₃-C₈)-cycloalkenyloxy, (C₃-C₈)-cycloalkenyl-(C₁-C₆)-alkyl, (C₃-C₈)-cycloalkenyl-(C₁-C₆)-haloalkyl, (C₃-C₈)-cycloalkenyl-(C₁-C₆)-alkoxy, (C₃-C₈)-cycloalkenyl-(C₁-C₆)-haloalkoxy, (C₃-C₈)-cycloalkenylcarbonyl, (C₃-C₈)-cycloalkenyloxycarbonyl, (C₃-C₈)-cycloalkenyl-(C₁-C₆)-alkylcarbonyl, (C₃-C₈)-cycloalkenyl-(C₁-C₆)-haloalkylcarbonyl, (C₃-C₈)-cycloalkenyl-(C₁-C₆)-alkoxycarbonyl, (C₃-C₈)-cycloalkenyl-(C₁-C₆)-haloalkoxycarbonyl, (C₃-C₈)-cycloalkenylcarbonyloxy, (C₃-C₈)-cycloalkenyloxycarbonyloxy, (C₃-C₈)-cycloalkenyl-(C₁-C₆)-alkylcarbonyloxy, (C₃-C₈)-cycloalkenyl-(C₁-C₆)-haloalkylcarbonyloxy, (C₃-C₈)-cycloalkenyl-(C₁-C₆)-alkoxycarbonyloxy, (C₃-C₈)-cycloalkenyl-(C₁-C₆)-haloalkoxycarbonyloxy;
- hydroxy-(C₁-C₆)-alkyl, hydroxy-(C₁-C₆)-alkoxy, cyano-(C₁-C₆)-alkoxy, cyano-(C₁-C₆)-alkyl;
- (C₁-C₆)-alkylsulfonyl, (C₁-C₆)-alkylthio, (C₁-C₆)-alkylsulfinyl, (C₁-C₆)-haloalkylsulfonyl, (C₁-C₆)-haloalkylthio, (C₁-C₆)-haloalkylsulfinyl, (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl, (C₁-C₆)-alkylsulfonyloxy, (C₁-C₆)-haloalkylsulfonyloxy, (C₁-C₆)-alkylthiocarbonyl, (C₁-C₆)-haloalkylthiocarbonyl, (C₁-C₆)-alkylthiocarbonyloxy, (C₁-C₆)-haloalkylthiocarbonyloxy, (C₁-C₆)-alkylthio-(C₁-C₆)-alkyl, (C₁-C₆)-alkylthio-(C₁-C₆)-alkoxy, (C₁-C₆)-alkylthio-(C₁-C₆)-alkylcarbonyl, (C₁-C₆)-alkylthio-(C₁-C₆)-alkylcarbonyloxy; (C₄-C₁₄)-arylsulfonyl, (C₆-C₁₄)-arylthio, (C₆-C₁₄)-arylsulfinyl, (C₃-C₈)-cycloalkylthio, (C₃-C₈)-alkenylthio, (C₃-C₈)-cycloalkenylthio and (C₃-C₆)-alkynylthio;
R¹ and R^{2a} are joined to one another via a bond so as to form, together with carbons 4 and 5 of the pyrimidine and the carbonyl group in the 5 position of the pyrimidine, a 5- to 7-membered partly hydrogenated carbo- or heterocycle in which the heteroatoms are selected from the group consisting of oxygen and sulfur, where a sulfoxide group or a sulfonyl group may be present in place of the sulfur, and the carbo- or heterocycle is unsubstituted or substituted by one or more substituents selected from the group consisting of (C₁-C₆)-alkyl, (C₁-C₆)-haloalkyl, (C₃-C₆)-cycloalkyl, spiro-(C₂-C₅)-cycloalkyl and (C₆-C₁₄)-aryl which may be substituted by one or more substituents selected from fluorine, chlorine, bromine or iodine;
R³ is selected from the group consisting of
- hydrogen; (C₁-C₆)-alkyl, (C₁-C₆)-haloalkyl, (C₁-C₆)-alkylcarbonyl-(C₁-C₄)-alkyl;
- (C₂-C₆)-alkenyl, (C₂-C₆)-haloalkenyl;
- (C₂-C₆)-alkynyl, (C₂-C₆)-haloalkynyl; tri-(C₁-C₆)-alkylsilyl-(C₂-C₆)-alkynyl, di-(C₁-C₆)-alkylsilyl-(C₂-C₆)-alkynyl, mono-(C₁-C₆)-alkylsilyl-(C₂-C₆)-alkynyl; phenylsilyl-(C₂-C₆)-alkynyl;
- (C₁-C₆)-alkoxycarbonyl-(C₁-C₆)-alkyl, (C₁-C₆)-haloalkoxycarbonyl-(C₁-C₆) -alkyl, (C₁-C₆)-alkoxycarbonyl-(C₁-C₆)-haloalkyl, (C₁-C₆)-haloalkoxycarbonyl-(C₁-C₆)-haloalkyl;
- (C₆-C₁₄)-aryl which may be substituted on the aryl moiety in each case by halogen, (C₁-C₆)-alkyl and/or (C₁-C₆)-haloalkyl;
- (C₂-C₁₄)-hetaryl which may be substituted on the hetaryl moiety in each case by halogen, (C₁-C₆)-alkyl and/or (C₁-C₆)-haloalkyl;
- (C₆-C₁₄)-aryl-(C₁-C₆)-alkyl;
- aminocarbonyl-(C₁-C₆)-alkyl;
- (C₁-C₆)-alkoxy, (C₁-C₆)-alkoxy-(C₁-C₆)-alkyl;
- (C₃-C₈)-cycloalkyl which may be unsubstituted or mono- or polysubstituted on the cycloalkyl radical by (C₁-C₆)-alkyl and/or halogen; (C₃-C₈)-cycloalkyl-(C₁-C₆)-alkyl, (C₃-C₈)-cycloalkyl-(C₁-C₆)-haloalkyl;
- (C₃-C₈)-cycloalkenyl-(C₁-C₆)-alkyl, (C₃-C₈)-cycloalkenyl-(C₁-C₆) -haloalkyl, hydroxy-(C₁-C₆)-alkyl, cyano-(C₁-C₆)-alkyl;
- (C₁-C₆)-alkylsulfonyl-(C₁-C₆)-alkyl, (C₁-C₆)-alkylthio-(C₁-C₆)-alkyl, (C₁-C₆)-alkylsulfinyl-(C₁-C₆)-alkyl, (C₁-C₆)-haloalkylsulfonyl-(C₁-C₆)-alkyl, (C₁-C₆)-haloalkylthio-(C₁-C₆)-alkyl, (C₁-C₆)-haloalkylsulfinyl-(C₁-C₆)-alkyl, (C₁-C₆)-alkylsulfonyl-(C₁-C₆)-haloalkyl, (C₁-C₆)-alkylthio-(C₁-C₆)-haloalkyl, (C₁-C₆)-alkylsulfinyl-(C₁-C₆)-haloalkyl, (C₁-C₆)-haloalkylsulfonyl-(C₁-C₆)-haloalkyl, (C₁-C₆)-haloalkylthio-(C₁-C₆)-haloalkyl, (C₁-C₆)-haloalkylsulfinyl-(C₁-C₆)-haloalkyl;
R⁴ and R⁵ are each independently selected from the group consisting of hydrogen, (C₁-C₆)-alkyl, (C₁-C₆) -haloalkyl, hydroxyl, (C₁-C₆)-alkoxy and (C₁-C₆) -haloalkoxy; or the R⁴ and R⁵ radicals together with the carbon atom to which they are bonded form a three- to seven-membered ring;
R⁶ and R⁷ are each independently selected from the group consisting of hydrogen, (C₁-C₆)-alkyl, (C₁-C₆)-haloalkyl, (C₁-C₆)-alkoxy, (C₁-C₆)-haloalkoxy, (C₆-C₁₄)-aryl, (C₆-C₁₄)-aryloxy, (C₆-C₁₄)-arylcarbonyl and (C₆-C₁₄)-aryloxycarbonyl; or the R⁶ and R⁷ radicals together form a (C₁-C₇)-alkylene group which may contain one or more oxygen and/or sulfur atoms, where the (C₁-C₇)-alkylene group may be mono- or polysubstituted by halogen and the respective halogen substituents may be the same or different;
n is the serial number 0, 1 or 2;
R⁸, R⁹, R¹⁰ and R¹¹ are each independently selected from the group consisting of hydrogen, halogen, cyano, C(O)OH, C(O)NH₂, (C₁-C₆)-alkyl, (C₁-C₆)-alkylcarbonyl, (C₁-C₆)-alkyloxycarbonyl, (C₁-C₆)-alkylaminocarbonyl, (C₁-C₆)-dialkylaminocarbonyl, (C₁-C₆)-haloalkyl, (C₁-C₆)-alkoxy, (C₁-C₆)-haloalkoxy, (C₂-C₆)-alkenyl, (C₂-C₆)-alkynyl, (C₂-C₆)-haloalkynyl, (C₂-C₆)-alkynylcarbonyl, (C₂-C₆)-haloalkynylcarbonyl, (C₂-C₆)-alkynyloxy, (C₂-C₆)-haloalkynyloxy, (C₂-C₆)-alkynyloxycarbonyl, (C₂-C₆)-haloalkynyloxycarbonyl and nitro, where the R⁹ and R¹⁰ radicals may be joined by an -O-CH₂-O-group to form a ring;
X is a chemical bond, CH₂, O, S, carbonyl, NH, CR¹²R¹³, NR¹⁴, CH₂O, or CH₂S, where the carbon atom in the two latter groups is bonded to the aromatic moiety and the heteroatom O or S to the partly hydrogenated amine moiety;
R¹² and R¹³ are each independently selected from the group consisting of hydrogen, (C₁-C₆)-alkyl and (C₁-C₆)-haloalkyl; and
R¹⁴ is selected from the group consisting of hydrogen, (C₁-C₆)-alkyl and (C₁-C₆)-haloalkyl.

2. Compounds of the general formula (I) according to Claim 1, **characterized in that** R¹ is selected from the group consisting of (C₁-C₆)-alkyl, (C₁-C₆)-haloalkyl, (C₁-C₆)-alkoxy-(C₁-C₆)-alkyl, (C₃-C₈)-cycloalkyl, (C₃-C₈)-cycloalkyl-(C₁-C₆)-alkyl and (C₃-C₈)-cycloalkyl-(C₁-C₆)-haloalkyl, where the cycloalkyl radical in each case is unsubstituted or substituted by (C₁-C₆)-alkyl and/or halogen.

3. Compounds of the general formula (I) according to Claim 1 or 2, **characterized in that** R^{2a} and R^{2b} are each independently selected from the group consisting of hydrogen, (C₁-C₆)-alkyl, (C₁-C₆)-haloalkyl, (C₁-C₆)-alkoxy, (C₁-C₆)-alkoxy-(C₁-C₆)-alkyl, (C₃-C₈)-cycloalkyl, (C₃-C₈)-cycloalkyl-(C₁-C₆)-alkyl and (C₃-C₈)-cycloalkyl-(C₁-C₆)-haloalkyl, where the cycloalkyl radical in each case is unsubstituted or substituted by (C₁-C₆)-alkyl and/or halogen.

4. Compounds of the general formula (I) according to any of Claims 1 to 3, **characterized in that** R¹ and R^{2a} may be joined to one another via a bond so as to give, together with carbons 4 and 5 of the pyrimidine and the carbonyl group in the 5 position of the pyrimidine, a 5- to 7-membered partly hydrogenated carbo- or heterocycle in which the heteroatoms are selected from the group consisting of oxygen and sulfur, where a sulfoxide group or a sulfonyl group may be present in place of the sulfur, and the carbo- or heterocycle is unsubstituted or is substituted by one or more substituents selected from the group consisting of (C₁-C₃)-alkyl, (C₁-C₃)-haloalkyl, (C₃-C₆)-cycloalkyl, and phenyl (C₆H₅) which may be mono- or polysubstituted by fluorine, chlorine, bromine or iodine.

5. Compounds of the general formula (I) according to any of Claims 1 to 4, **characterized in that** the R³ is selected from the group consisting of hydrogen, (C₁-C₆)-alkyl, (C₁-C₆)-haloalkyl, (C₁-C₆)-alkoxy, (C₁-C₆)-alkoxy-(C₁-C₆)-alkyl, (C₂-C₆)-alkenyl, (C₂-C₆)-haloalkenyl, (C₂-C₆)-alkynyl, and (C₂-C₆)-haloalkynyl and (C₆-C₁₄)-aryl-(C₁-C₆)-alkyl.

6. Compounds of the general formula (I) according to any of Claims 1 to 5, **characterized in that** R⁴ and R⁵ are each independently selected from the group consisting of hydrogen, hydroxyl, (C₁-C₆)-alkyl, (C₁-C₆)-alkylphenyl and (C₁-C₆)-alkoxy.

7. Compounds of the general formula (I) according to any of Claims 1 to 6, **characterized in that** R⁶ and R⁷ are each independently selected from the group consisting of hydrogen, (C₁-C₆)-alkyl and (C₆-C₁₄)-aryl.

8. Compounds of the general formula (I) according to any of Claims 1 to 7, **characterized in that** R⁸ is selected from the group consisting of of hydrogen, halogen, (C₁-C₆)-alkyl, (C₁-C₆)-alkoxy and (C₆-C₁₄)-aryl.

9. Compounds of the general formula (I) according to any of Claims 1 to 8, **characterized in that** R⁹ is selected from the group consisting of hydrogen, halogen, (C₁-C₆)-alkyl and (C₁-C₆)-alkoxy.

10. Compounds of the general formula (I) according to any of Claims 1 to 9, **characterized in that** R¹⁰ is selected from the group consisting of hydrogen, halogen, cyano, aminocarbonyl, hydroxycarbonyl, (C₁-C₆) -alkyl, (C₁-C₆) -haloalkyl, (C₁-C₆) -alkoxy, (C₂-C₆) -alkenyl, (C₂-C₆) -alkynyl, (C₁-C₆) -alkyl- (C₂-C₆) -alkynyl, hydroxy- (C₁-C₆) -alkyl- (C₂-C₆) -alkynyl, (C₁-C₆) -alkoxy- (C₂-C₆) -alkynyl and aryl- (C₂-C₆) - alkynyl.

11. Compounds of the general formula (I) according to any of Claims 1 to 10, **characterized in that** R¹¹ is selected from the group consisting of hydrogen and (C₁-C₆) -alkyl.

12. Compounds of the general formula (I) according to any of Claims 1 to 11, **characterized in that** X is selected from the group consisting of a chemical bond, CH₂, O, S, carbonyl, NH, CH (C₁-C₆) alkyl, N(C₁-C₆)alkyl, OCH₂, and SCH₂, where the carbon atom in the two latter groups is bonded to the aromatic moiety and the heteroatom O or S to the partly hydrogenated amine moiety.

13. Compounds of the general formula (I) according to any of Claims 1 to 12, **characterized in that** R⁹ and R¹⁰ are joined by a -O-CH₂-O- group to form a ring.

14. Compounds of the general formula (I) according to any of Claims 1 to 13, **characterized in that** the serial number n is 0 or 1.

15. Compounds of the general formula (I) 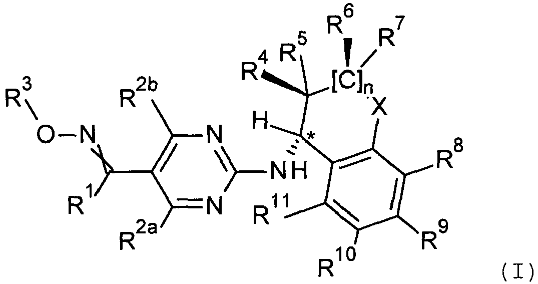 according to any of Claims 1 to 14, **characterized in that** the chiral carbon atom indicated by (*) has (R) configuration.

16. Compounds of the general formula (I) 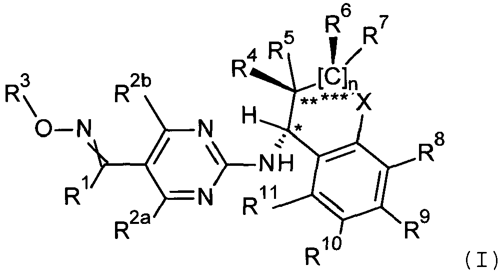 according to any of Claims 1 to 15, **characterized in that** the chiral carbon atom indicated by (*) has (R) configuration and the chiral carbon atom indicated by (**) has (S) configuration.

17. Process for preparing compounds of the general formula (I) and/or agrochemically acceptable salts thereof and/or agrochemically acceptable quaternized nitrogen derivatives thereof 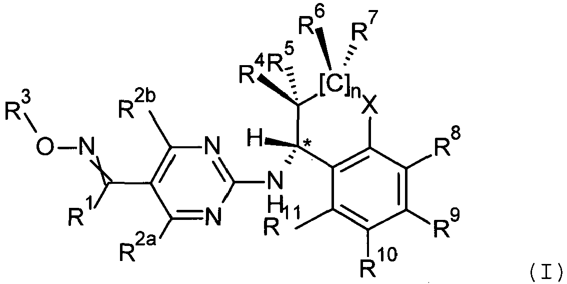 where the R¹ to R¹¹ and X radicals and n are as defined in any of Claims 1 to 14, and wherein
a compound of the general formula (II) 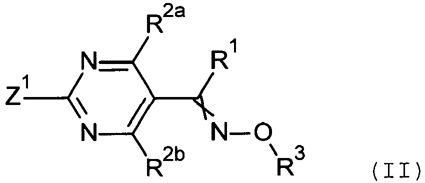 where R¹, R^{2a}, R^{2b} and R³ are as defined in any of Claims 1 to 14 and Z¹ is an exchangeable radical or a leaving group selected from the group consisting of fluorine, chlorine, bromine, iodine, a (C₁-C₄)-alkylsulfanyl, (C₁-C₄) -alkylsulfinyl, (C₁-C₄)-alkylsulfonyl, an unsubsituted or mono- or poly-fluorine-, -chlorine-, -bromine- or -(C₁-C₄)-alkyl- or -(C₁-C₄)-alkoxy-subsituted phenyl-(C₁-C₄)-alkylsulfonyl, or a (C₁-C₄)-alkylphenylsulfonyl, is reacted with an amine of the general formula (III) or with an acid addition salt of the amine of the general formula (III) 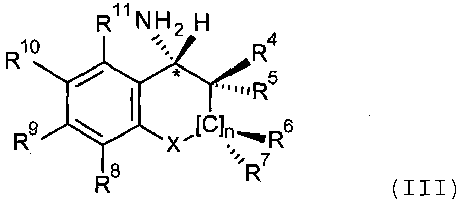 where the R⁴ to R¹¹ and X radicals and n are as defined in any of Claims 1 to 14.

18. Process for preparing compounds of the general formula (I) and/or agrochemically acceptable salts thereof and/or agrochemically acceptable quaternized nitrogen derivatives thereof 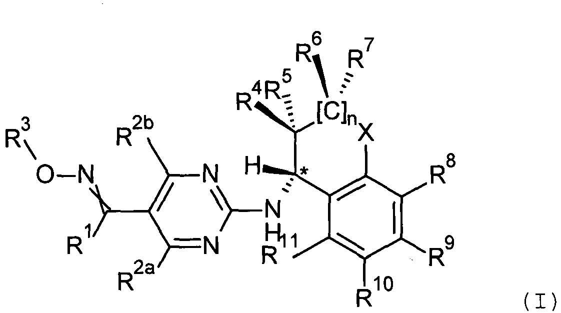 where the R¹ to R¹¹ and X radicals and n are as defined in any of Claims 1 to 14,
by condensation of a guanidine of the (IV) type or an acid addition salt thereof 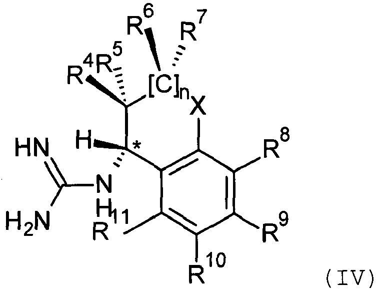 with a ketone of the formula (V) 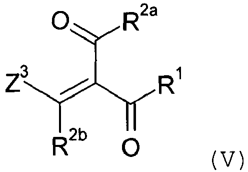 where the Z³ radical is a (C₁-C₆) -alkoxy or di-(C₁-C₆)-alkylamino,
followed by base-catalyzed reaction of the resulting ketones of the formula (Ia) where Z² is R³-CO 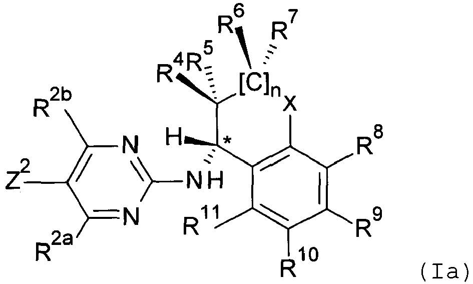 with hydroxylamine or O-substituted hydroxylamines to give the compounds of the general formula (I).

19. Herbicidal composition or plant growth-regulating composition, **characterized in that** it comprises one or more compounds of the general formula (I) or salts thereof according to any of Claims 1 to 16.

20. Method of controlling harmful plants or of regulating the growth of plants, **characterized in that** an effective amount of one or more compounds of the general formula (I) or salts thereof according to any of Claims 1 to 16 is applied to plants, plant parts, plant seeds or an area under cultivation.

21. Use of compounds of the general formula (I) or salts thereof according to any of Claims 1 to 16 as herbicides or as plant growth regulators.

22. Use according to Claim 21, **characterized in that** the compounds of the general formula (I) or salts thereof are used to control harmful plants or to regulate the growth of plants in crops of useful plants or ornamental plants.

23. Use according to Claims 21 or 22, **characterized in that** the crop plants are transgenic crop plants.

## Revendications

1. Composés de la formule générale (I) : 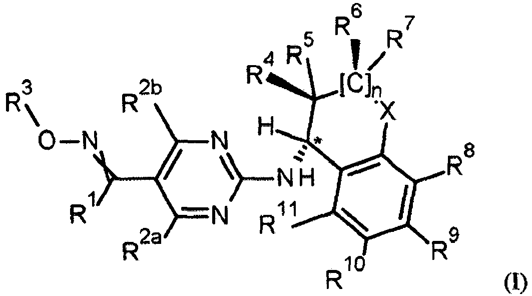 et leurs sels agrochimiquement compatibles, dans lesquels
R¹ est choisi dans le groupe constitué par :
- alkyle en (C₁-C₆), halogénoalkyle en (C₁-C₆), alkylcarbonyle en (C₁-C₆) -alkyle en (C₁-C₄) ;
- alcoxycarbonyle en (C₁-C₆)-alkyle en (C₁-C₆), haloalcoxycarbonyle en (C₁-C₆) -alkyle en (C₁-C₆) , alcoxycarbonyle en (C₁-C₆)-halogénoalkyle en (C₁-C₆), halogénoalcoxycarbonyle en (C₁-C₆) -halogénoalkyle en (C₁-C₆) ;
- alcényle en (C₂-C₆), halogénoalcényle en (C₂-C₆) ;
- alcynyle en (C₂-C₆), halogénoalcynyle en (C₂-C₆) ; tri-alkylsilyle en (C₁-C₆) -alcynyle en (C₂-C₆), di-alkylsilyle en (C₁-C₆) -alcynyle en (C₂-C₆), mono-alkylsilyle en (C₁-C₆)-alcynyle en (C₂-C₆) ; phénylsilyl-alcynyle en (C₂-C₆) ;
- aryle en (C₆-C₁₄), qui peut à chaque fois être substitué dans la partie aryle avec halogène, alkyle en (C₁-C₆) et/ou haloalkyle en (C₁-C₆) ;
- aryle en (C₆-C14)-alkyle en (C₁-C₆) ;
- aminocarbonyl-alkyle en (C₁-C₆) ;
- alcoxy en (C₁-C₆) -alkyle en (C₁-C₆) ;
- cycloalkyle en (C₃-C₈), qui est non substitué ou substitué une fois ou plusieurs fois sur le radical cycloalkyle par alkyle en (C₁-C₆) et/ou halogène ; cycloalkyle en (C₃-C₈)-alkyle en (C₁-C₆), cycloalkyle en (C₃-C₈)-haloalkyle en (C₁-C₆) ;
- cycloalcényle en (C₃-C₈), cycloalcényle en (C₃-C₈)-alkyle en (C₁-C₆), cycloalcényle en (C₃-C₈)-halogénoalkyle en (C₁-C₆) ;
- hydroxy-alkyle en (C₁-C₆), cyano-alkyle en (C₁-C₆) ;
- alkylsulfonyle en (C₁-C₆) -alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆) -alkyle en (C₁-C₆), haloalkylsulfinyle en (C₁-C₆) -alkyle en (C₁-C₆), alkylsulfonyle en (C₁-C₆) -haloalkyle en (C₁-C₆), alkylthio en (C₁-C₆)-haloalkyle en (C₁-C₆), alkylsulfinyle en (C₁-C₆) -haloalkyle en (C₁-C₆), haloalkylsulfonyle en (C₁-C₆) -haloalkyle en (C₁-C₆), haloalkylthio en (C₁-C₆)-haloalkyle en (C₁-C₆), haloalkylsulfinyle en (C₁-C₆) -haloalkyle en (C₁-C₆) ;
R^{2a} et R^{2b} sont choisis, chacun indépendamment l'un de l'autre, dans le groupe constitué par :
- hydrogène, halogène, hydroxy, cyano, C(O)OH, C(O)NH₂ ;
- alkyle en (C₁-C₆), halogénoalkyle en (C₁-C₆), alkylcarbonyle en (C₁-C₆), haloalkylcarbonyle en (C₁-C₆), alkylcarbonyloxy en (C₁-C₆), halogénoalkylcarbonyloxy en (C₁-C₆), alkylcarbonyle en (C₁-C₆) -alkyle en (C₁-C₄) ;
- alcoxy en (C₁-C₆), halogénoalcoxy en (C₁-C₆), alcoxycarbonyle en (C₁-C₆), haloalcoxycarbonyle en (C₁-C₆), alcoxycarbonyle en (C₁-C₆)-alkyle en (C₁-C₆), haloalcoxycarbonyle en (C₁-C₆) -alkyle en (C₁-C₆), alcoxycarbonyle en (C₁-C₆)-halogénoalkyle en (C₁-C₆), halogénoalcoxycarbonyle en (C₁-C₆)-halogénoalkyle en (C₁-C₆) ;
- alcényle en (C₂-C₆), halogénoalcényle en (C₂-C₆), alcénylcarbonyle en (C₂-C₆), haloalcénylcarbonyle en (C₂-C₆), alcényloxy en (C₂-C₆), haloalcényloxy en (C₂-C₆), alcényloxycarbonyle en (C₂-C₆), haloalcényloxycarbonyle en (C₂-C₆) ;
- alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) ; tri-alkylsilyle en (C₁-C₆)-alcynyle en (C₂-C₆), di-alkylsilyle en (C₁-C₆)-alcynyle en (C₂-C₆), mono-alkylsilyle en (C₁-C₆)-alcynyle en (C₂-C₆) ; phénylsilyl-alcynyle en (C₂-C₆) ;
- aryle en (C₆-C₁₄), aryloxy en (C₆-C₁₄), arylcarbonyle en (C₆-C₁₄) et aryloxycarbonyle en (C₆-C₁₄), qui peuvent à chaque fois être substitués dans la partie aryle avec halogène, alkyle en (C₁-C₆) et/ou haloalkyle en (C₁-C₆) ;
- aryle en (C₆-C14)-alkyle en (C₁-C₆), aryle en (C₆-C₁₄)-alcoxy en (C₁-C₆), aryle en (C₆-C14)-alkylcarbonyle en (C₁-C₆), aryle en (C₆-C14)-alkylcarbonyloxy en (C₁-C₆), aryle en (C₆-C14)-alcoxycarbonyle en (C₁-C₆), aryle en (C₆-C14)-alcoxycarbonyloxy en (C₁-C₆) ;
- aminocarbonyl-alkyle en (C₁-C₆), di-alkylaminocarbonyle en (C₁-C₆)-alkyle en (C₁-C₆) ;
- N-(haloalcanoyle en (C₁-C₆))-amino-carbonyle, mono-(aryle en (C₆-C₁₄))-amino-carbonyle, di-(aryle en (C₆-C₁₄))-amino-carbonyle ;
- alcoxy en (C₁-C₆) -alkyle en (C₁-C₆), alcoxy en (C₁-C₆)-alcoxy en (C₁-C₆), alcoxycarbonyle en (C₁-C₆) -alcoxy en (C₁-C₆) ;
- cycloalkyle en (C₃-C₈), qui est non substitué ou substitué une fois ou plusieurs fois sur le radical cycloalkyle par alkyle en (C₁-C₆) et/ou halogène ; cycloalcoxy en (C₃-C₈), cycloalkyle en (C₃-C₈)-alkyle en (C₁-C₆), cycloalkyle en (C₃-C₈)-haloalkyle en (C₁-C₆), cycloalkyle en (C₃-C₈)-alcoxy en (C₁-C₆), cycloalkyle en (C₃-C₈)-haloalcoxy en (C₁-C₆), cycloalkylcarbonyle en (C₃-C₈), cycloalcoxycarbonyle en (C₃-C₈), cycloalkyle en (C₃-C₈)-alkylcarbonyle en (C₁-C₆), cycloalkyle en (C₃-C₈)-haloalkylcarbonyle en (C₁-C₆), cycloalkyle en (C₃-C₈) -alcoxycarbonyle en (C₁-C₆), cycloalkyle en (C₃-C₈)-haloalcoxycarbonyle en (C₁-C₆), cycloalkylcarbonyloxy en (C₃-C₈), cycloalcoxycarbonyloxy en (C₃-C₈), cycloalkyle en (C₃-C₈)-alkylcarbonyloxy en (C₁-C₆), cycloalkyle en (C₃-C₈)-halogénoalkylcarbonyloxy en (C₁-C₆), cycloalkyle en (C₃-C₈)-alcoxycarbonyloxy en (C₁-C₆), cycloalkyle en (C₃-C₈)-haloalcoxycarbonyloxy en (C₁-C₆) ;
- cycloalcényle en (C₃-C₈), cycloalcényloxy en (C₃-C₈), cycloalcényle en (C₃-C₈)-alkyle en (C₁-C₆), cycloalcényle en (C₃-C₈) -halogénoalkyle en (C₁-C₆), cycloalcényle en (C₃-C₈)-alcoxy en (C₁-C₆), cycloalcényle en (C₃-C₈) -halogénoalcoxy en (C₁-C₆), cycloalcénylcarbonyle en (C₃-C₈), cycloalcényloxycarbonyle en (C₃-C₈), cycloalcényle en (C₃-C₈)-alkylcarbonyle en (C₁-C₆), cycloalcényle en (C₃-C₈)-halogénoalkylcarbonyle en (C₁-C₆), cycloalcényle en (C₃-C₈)-alcoxycarbonyle en (C₁-C₆), cycloalcényle en (C₃-C₈)-haloalcoxycarbonyle en (C₁-C₆), cycloalcénylcarbonyloxy en (C₃-C₈), cycloalcényloxycarbonyloxy en (C₃-C₈), cycloalcényle en (C₃-C₈)-alkylcarbonyloxy en (C₁-C₆), cycloalcényle en (C₃-C₈) -halogénoalkylcarbonyloxy en (C₁-C₆), cycloalcényle en (C₃-C₈)-alcoxycarbonyloxy en (C₁-C₆), cycloalcényle en (C₃-C₈)-haloalcoxycarbonyloxy en (C₁-C₆) ;
- hydroxy-alkyle en (C₁-C₆), hydroxy-alcoxy en (C₁-C₆), cyano-alcoxy en (C₁-C₆), cyano-alkyle en (C₁-C₆) ;
- alkylsulfonyle en (C₁-C₆), alkylthio en (C₁-C₆), alkylsulfinyle en (C₁-C₆), haloalkylsulfonyle en (C₁-C₆), halogénoalkylthio en (C₁-C₆), halogénoalkylsulfinyle en (C₁-C₆), alkylsulfonyle en (C₁-C₆) -alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆)-alkyle en (C₁-C₆), haloalkylsulfinyle en (C₁-C₆)-alkyle en (C₁-C₆), alkylsulfonyle en (C₁-C₆) -haloalkyle en (C₁-C₆), alkylthio en (C₁-C₆) -haloalkyle en (C₁-C₆), alkylsulfinyle en (C₁-C₆) -haloalkyle en (C₁-C₆), haloalkylsulfonyle en (C₁-C₆) haloalkyle en (C₁-C₆), haloalkylthio en (C₁-C₆) -haloalkyle en (C₁-C₆), haloalkylsulfinyle en (C₁-C₆) -haloalkyle en (C₁-C₆), alkylsulfonyloxy en (C₁-C₆), halogénoalkylsulfonyloxy en (C₁-C₆), alkylthiocarbonyle en (C₁-C₆), haloalkylthiocarbonyle en (C₁-C₆), alkylthiocarbonyloxy en (C₁-C₆), haloalkylthiocarbonyloxy en (C₁-C₆), alkylthio en (C₁-C₆)-alkyle eh (C₁-C₆), alkylthio en (C₁-C₆)-alcoxy en (C₁-C₆), alkylthio en (C₁-C₆)-alkylcarbonyle en (C₁-C₆), alkylthio en (C₁-C₆)-alkylcarbonyloxy en (C₁-C₆) ; arylsulfonyle en (C₄-C₁₄), arylthio en (C₆-C₁₄), arylsulfinyle en (C₆-C₁₄), cycloalkylthio en (C₃-C₈), alcénylthio en (C₃-C₈), cycloalcénylthio en (C₃-C₈) et alcynylthio en (C₃-C₆) ; R¹ et R^{2a} sont reliés l'un avec l'autre par une liaison, de manière à former ensemble avec les carbones 4 et 5 de la pyrimidine et le groupe carbonyle à la position 5 de la pyrimidine un carbo- ou hétérocycle partiellement hydrogéné de 5 à 7 chaînons, où les hétéroatomes sont choisis dans le groupe constitué par l'oxygène ou le soufre, un groupe sulfoxyde ou un groupe sulfonyle pouvant être présent à la place du soufre, et le carbo- ou l'hétérocycle étant non substitué ou substitué avec un ou plusieurs substituants choisis dans le groupe constitué par alkyle en (C₁-C₆), haloalkyle en (C₁-C₆), cycloalkyle en (C₃-C₆), spiro-cycloalkyle en (C₂-C₅) et aryle en (C₆-C₁₄), qui peut être substitué avec un ou plusieurs substituants choisis parmi fluor, chlore, brome ou iode ;
R³ est choisi dans le groupe constitué par :
- hydrogène ;
- alkyle en (C₁-C₆), halogénoalkyle en (C₁-C₆), alkylcarbonyle en (C₁-C₆)-alkyle en (C₁-C₄) ;
- alcényle en (C₂-C₆), halogénoalcényle en (C₂-C₆) ;
- alcynyle en (C₂-C₆), halogénoalcynyle en (C₂-C₆) ; tri-alkylsilyle en (C₁-C₆) -alcynyle en (C₂-C₆), di-alkylsilyle en (C₁-C₆) -alcynyle en (C₂-C₆), mono-alkylsilyle en (C₁-C₆) -alcynyle en (C₂-C₆) ; phénylsilyl-alcynyle en (C₂-C₆) ;
- alcoxycarbonyle en (C₁-C₆) -alkyle en (C₁-C₆), haloalcoxycarbonyle en (C₁-C₆)-alkyle en (C₁-C₆), alcoxycarbonyle en (C₁-C₆) -halogénoalkyle en (C₁-C₆), halogénoalcoxycarbonyle en (C₁-C₆) -halogénoalkyle en (C₁-C₆) ;
- aryle en (C₆-C₁₄), qui peut à chaque fois être substitué dans la partie aryle avec halogène, alkyle en (C₁-C₆) et/ou haloalkyle en (C₁-C₆) ;
- het-aryle en (C₂-C₁₄), qui peut à chaque fois être substitué dans la partie het-aryle avec halogène, alkyle en (C₁-C₆) et/ou haloalkyle en (C₁-C₆) ;
- aryle en (C₆-C₁₄)-alkyle en (C₁-C₆) ;
- aminocarbonyl-alkyle en (C₁-C₆) ;
- alcoxy en (C₁-C₆), alcoxy en (C₁-C₆)-alkyle en (C₁-C₆) ;
- cycloalkyle en (C₃-C₈), qui peut être non substitué ou substitué une fois ou plusieurs fois sur le radical cycloalkyle par alkyle en (C₁-C₆) et/ou halogène ; cycloalkyle en (C₃-C₈) -alkyle en (C₁-C₆), cycloalkyle en (C₃-C₈)-haloalkyle en (C₁-C₆) ;
- cycloalcényle en (C₃-C₈)-alkyle en (C₁-C₆), cycloalcényle en (C₃-C₈)-halogénoalkyle en (C₁-C₆), hydroxy-alkyle en (C₁-C₆), cyano-alkyle en (C₁-C₆) ;
- alkylsulfonyle en (C₁-C₆)-alkyle en (C₁-C₆), alkylthio en (C₁-C₆)-alkyle en (C₁-C₆), alkylsulfinyle en (C₁-C₆)-alkyle en (C₁-C₆), haloalkylsulfonyle en (C₁-C₆)-alkyle en (C₁-C₆), haloalkylthio en (C₁-C₆)-alkyle en (C₁-C₆), haloalkylsulfinyle en (C₁-C₆) -alkyle en (C₁-C₆), alkylsulfonyle en (C₁-C₆) -haloalkyle en (C₁-C₆), alkylthio en (C₁-C₆) -haloalkyle en (C₁-C₆), alkylsulfinyle en (C₁-C₆) -haloalkyle en (C₁-C₆), haloalkylsulfonyle en (C₁-C₆) -haloalkyle en (C₁-C₆), haloalkylthio en (C₁-C₆) -haloalkyle en (C₁-C₆), haloalkylsulfinyle en (C₁-C₆) -haloalkyle en (C₁-C₆) ;
R⁴ et R⁵ sont chacun choisis indépendamment l'un de l'autre dans le groupe constitué par hydrogène, alkyle en (C₁-C₆), haloalkyle en (C₁-C₆), hydroxy, alcoxy en (C₁-C₆) et halogénoalcoxy en (C₁-C₆) ; ou les radicaux R⁴ et R⁵ forment ensemble avec l'atome de carbone auquel ils sont reliés un cycle de trois à sept chaînons ;
R⁶ et R⁷ sont chacun choisis indépendamment l'un de l'autre dans le groupe constitué par hydrogène, alkyle en (C₁-C₆), haloalkyle en (C₁-C₆), alcoxy en (C₁-C₆), halogénoalcoxy en (C₁-C₆), aryle en (C₆-C₁₄), aryloxy en (C₆-C₁₄), arylcarbonyle en (C₆-C₁₄) et aryloxycarbonyle en (C₆-C₁₄) ; ou les radicaux R⁶ et R⁷ forment ensemble un groupe alkylène en (C₁-C₇), qui peut contenir un ou plusieurs atomes d'oxygène et/ou de soufre, le groupe alkylène en (C₁-C₇) pouvant être substitué une fois ou plusieurs fois par halogène, et les substituants halogène respectifs pouvant être identiques ou différents ;
n représente le nombre 0, 1 ou 2 ;
R⁸, R⁹, R¹⁰ et R¹¹ sont chacun choisis indépendamment les uns des autres dans le groupe constitué par hydrogène, halogène, cyano, C(O)OH, C(O)NH₂, alkyle en (C₁-C₆), alkylcarbonyle en (C₁-C₆), alkyloxycarbonyle en (C₁-C₆), alkylaminocarbonyle en (C₁-C₆), di-alkylaminocarbonyle en (C₁-C₆), haloalkyle en (C₁-C₆), alcoxy en (C₁-C₆), halogénoalcoxy en (C₁-C₆), alcényle en (C₂-C₆), alcynyle en (C₂-C₆), halogénoalcynyle en (C₂-C₆), alcynylcarbonyle en (C₂-C₆), haloalcynylcarbonyle en (C₂-C₆), alcynyloxy en (C₂-C₆), haloalcynyloxy en (C₂-C₆), alcynyloxycarbonyle en (C₂-C₆), halogénoalcynyloxycarbonyle en (C₂-C₆) et nitro, les radicaux R⁹ et R¹⁰ pouvant être reliés par un groupe -O-CH₂-O- en formant un cycle ;
X représente une liaison chimique, CH₂, O, S, carbonyle, NH, CR¹²R¹³, NR¹⁴, CH₂O ou CH₂S, l'atome de carbone dans les deux derniers groupes cités étant relié à la partie aromatique et l'hétéroatome O ou S étant relié à la partie partiellement hydrogénée de l'amine ;
R¹² et R¹³ sont chacun choisis indépendamment l'un de l'autre dans le groupe constitué par hydrogène, alkyle en (C₁-C₆) et haloalkyle en (C₁-C₆) ; et
R¹⁴ est choisi dans le groupe constitué par hydrogène, alkyle en (C₁-C₆) et haloalkyle en (C₁-C₆) .

2. Composés de la formule générale (I) selon la revendication 1, **caractérisés en ce que** R¹ est choisi dans le groupe constitué par alkyle en (C₁-C₆), haloalkyle en (C₁-C₆), alcoxy en (C₁-C₆)-alkyle en (C₁-C₆), cycloalkyle en (C₃-C₈), cycloalkyle en (C₃-C₈)-alkyle en (C₁-C₆) et cycloalkyle en (C₃-C₈)-haloalkyle en (C₁-C₆), le radical cycloalkyle étant à chaque fois non substitué ou substitué par alkyle en (C₁-C₆) et/ou halogène.

3. Composés de la formule générale (I) selon la revendication 1 ou 2, **caractérisés en ce que** R^{2a} et R^{2b} sont chacun choisis indépendamment l'un de l'autre dans le groupe constitué par hydrogène, alkyle en (C₁-C₆), haloalkyle en (C₁-C₆), alcoxy en (C₁-C₆), alcoxy en (C₁-C₆)-alkyle en (C₁-C₆), cycloalkyle en (C₃-C₈), cycloalkyle en (C₃-C₈) -alkyle en (C₁-C₆) et cycloalkyle en (C₃-C₈)-haloalkyle en (C₁-C₆), le radical cycloalkyle étant à chaque fois non substitué ou substitué par alkyle en (C₁-C₆) et/ou halogène.

4. Composés de la formule générale (I) selon l'une quelconque des revendications 1 à 3, **caractérisés en ce que** R¹ et R^{2a} sont reliés l'un avec l'autre par une liaison, de manière à former ensemble avec les carbones 4 et 5 de la pyrimidine et le groupe carbonyle à la position 5 de la pyrimidine un carbo- ou hétérocycle partiellement hydrogéné de 5 à 7 chaînons, où les hétéroatomes sont choisis dans le groupe constitué par l'oxygène ou le soufre, un groupe sulfoxyde ou un groupe sulfonyle pouvant être présent à la place du soufre, et le carbo- ou l'hétérocycle étant non substitué ou substitué avec un ou plusieurs substituants choisis dans le groupe constitué par alkyle en (C₁-C₃), haloalkyle en (C₁-C₃), cycloalkyle en (C₃-C₆) et phényle (C₆H₅), qui peut être substitué une fois ou plusieurs fois avec fluor, chlore, brome ou iode.

5. Composés de la formule générale (I) selon l'une quelconque des revendications 1 à 4, **caractérisés en ce que** R³ est choisi dans le groupe constitué par hydrogène, alkyle en (C₁-C₆), halogénoalkyle en (C₁-C₆), alcoxy en (C₁-C₆), alcoxy en (C₁-C₆) -alkyle en (C₁-C₆), alcényle en (C₂-C₆), halogénoalcényle en (C₂-C₆), alcynyle en (C₂-C₆) et halogénoalcynyle en (C₂-C₆), et aryle en (C₆-C₁₄)-alkyle en (C₁-C₆).

6. Composés de la formule générale (I) selon l'une quelconque des revendications 1 à 5, **caractérisés en ce que** R⁴ et R⁵ sont chacun choisis indépendamment l'un de l'autre dans le groupe constitué par hydrogène, hydroxy, alkyle en (C₁-C₆), alkylphényle en (C₁-C₆) et alcoxy en (C₁-C₆) .

7. Composés de la formule générale (I) selon l'une quelconque des revendications 1 à 6, **caractérisés en ce que** R⁶ et R⁷ sont choisis indépendamment l'un de l'autre dans le groupe constitué par hydrogène, alkyle en (C₁-C₆) et aryle en (C₆-C₁₄).

8. Composés de la formule générale (I) selon l'une quelconque des revendications 1 à 7, **caractérisés en ce que** R⁸ est choisi dans le groupe constitué par hydrogène, halogène, alkyle en (C₁-C₆), alcoxy en (C₁-C₆) et aryle en (C₆-C₁₄).

9. Composés de la formule générale (I) selon l'une quelconque des revendications 1 à 8, **caractérisés en ce que** R⁹ est choisi dans le groupe constitué par hydrogène, halogène, alkyle en (C₁-C₆) et alcoxy en (C₁-C₆).

10. Composés de la formule générale (I) selon l'une quelconque des revendications 1 à 9, **caractérisés en ce que** R¹⁰ est choisi dans le groupe constitué par hydrogène, halogène, cyano, aminocarbonyle, hydroxycarbonyle, alkyle en (C₁-C₆), haloalkyle en (C₁-C₆), alcoxy en (C₁-C₆), alcényle en (C₂-C₆), alcynyle en (C₂-C₆), alkyle en (C₁-C₆)-alcynyle en (C₂-C₆), hydroxy-alkyle en (C₁-C₆) -alcynyle en (C₂-C₆), alcoxy en (C₁-C₆)-alcynyle en (C₂-C₆) et aryl-alcynyle en (C₂-C₆).

11. Composés de la formule générale (I) selon l'une quelconque des revendications 1 à 10, **caractérisés en ce que** R¹¹ est choisi dans le groupe constitué par hydrogène et alkyle en (C₁-C₆).

12. Composés de la formule générale (I) selon l'une quelconque des revendications 1 à 11, **caractérisés en ce que** X est choisi dans le groupe constitué par une liaison chimique, CH₂, O, S, carbonyle, NH, CH-alkyle en (C₁-C₆), N-alkyle en (C₁-C₆), OCH₂ et SCH₂, l'atome de carbone dans les deux derniers groupes cités étant relié à la partie aromatique et l'hétéroatome O ou S étant relié à la partie partiellement hydrogénée de l'amine.

13. Composés de la formule générale (I) selon l'une quelconque des revendications 1 à 12, **caractérisés en ce que** R⁹ et R¹⁰ sont reliés par un groupe -O-CH₂-O- en formant un cycle.

14. Composés de la formule générale (I) selon l'une quelconque des revendications 1 à 13, **caractérisés en ce que** le nombre n vaut 0 ou 1.

15. Composés de la formule générale (I) : 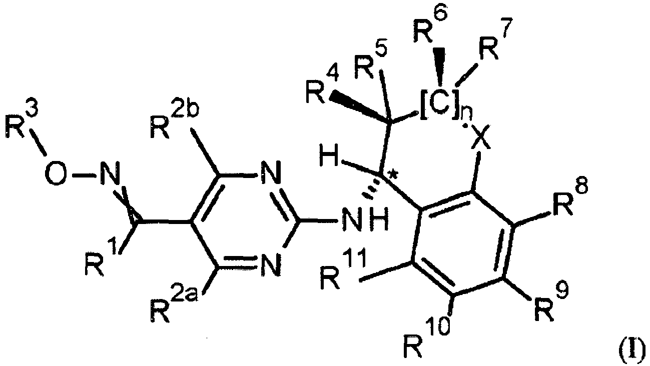 selon l'une quelconque des revendications 1 à 14, **caractérisés en ce que** l'atome de carbone chiral désigné par (*) présente une configuration (R).

16. Composés de la formule générale (I) : 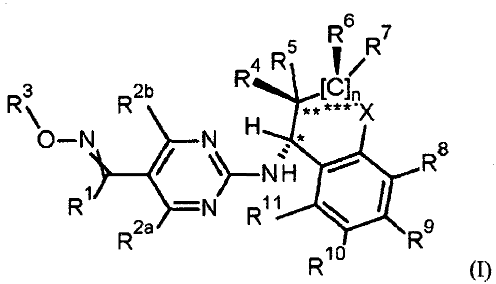 selon l'une quelconque des revendications 1 à 15, **caractérisés en ce que** l'atome de carbone chiral désigné par (*) présente une configuration (R) et l'atome de carbone chiral désigné par (**) présente une configuration (S).

17. Procédé de fabrication de composés de la formule générale (I) et/ou leurs sels agrochimiquement compatibles et/ou leurs dérivés azotés quaternisés agrochimiquement compatibles 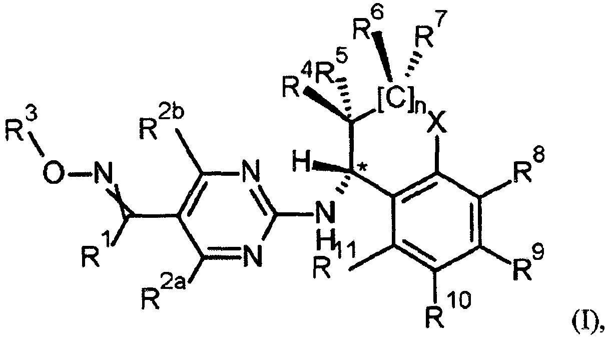 dans laquelle les radicaux R¹ à R¹¹, ainsi que X et n sont tels que définis dans l'une quelconque des revendications 1 à 14, selon lequel
un composé de la formule générale (II) : 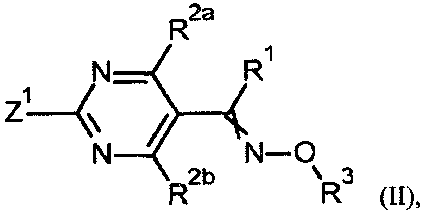 dans laquelle R¹, R^{2a}, R^{2b} et R³ sont tels que définis dans l'une quelconque des revendications 1 à 14, et Z¹ représente un radical échangeable ou un groupe partant, choisi dans le groupe constitué par fluor, chlore, brome, iode, un alkylsulfanyle en (C₁-C₄), un alkylsulfinyle en (C₁-C₄), un alkylsulfonyle en (C₁-C₄), un phényl-alkylsulfonyle en (C₁-C₄) non substitué ou substitué une fois ou plusieurs fois avec fluor, chlore, brome ou alkyle en (C₁-C₄) ou alcoxy en (C₁-C₄), ou un alkylphénylsulfonyle en (C₁-C₄),
est mis en réaction avec une amine de la formule générale (III) ou avec un sel d'addition acide de l'amine de la formule générale (III) : 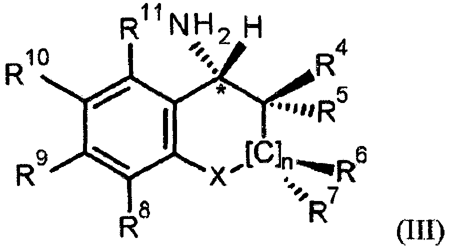 dans laquelle les radicaux R⁴ à R¹¹, ainsi que X et n sont tels que définis dans l'une quelconque des revendications 1 à 14.

18. Procédé de fabrication de composés de la formule générale (I) et/ou leurs sels agrochimiquement compatibles et/ou leurs dérivés azotés quaternisés agrochimiquement compatibles 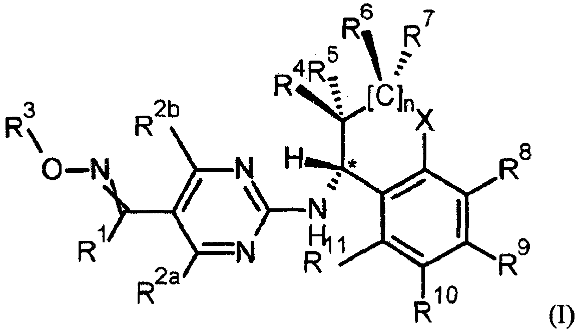 dans laquelle les radicaux R¹ à R¹¹, ainsi que X et n sont tels que définis dans l'une quelconque des revendications 1 à 14,
par condensation d'une guanidine de type (IV) ou d'un sel d'addition acide de celle-ci : 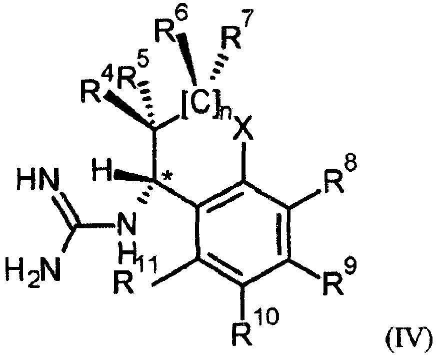 avec une cétone de la formule (V) : 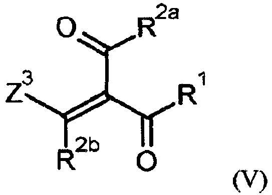 dans laquelle le radical Z³ représente un alcoxy en (C₁-C₆) ou un di-alkylamino en (C₁-C₆),
puis mise en réaction sous catalyse basique de la cétone de la formule (Ia) obtenue, dans laquelle Z² représente R³-CO, 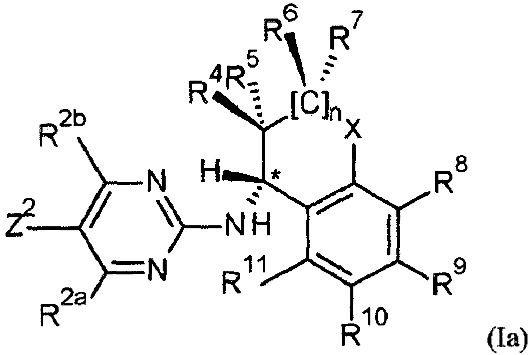 avec de l'hydroxylamine ou des hydroxylamines O-substituées pour former les composés de la formule générale (I).

19. Agent herbicide ou agent de régulation de la croissance des plantes, **caractérisé en ce qu'**il contient un ou plusieurs composés de la formule générale (I) ou leurs sels selon l'une quelconque des revendications 1 à 16.

20. Procédé de lutte contre des plantes nocives ou de régulation de la croissance de plantes, **caractérisé en ce qu'**une quantité efficace d'un ou de plusieurs composés de la formule générale (I) ou leurs sels selon l'une quelconque des revendications 1 à 16 est appliquée sur des plantes, des parties de plantes, des graines de plantes ou sur une surface de culture.

21. Utilisation de composés de la formule générale (I) ou leurs sels selon l'une quelconque des revendications 1 à 16 en tant qu'herbicides ou en tant que régulateurs de la croissance de plantes.

22. Utilisation selon la revendication 21, **caractérisée en ce que** les composés de la formule générale (I) ou leurs sels sont utilisés pour lutter contre des plantes nocives ou pour réguler la croissance de plantes dans des cultures de plantes utiles ou ornementales.

23. Utilisation selon la revendication 21 ou 22, **caractérisée en ce que** les plantes cultivées sont des plantes cultivées transgéniques.
